# EUROPEAN PATENT APPLICATION

(11) **EP 2 687 232 A1**
(43) Date of publication of application: **22.01.2014**
(21) Application number: 13168817.8
(22) Date of filing: 06.12.2007
(51) Int. Cl.: A61K 39/395, C07K 16/00, C07K 16/24

(54) **Methods of treating systemic lupus erythematosus**

(30) Priority: 06.12.2006 US 873008 P; 16.04.2007 US 907762 P; 16.04.2007 US 907767 P; 03.05.2007 US 924220 P; 03.05.2007 US 924219 P; 21.05.2007 US 924584 P; 19.09.2007 US 960187 P; 05.11.2007 US 996174 P; 05.11.2007 US 996176 P; 06.11.2007 US 996219 P
(62) Divisional of application: 07867634.3
(71) Applicant: MedImmune, LLC, Gaithersburg, MD 20878 (US)
(72) Inventor: White, Barbara, Finksburg, MD Maryland 21048 (US)
(74) Representative: Winter, Christopher Spencer

(57) **Abstract**

An anti-interferon a monoclonal antibody for use in reducing SLEDAI flares in a human subject with moderate, severe, or very severe SLE, wherein the human subject has a pre-treatment SLEDAI score of at least 5.

## Description

This application claims priority to U.S. Provisional Application Serial No. 60/873,008 filed December 6, 2006, U.S. Provisional Application Serial No. 60/907,762 filed April 16, 2007, U.S. Provisional Application Serial No. 60/924,219 filed May 3, 2007, U.S. Provisional Application Serial No. 60/924,584 filed May 21, 2007, U.S. Provisional Application Serial No. 60/960,187 filed September 19, 2007, U.S. Provisional Application Serial No. 60/996,176 filed November 5, 2007, International Patent Application No. PCT___________ entitled "Interferon Alpha-induced Pharmacodynamic Markers" filed December 6, 2007 (Attorney docket no. IA200PCT), U.S. Provisional Application Serial No. 60/924,220 filed May 3, 2007, U.S. Provisional Application entitled "Auto-Antibody Markers of Autoimmune Disease" filed November 6, 2007 (Attorney docket no. IA201 P2), U.S. Provisional Application entitled "Auto-Antibody Markers of Autoimmune Disease" filed December 6, 2007 (Attorney docket no. IA201P3), U.S. Provisional Application Serial No. 60/907,767 filed April 16, 2007, and U.S. Provisional Application Serial No. 60/996,174 filed November 5, 2007, each of which is incorporated herein by reference in their entirety.

### FIELD OF THE INVENTION

The present invention provides methods of treating systemic lupus erythematosus (SLE) in human patients, the methods comprising administration of an anti-interferon α antibody.

### BACKGROUND OF THE INVENTION

### Systemic Lupus Erythematosus

Systemic lupus erythematosus (SLE), often referred to as lupus, is a prototypic systemic autoimmune disease. (American College of Rheumatology Ad Hoc Committee on Systemic Lupus Erythematosus Guidelines. Guidelines for Referral and Management of systemic lupus erythematosus in adults. Arthritis Rheum 1999; 42: 1785-1796). The disease includes constitutional symptoms and signs, musculoskeletal, cutaneous, renal, gastrointestinal, pulmonary, cardiac, reticuloendothelial, hematologic and neuropsychiatric manifestations. The cutaneous manifestations are among the most common in SLE. In addition, serological abnormalities include antinuclear antibodies, antibodies to doublestranded (ds) DNA, Sm, RNP, Ro/SSA and La/SSB, and low complement levels.

### Epidemiology of SLE

The prevalence of SLE in the United States is on the order of 40 to 50 cases per 100,000 population.14 Reported incidence rates vary from 2 to 8 new cases per 100,000 population per year. (Uramoto KM; Michef CJ Jr; Thumboo J; Stenku J; O'Fallon WM; Garbiel SE. Trends in the incidence and mortality of systemic lupus erythematosus,, 1950-1992. Arthritis Rheum. 1999 Jan; 42(1):46-50).

The frequency of lupus is influenced by ethnicity. In the United States, SLE is more common among Asians, blacks, and those from the Caribbean and Polynesians in Hawaii. In the United Kingdom, Asians of Indian descent have a higher frequency of SLE. (Cooper GS; Dooley MA; Treadwell EL; St Clair EW; Parks CG; Gilkeson GS. Hormonal, environmental, and infectious risk factors for developing systemic lupus erythematosus. Arthritis Rheum 1998 Oct;41(10):1714-24).

SLE predominantly affects women of childbearing years, which is thought, in part, to be related to hormones. (Cooper GS; Dooley MA; Treadwell EL; St Clair EW; Parks CG; Gilkeson GS. Hormonal, environmental, and infectious risk factors for developing systemic lupus erythematosus. Arthritis Rheum 1998 Oct;41(10):1 1714-24). The disease may occur at any age. In children, ratio of females to males is about 3:1, and this rises at puberty. During the childbearing age range, the female-to-male ratio rises to 10 to 15:1, and in older individuals drops to 8:1. For lupus patients of all ages, the overall female-to-male ratio is given as 9 or 10:1. About 20% of patients develop lupus before 16 years of age, 65% between 16 and 65 years, and 15% after the age of 55 years.

There is evidence to support a role of genetic risk factors for SLE. (Mills JA. Systemic lupus erythematosus. N Engl J Med 1994;330(26):1871-9). Familial aggregation of SLE occurs. In addition, a variety of genetic markers appear to be more frequent in patients with SLE than in the general population, including HLA-A1, -B8, -DR3, and C4 null. In addition, a number of murine models of SLE support the existence of genes associated with the disease. Environmental factors may be important in that the dogs of patients with lupus are more likely to have anti-DNA antibodies than dogs belonging those of the general population. Viruses such as Epstein-Barr (EBV) virus, cytomegalovirus (CMV), and human immunodeficiency virus (HIV) have been implicated in precipitating or exacerbating SLE. Chemicals such as L-canavanine, contained in alfalfa sprouts, may induce an SLE-like illness. A number of drugs, including procainamide, isoniazid, and hydralazine, may induce an SLE-like illness. Cutaneous lesions may be triggered by exposure to ultraviolet light.

The severity of SLE varies by age, sex, ethnicity, and other factors. Children have more severe manifestations than adults. (Mills JA. Systemic lupus erythematosus. N Engl J Med 1994;330(26):1871-9). Men with SLE have a higher mortality, are more likely to have serositis, and are less likely to have photosensitivity. Ethnicity not only affects prevalence, but also severity of the disease. (Alarcon GS, McGwin G Jr, Bastian HM, Roseman J, Lisse J, Fessler BJ, Friedman AW, Reveille JD. Systemic lupus erythematosus in three ethnic groups. VII [correction of VIII]. Predictors of early mortality in the LUMINA cohort. LUMINA Study Group. Arthritis Rheum (Arthritis Care and Research). 2001 Apr;45(2):191-202. Erratum in: Arthritis Rheum 2001 Jun;45(3):306 and Reveille JD; Bartolucci A; Alarcon GS. Prognosis in systemic lupus erythematosus. Negative impact of increasing age at onset, black race, and thrombocytopenia, as well as causes of death. Arthritis Rheum 1990 Jan;33(1):37-48). American blacks have more severe disease than Caucasians, and are more likely to have anti-Sm and anti-RNP antibodies, proteinuria, serositis and psychosis. Other factors with a negative impact on prognosis of SLE include lower level of education and lower socioeconomic status, which may also affect access to medical care. (Callahan LF; Pincus T. Associations between clinical status questionnaire scores and formal education level in persons with systemic lupus erythematosus. Arthritis Rheum 1990 Mar;33(3):407-11).

### Manifestations of SLE

The most common manifestations of SLE at some time during the course of the disease are: fatigue (74%-100%), arthritis or arthralgia (83%-95%), skin involvement (80%-91%), fever (40%-80%), weight loss (44%-60%), renal (34%-73%), gastrointestinal (38%-44%), pulmonary (24%-98%), cardiac (20%-46%), lymphadenopathy (21%-50%), splenomegaly (9%-20%), hepatomegaly (7%-25%), and central nervous system involvement (25%-75%). (Buyon JP. Systemic Lupus Erythematosus: Clinical and Laboratory Features. In: Klippel JH, Crofford LJ, Stone JH, Weyand CM, editors. Primer on the Rheumatic Diseases. 12 Ed. Atlanta: Arthritis Foundation, 2001. p. 335-346 and Von Feldt JM. Systemic lupus erythematosus: recognizing its various presentations. Postgrad Med 1995;97(4):79-94). Of note, pneumonia may occur in 29% of patients, and pericarditis may occur in 8%-48% of patients, murmurs in 23% and electrocardiogram (ECG) changes in 34%-70%. Central nervous system involvement, including cognitive difficulties as reflected by functional impairment, affects most patients. Psychosis and convulsions, which are included in the 1982 ACR SLE classification criteria, occur in 5%-52% and 2%-20% of patients, respectively, over the course of the disease.

### Cutaneous SLE

A variety of cutaneous manifestations occur in SLE. Skin involvement in SLE may be classified as: (1) acute cutaneous lupus erythematosus (ACLE), (2) subacute cutaneous lupus erythematosus (SCLE), (3) chronic cutaneous lupus erythematosus (CCLE), (4) cutaneous vascular disease, (5) non-scarring alopecia, (6) other cutaneous manifestions. (23 Werth, VP. Clinical manifestations of cutaneous lupus erythematosus. Autoimmunity Reviews 2005: 4:296-302).

ACLE is most strongly associated and CCLE is least associated with systemic disease. ACLE includes the typical red, macular, malar eruption in a butterfly pattern over the cheeks and the bridge of the nose and a more generalized maculopapular eruption representing a photosensitive dennatitis. The malar rash occurs in 20%-60% of patients, and in addition to the butterfly pattern may involve the forehead, periorbital areas and the neck. The maculopapular eruption occurs in about 35% of patients with SLE. In some patients a generalized photosensitive eruption may occur. Rarely, the widespread eruption may include blistering lesions that resemble toxic epidermal necrolysis. ACLE lesions may be transient, lasting days to weeks, or prolonged. ACLE tends to be associated with the presence of oral ulceration most often involving the posterior surface of the hard palate. The lupus band test may be performed on skin biopsies to demonstrate the presence of immunoglobulins and C3 complement along the dermal-epidermal junction. In nonlesional skin, positive lupus band test results correlate strongly with more aggressive systemic disease.

SCLE is a photosensitive skin condition that does not produce scarring or atrophy. The primary lesion of SCLE is an erythematous papule or a small plaque with slight scaling that tends to expand and merge to form one of two variants: the papulosquamous variant (plaques with scaling) or the annular variant (annular and/or polycyclic lesions). SCLE is more common in Caucasians (85%) and women (female-to-male ratio 4: 1). Of patients with cutaneous lupus, 10%-50% have SCLE.

SCLE occurs in genetically predisposed individuals, most often in patients with human leukocyte antigen B8 (HLA-B8), HLA-DR3, HLA-DRw52, and HLA-DQ1. A strong association exists with anti-Ro (SS-A) autoantibodies. The reaction is believed to be related to ultraviolet (UV) light modulation of autoantigens, epidermal cytokines, and adhesion molecules, with resultant keratinocyte apoptosis.

CCLE includes the following: (1) classic discoid lupus erythematosus (DLE), which may present as localized or generalized DLE; (2) hypertrophic (also called verrucous) DLE; (3) lupus profundus (also known as lupus panniculitis); (4) mucosal DLE, which may be oral or conjunctival; (5) lupus tumidus (also called urticarial plaque of LE); (6) chilblains LE; (7) lichenoid DLE, which may be considered as an overlap between cutaneous lupus and lichen planus and is also known as lupus planus. DLE is a chronic, scarring, atrophy-producing, photosensitive dermatosis. Scarring alopecia is a distressing complication. DLE may occur in patients with SLE. Some patients also have the lesions of SCLE, and some may have a malar rash. DLE is responsible for 50%-85% of cutaneous lupus. DLE may occur at any age but is most frequent in individuals aged 20-40 years. The mean age is about 38 years. The female-to-male ratio of DLE is 2:1. DLE is somewhat more common in African Americans than in Caucasians or Asians.

The primary DLE lesion is a red papule or plaque with slight-to-moderate scaling. As the lesion progresses, the scale may thicken and become adherent, and alterations in pigmentation may occur, with hypopigmentation in the central or inactive area and hyperpigmentation at the active edge. Lesions spread outward from the center and may merge. Dilation of follicular openings occurs with a keratinous plug, termed follicular plugging or patulous follicles. Resolution of the active lesion results in atrophy and scarring.

DLE lesions often are found in sun-exposed areas, but relatively unexposed skin also may be affected. The scalp is commonly involved, and permanent alopecia may result. There are two subsets of DLE: localized and widespread. Localized DLE involves only the head and neck, whereas widespread DLE affects other areas whether or not the head and neck are involved. Patients with widespread involvement are more likely to have hematologic and serologic abnormalities or to develop SLE, and are more difficult to treat.

Cutaneous vascular disease has been classified by Sontheimer as follows: (1) vasculitis, which may occur in two forms, leukocytoclastic vasculitis-which manifests as palpable purpura or urticarial vasculitis-and the other form which manifests as periarteritis-like cutaneous lesions; (2) vasculopathy, which includes Degos' disease-like lesions 37 and secondary atrophie blanche (livedoid or livedo vasculitis); (3) periungual telangiectasia; (4) livedo reticularis; (5) thrombophlebitis; (6) Raynaud's phenomenon; and erythromelalgia (also known as erythermalgia). Note that Raynaud's phenomenon represents a systemic vasculopathy in which vasoconstriction occurs on exposure to cold. Blanching of fingers, toes, and other areas may occur.

Three forms of non-scarring alopecia may occur: (1) "Lupus hair" (hair broken off above the surface of the scalp); (2) telogen effluvium; and (3) alopecia areata. Other cutaneous manifestations of lupus include: calcinosis cutis; LE-nonspecific bullous lesions; urticaria; papulonodular mucinosis; cutis laxa; acanthosis nigricans; erythema multiforme; leg ulcers; and lichen planus. (Van den Broek MF, Muller U, Huang S, Zinkernagel RM, Aguet M. Immune defense in mice lacking type I and/or type II interferon receptors. Immunol Rev. 1995 Dec;148:5-18).

### Clinical Monitoring and Scoring of SLE Patients

Numerous different monitoring and scoring scales of SLE are well known to the skilled clinician, and have been routinely used in the evaluation of SLE patients. Examples of these scales are disclosed herein.

### Systeimic Lupus Erythematosus_Disease Activity Index (SLEDAI)

### Systemic Lupus Erythematosus Disease Activity Index (SLEDAI)

The clinical score known as SLEDAI is an index of SLE disease activity as measured and evaluated within the last 10 days (Bombardier C, Gladman D D, Urowitz M B, Caron D, Chang C H and the Committee on Prognosis Studies in SLE: Derivation of the SLEDAI for Lupus Patients. Arthritis Rheum 35:630-640, 1992.). Disease activity under the SLEDAI scoring system can range from 0 to 105. The following categories of SLEDAI activity have been defined: no activity (SLEDAI = 0); mild activity (SLEDAI = 1-5); moderate activity (SLEDAI = 6-10); high activity (SLEDAI = 11-19); very high activity (SLEDAI = 20 or higher). (Griffiths, et al., Assessment of Patients with Systemic Lupus Erythematosus and the use of Lupus Disease Activity Indices).

In certain instances, moderate SLE is defined as patients with SLEDAI scores of between and including 6-10. Alternatively, moderate SLE may be defined as patients with SLEDAI scores of between and including 5-10, 5-11, 5-12, 6-11, or even 6-12.

In certain instances, severe SLE is defined as patients with SLEDAI scores of between and including 11-19. Alternatively, severe SLE may be defined as patients with SLEDAI scores of between and including 12-20, or even 13-20.

In certain instances, very severe SLE is defined as patients with SLEDAI scores of greater than 20.

### The British Isles Lupus Asessment Group (BILAG Index

The BILAG index is an activity index of SLE that is based on specific clinical manifestations in eight organ systems: general, mucocutanebus, neurological, musculoskeletal, cardiovascular, respiratory, renal, and hematology results. Scoring is based on a letter system, but weighted numerical scores can also be assigned to each letter, making it possible to calculate a BILAG score in the range of 0-72. (Griffiths, et al., Assessment of Patients with Systemic Lupus Erythematosus and the use of Lupus Disease Activity Indices).

The BILAG index was developed in 1984 as a com-puterised index by rheumatologists from five centres inthe United Kingdom. The BILAG group wished tomove away from the global score approach, represented by the SLEDAI and SLAM indices. Thus, the BILAGindex is based on the intention to treat, on the premisethat, while physicians might not agree about the significance of individual clinical features or laboratory tests,there was broad agreement about when to treat lupuspatients with disease-modifying therapy such as highdose corticosteroids or immunosuppressants. It is atransitional index and scores both newly presenting fea-tures and changes in previously reported features.

Three versions have now been tested. The first version wastested for between-rater reliability, construct validity andface validity. The latter measure was not satisfactory anda second version produced that was shown to have highbetween-rater and within-rater reliability. It was also the most sensitive measure to assess disease activity fol-lowing the commencement of specific therapy, com-pared with the other systems tested, but was difficult tocomplete, because of lack of definitions and because thescore was calculated manually. The results from a multi-centre study in the UK, demonstrated both systematicand random between-rater variability in scoring musculoskeletal and vasculitic involvement, difficulty in scoring migrainous headaches and the ability to differentiatebetween patients with mild stable involvement and thosewith previous involvement of a system. The modified third version of the BILAG index was produced using a nominal consensus technique. Modifications included the clarification of questions to avoid ambiguity, the provision of a glossary, definition ofa time scale (1 month), standardization of the methodused to calculate BILAG scores and the addition of a newcategory, to indicate complete non-involvement of anorgan system.

The index now includes 86 items in 8 organ systems, general, mucocutaneous, neurological, musculoskeletal, cardiovascular/respiratory, vasculitic, renal and haematological. The item is scored as present or absent within theprevious month and some items are scored as being new,improved, the same or worse. Laboratory tests involved in the index include the presence and degree of protein-uria, plasma creatinine or creatinine clearance and thepresence of an active urinary sediment or histologicalevidence of active nephritis (in the previous 3 months). The full blood count and differential, evidence of haemolysis or a positive Coomb's test and evidence of circulating anticoagulant are also ideally required. Additional information that can be assessed includes the ESR, titre of anti-dsDNA antibodies and level of com-plement component C3. A global assessment, often a 10cm visual analogue scale with no activity at one end andworst possible activity at the other, is usually completedby both the physician and patient. Current disease-modifying or immunosuppressant drug therapy is documented, together with any changes made at the clinic visit, asare a wide range of additional therapies, such as anti-hypertensives, anticoagulants, hormone preparations oranalgesics. Each organ system is given a score of A to E repre-senting: (A) denotes disease thought to be sufficiently active to require disease-modifying treatment (pred-nisolone > 20mg/day or immunosuppressants); (B)denotes disease that is less active than in (A), mildreversible problems requiring only symptomatic therapysuch as anti-malarials, non-steroidal anti-inflammatorydrugs or prednisolone < 20mg/day; (C) indicates stable,mild disease; (D) indicates a previously affected but cur-rently inactive system; (E) indicates system never involved. For the purpose of comparing the differentactivity indices, it is possible to convert BILAG into aglobal score system. The system in current use assigns:A=9, B=3, C=1 and D and E=O, resulting in a potential range from 0 to 72.

### Physicians Global Assessment (PGA) Score

The PGA is the physicians' overall evaluation of a patients' disease activity. It is performed by the physician marking their assessment of a patients' overall disease activity on a 3 inch visual analogue scale with anchors at 0 (none), 1 inch (mild), 2 inches (moderate), and 3 inches (severe). Improvement is measured by reduction in the PGA score from visit to visit.

### ACR Criteria

To assist the clinician in the diagnosis and monitoring of lupus, the American College of Rheumatology (ACR) in 1982 issued a list of 11 symptoms or signs that help distinguish lupus from other diseases. A person should have four or more of these symptoms to suspect lupus. The symptoms do not all have to occur at the same time.

The eleven ACR criteria used for the diagnosis of lupus and a brief description follow.

Malar Rash: Rash over the cheeks.

Discoid Rash: Red raised patches.

Photosensitivity: Reaction to sunlight, resulting in the development of or increase in skin rash.

Oral Ulcers: Ulcers in the nose or mouth, usually painless.

Arthritis: Nonerosive arthritis involving two or more peripheral joints (arthritis in which the bones around the joints do not become destroyed).

Serositis: Pleuritis or pericarditis (inflammation of the lining of the lung or heart).

Renal Disorder: Excessive protein in the urine (greater than 0.5 gm/day or 3+ on test sticks) and/or cellular casts (abnormal elements the urine, derived from red and/or white cells and/or kidney tubule cells).

Neurologic Disorder: Seizures (convulsions) and/or psychosis in the absence of drugs or metabolic disturbances which are known to cause such effects.

Hematologic Disorder: Hemolytic anemia or leukopenia (white blood count below 4,000 cells per cubic millimeter) or lymphopenia (less than 1,500 lymphocytes per cubic millimeter) or thrombocytopenia (less than 100,000 platelets per cubic millimeter). The leukopenia and lymphopenia must be detected on two or more occasions. The thrombocytopenia must be detected in the absence of drugs known to induce it.

Antinuclear Antibody: Positive test for antinuclear antibodies (ANA) in the absence of drugs known to induce it.

Immunologic Disorder: Positive anti-double stranded anti-DNA test, positive anti-Sm test, positive antiphospholipid antibody such as anticardiolipin, or false positive syphilis test (VDRL). Adapted from: Tan, E.M., et. al. The 1982 Revised Criteria for the Classification of SLE. Arth Rheum 25: 1271-1277.

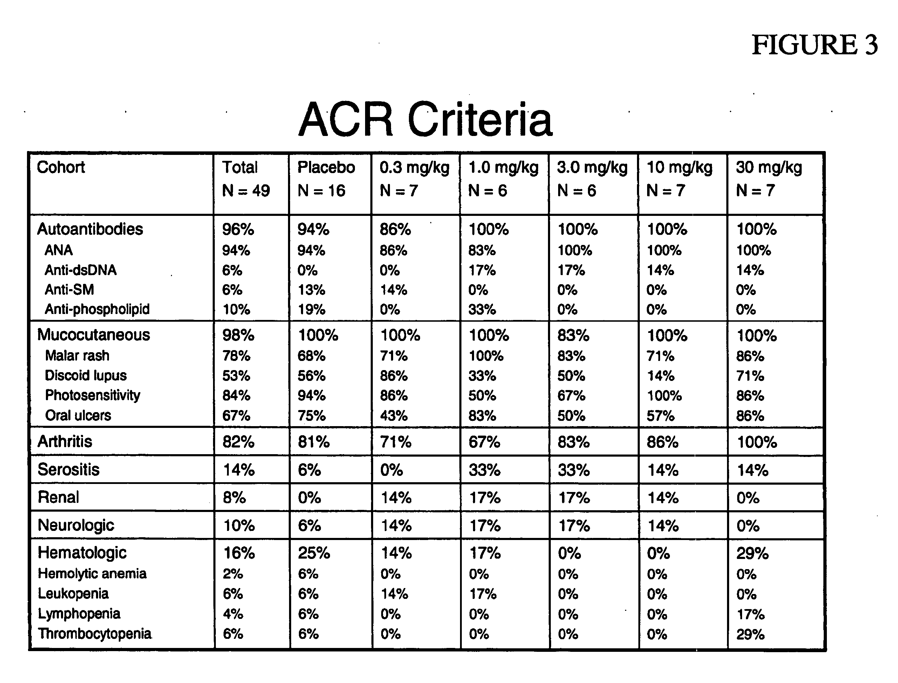

In addition to the eleven ACR criteria from 1982, five additional criteria are often used by clinicians, with the total of 16 criteria used being summarized in Figure 3 herein.

### Treatment of SLE

It has been four decades since a new treatment has been specifically approved for the treatment of SLE. As a consequence, most current treatments for SLE are not approved for use in this disease. These include a variety of immunosuppressant agents such as cyclophosphamide, methotrexate, and mycopheylate mofetil. Current treatments for mild SLE include non-steroidal anti-inflammatory agents and analgesics for fever, arthralgias and arthritis, and topical sun screens to minimize photosensitivity. If the latter agents fail to adequately control symptoms of mild SLE, antimalarial agents (such as hydroxychloroquine) and corticosteroids (such as prednisone) may be added to control arthralgia, arthritis, and rashes. Patients with moderate-to-severe disease activity are treated with corticosteroids, and steroid-sparing agents, such as azathioprine, cyclophosphamide, or mycophenylate mofetil. It is often difficult to taper patients with moderate or severe disease completely off corticosteroids, which cause long-term morbidity and may contribute toward early cardiovascular mortality. (Hahn, BH. Systemic lupus erythematosus and accelerated atherosclerosis. N Engl J Med 2003 Dec 18: 349(25):2379-80). The morbidity associated with prolonged corticosteroid treatment includes osteoporosis, avascular necrosis of bone, Cushingoid features, cutaneous changes, muscle wasting and weakness, easy bruising and many other complications.

Standard treatments have greatly improved the outcome in SLE patients, and mortality has been substantially decreased. However, there remains an unmet medical need as the burden of illness remains substantial. As a result, new effective treatments for SLE are needed.

### Role of Interferon-alpha in Systemic Lupus Erythematosus

Type I IFNs, α, β, θ, κ, and ω, are cytokines expressed from 13 functional IFN-α genes, one IFN-β gene, one IFN-θ gene, one IFN-κ gene, and one IFN-ω gene. (Theofilopoulos AN, Baccala R, Beutler B, Kono DH. Type I Interferons (α/β) in immunity and autoimmunity. Immunol Rev. 2005 Apr;204:9-26). There are at least 28 potential IFN-α subtypes, with the following being a partial listing of these: α1, α2a, α2b, α4, α5, α6, α7, α8, 0.10, α16, α17, and α21. In certain instances, reference to interferon alpha subtype α2 encompasses both α2a and α2b.

The characterization of IFN activity previously has focused primarily on the anti-viral properties of these molecules but in recent years, the role of Type I IFNs in both innate and adaptive immunity has been the subject of intense research, and a greater role of Type I IFNs in immune homeostasis is becoming apparent. Type I IFNs bind to the IFN-α Receptor (IFNAR), a cellular receptor that is a heterodimer composed of IFNAR-1 and 2 chains. Binding to this receptor results in the activation of intracellular signal transduction pathways (Stark GR, Kerr IM, Williams BR, Silverman RH, Schreiber RD. Annu Rev Biochem 1998;67:227-64), initiated by the activation of Jak kinases, Jak1 and Tyk2. These kinases subsequently phosphorylate signal transducer and activator of transcription (STAT) proteins, STATs 1 and 2. Phosphorylated STAT proteins form the transcription factor complex, IFN-stimulated gene factor 3 (ISGF3) that, together with p48, translocates into the nucleus. These complexes activate the IFN-stimulated response element (ISRE) that induces the expression of IFN-inducible genes.

In addition to specific antiviral function, Type I IFNs play a critical role in the regulation of the immune system. (Theofilopoulos AN, Baccala R, Beutler B, Kono DH. Type I Interferons (a/b) in immunity and autoimmunity. Immunol Rev. 2005 Apr;204:9-26 and Belardelli F, Gresser I. The neglected role of type I interferon in the T-cell response: implications for its clinical use. Immunol Today 1996; 17:369-72). Various types of cells including monocytes, macrophages, DCs, and lymphocytes, as well as other hematological cells, produce Type I IFNs in response to pro-inflammatory cytokines, as well as components of various pathogens. These cells also respond to Type I IFN and enhance the expression of immunologically important molecules such as MHC class I**,** CD38, interleukins (BLyS, IL-6, IL-10 and IL-15), and chemokines (IL-8, MCP-1, MCP-2, MIG, MIP1a, MIP1b, and IP10). Moreover, Type I IFNs induce multiple biological functions in key components of the immune system including dendritic, T, B, and natural killer (NK) cells. For example, Type 1 IFNs promote DC maturation, memory CD8+ T cell proliferation, inhibition of CD4+ T cell apoptosis, NK cell activation, and B cell differentiation. (Banchereau J, Pascual V, Palucka AK. Autoimmunity through cytokine-induced dendritic cell activiation. Immunity, Vol. 20, 539-550, May, 2004 and Taki S. Cytokine & Growth Factor Reviews 13 (2002) 379-391 and Mailliard RB, Son YI, Redlinger R, Coates PT, Giermasz A, Morel PA, Storkus WJ, Kalinski P. J Immunol. 2003 Sep 1;171(5):2366-73).

While almost all cells can produce Type I IFNs in response to stimulation by viral and bacterial components, plasmacytoid dendritic cells (pDCs), or "natural IFN-producing cells" produce up to 1000-fold more Type I IFN than other cell types. Type I IFN production can be induced by the stimulation of endosomal Toll-like receptors (TLR), such as TLR7 and TLR9, with single stranded RNA (ssRNA), hypomethylated CpGs in bacterial DNA, or autoantigen-antibody immune completes.

### Role of Type I Interferons in Viral Responses

Type I IFNs are significant in that they are promptly and widely expressed following viral infection and they participate in the innate (constitutive and immediate) immune response, both as a signal for the presence of infection and as an effector molecule inhibiting the spread of virus (anti-proliferative activity). For example, CMV replication in the tibialis anterior muscles of mice is markedly reduced by overexpression of the IFN-a1 transgene. (35 Yeow WS, Lawson CM, Beilharz MW. Antiviral activities of individual murine IFN-alpha subtypes in vivo: intramuscular injection of IFN expression constructs reduces cytomegalovirus replication. J Immunol. 1998 Mar 15; 160(6):2932-9). Similarly, IFN-a and IFN-b provide protective anti-viral immunity after herpes simplex virus Type I (HSV-1) infection. In this regard, studies comparing the efficacy of IFN-as, including Type I IFNs-a1, -a4, -a5, -a6, -a9, and -b, against HSV-1 infection suggest that IFN-b is the most potent inhibitory Type I IFN in viral replication in vitro.

The specific role of Type I and Type II IFNs in controlling a range of acute viral, bacterial, and parasitic infection has been widely studied in vivo in mice lacking expression of either IFNAR or the IFN-g receptor (IFNGR). (Van den Broek MF, Muller U, Huang S, Zinkernagel RM, Aguet M. Immune defense in mice lacking type I and/or type II interferon receptors. Immunol Rev. 1995 Dec;148:5-18). These studies support a complex interplay between Type I and Type II IFNs in regulating the inhibition and clearance of a number of experimental viruses. Data accumulated over the last decade suggest coordinated interactions among the Type I and Type II IFNs, IL-12, and IL-15 for activation of protective NK cell responses during viral infections. (Waldmann TA, Tagaya Y. The multifaceted regulation of interleukin-15 expression and the role of this cytokine in NK cell differentiation and host response to intracellular pathogens. Annu Rev Immunol). For example, after CMV infection, IL-12/STAT-4 was critical for NK cell IFN-g expression, whereas IFNs-a and b/STAT-1 were required for induction of cytotoxicity. The accumulation and survival of proliferating NK cells were STAT-4-independent, but required IFN-a and b/STAT-1 induction of IL-15. (Lee CK, Rao DT, Gertner R, Gimeno R, Frey AB, Levy DE. Distinct requirements for IFNs and STAT1 in NK cell function. J Immunol. 2000 Oct 1;165(7):3571-7).

Some viruses, including Lymphocytic Choriomeningitis Virus (LCMV) and Vesicular Stomatitis Virus (VSV), require only Type I IFNs for clearance (Steinhoff U, Muller U, Schertler A, Hensgartner H, Aguet M, Zinkernagel RM. Antiviral protection by vesicular stomatitis virus-specific antibodies in alpha/beta interferon receptor-deficient mice. J Virol. 1995 Apr;69(4):2153-8), whereas other viruses, such as HSV-1, require both Type I and Type II IFNs. Evidence of this is illustrated in IFNAR-KO x IFNGR-KO double deficient mice that succumb to HSV-1 infections, whereas IFNAR-KO or IFNGR-KO single deficient mice are able to control HSV-1 dissemination. (Luker GD,. Prior JL, Song J,. Pica CM , and. Leib DA. Bioluminescence imaging reveals systemic dissemination of herpes simplex virus type 1 in the absence of interferon receptors. J. Virol. 2003 Oct; 77 (20):11082-11093). Conversely, vaccinia virus (VV) resulted in uncontrolled spreading and death in both IFNAR-KO and IFNGR-KO single deficient mice. Most likely, a combination of the rate of virus replication and dissemination, the specific cellular tropism, the type of immune responses elicited by different viruses, and the particular experimental conditions all contribute to the explanation of the respective role of Type I and II IFNs in these virus challenge animal models.

In contrast to the viral data described above, influenza viral titers in the lungs of mice unable to respond to Type I IFNs were not significantly different from those of control animals for both primary and secondary infection. Thus, these observations reveal no significant contribution for IFN-controlled pathways in shaping acute or memory T cell responses to pneumotropic influenza virus infection. (Price GE, Gaszewska-Mastarlarz A, Moskophidis D.The role of alpha/beta and gamma interferons in development of immunity to influenza A virus in mice. J Virol. 2000 May;74(9):3996-4003).

While there is certainly a role for Type I IFNs in regulation of the host response to at least some viruses, the specific neutralization of the antiviral activity of IFN-a subtypes, or any other Type I IFN family member, and its consequences on the spread of viral infections remain unresolved. For example, Filovirus virulence (Ebola and Marburg) was assessed in normal Balb/c mice, Balb/c mice treated with IFN-a/b antibodies42, or IFNAR-KO mice. Infection resulted in generally more severe disease in IFNAR-KO, whereas mild disease was observed in animals treated with neutralizing IFN-a/b antibodies. Taken together, these results suggest that while impairment of signaling through Type I receptors may result in elevated viral titers, neutralization of a/b IFNs may allow for other Type I IFNs such as IFN ω, IFN-d or IFN-k, to provide anti-viral immunity. Despite the significance of the role of Type I IFNs in anti-viral immunity, the role of individual a subtypes or the role of a versus b IFNs remains poorly understood.

IFN-α subtypes are cytokines that belong to the Type I interferon family. The Type I family of IFNs includes α, β, θ, κ, and ω, which are expressed from 13 functional LFN-α genes, one IFN-β gene, one IFN-θ gene, one IFN-κ gene, and one IFN-ω gene. Interferons are produced in response to various stimuli. Type I IFNs bind to IFN-α Receptor (IFNαR), a cellular receptor that is a heterodimer composed of IFNαR1 and 2 chains. Binding to this receptor results in the activation of intracellular signal transduction pathways7 initiated by the activation of Jak kinases, Jak1 and Tyk2, ultimately resulting in the expression of IFN-inducible genes.

Characterization of IFN activity previously has focused primarily on the anti-viral properties of these molecules but in recent years, the role of Type I IFNs in both innate and adaptive immunity has been the subject of intense research, and a greater role of Type I IFNs in immune homeostasis is becoming apparent. Type I interferons are known to inhibit viral replication and contribute to immune system regulation. A substantial body of evidence suggests that Type I IFNs, including IFN-α subtypes, may also play a role in autoimmune diseases, such as systemic lupus erythematosus (SLE).

Citation or discussion of a reference herein shall not be construed as an admission that such is prior art to the present invention.

### SUMMARY OF THE INVENTION

The invention provides a method of treating SLE in a human subject comprising administration of an anti-interferon α antibody.

### BRIEF DESCRIPTION OF THE FIGURES

For the purpose of illustrating the invention, there are depicted in the drawings certain embodiments on the invention. However, the invention is not limited to the precise arrangements and instrumentalities of the embodiments depicted in the drawings.

**Figure 1****.** MEDI-545 Neutralization of SLE Plasma Activity (HIL3-luciferase Reporter Assay). This figure summarizes the results of a neutralization assay that demonstrates the ability of MEDI-545 to neutralize SLE patient plasma activity on an interferon α responsive cell line.

**Figure 2****.** Patient Demographics of the MI-CP-126 clinical study are summarized in this figure.

**Figure 3****.** ACR Criteria. This figure summarizes the ACR criteria used in evaluating SLE patients.

**Figure 4****.** Placebo Effect in MI-CP-126: Regression to the Mean. This figure summarizes the regression to the mean data analysis, comparing SLEDAI and BILAG scores of placebo, scores of ≥6, and scores of ≤6.

**Figure 5****.** MEDI-545 Neutralizes Type I IFN Gene Signature in Whole Blood. This figure summarizes gene signature analysis of patient whole blood, indicating that MEDI-545 neutralizes the IFN gene signature. Fraction of neutralization is denoted in the Y-axis, and days post-treatment is denoted in the X-axis.

**Figure 6****.** Dose-Dependent Effect of MEDI-545 in SLE. This figure summarizes gene signature analysis of patient whole blood, indicating that MEDI-545 neutralizes the IFN gene signature in a dose dependent manner. Fraction of neutralization is denoted in the Y-axis, and days post-treatment is denoted in the X-axis.

**Figure 7****.** MEDI-545 Reduces Type I IFN Gene Signature and Type I IFN-Induced Proteins in Skin. This figure summarizes gene signature analysis of patient skin and whole blood, indicating that MEDI-545 neutralizes the IFN gene signature. In addition, this figure provides histological analyses of patient skin and associated Type I IFN-induced proteins, demonstrating that MEDI-545 reduces the expression of IFN-induced proteins.

**Figure 8****.** MEDI-545 Can Normalize Type I IFN Gene Signature in Blood. This figure provides a heat map that summarizes target modulation calculated based on top 25 type I IFN-inducible genes upregulated in whole blood of one patient treated with 30 mg/kg MEDI-545 (day 0, 1, 4, 7, 14).

**Figure 9****.** Responses and Flares. This figure summarizes patient responses and flares using the SLEDAI and BILAG scoring systems, demonstrating the effect of MEDI-545 treatment compared to placebo, and the dose dependent effect exhibited by responders.

**Figures 10A** **and** **10B****.** SLEDAI Flares Tend to be Less Frequent in MEDI-545 Patients. Figure 10A summarizes the marked difference in flare reduction as measured with the SLEDAI index of MEDI-545 treated patients as compared to placebo on day 28 and day 56. The Y-axis denotes the percent of patients with flares. Figure 10B summarizes the marked difference in flare reduction as judged by the investigator of MEDI-545 treated patients as compared to placebo on day 14, 28 and day 56. The Y-axis denotes the percent of patients with flares.

**Figure 11**. BILAG A or B Flares Tend to be Less Frequent in MEDI-545 Patients. This figure summarizes the marked difference in flare reduction as measured using the BILAG index of MEDI-545 treated patients as compared to placebo on day 28 and day 56. The Y-axis denotes the percent of patients with flares.

**Figures 12A** **and** **12B****.** Overall, SLE Patients Tend to Have Improvement with MEDI-545. Figure 12A summarizes the day 28 and day 56 SLEDAI analysis of MEDI-545 treated patients as compared to placebo. Figure 12B summarizes the day 28 and day 56 BILAG analysis of MEDI-545 treated patients as compared to placebo.

**Figures 13A** **and** **13B**. SLE Patients with High Disease Activity Have Improvement with MEDI-545. Figure 13A summarizes the day 28 and day 56 SLEDAI analysis of MEDI-545 treated patients with SLEDAI baseline scores of ≥6 as compared to placebo. Figure 13B summarizes the day 28 and day 56 BILAG analysis of MEDI-545 treated patients with BILAG baseline scores of ≥6 as compared to placebo.

**Figures 14A** **and** **14B****.** SLE Patients with Low Disease Activity Have Little Change in Disease Activity with MEDI-545. Figure 14A summarizes the day 14, day 28 and day 56 SLEDAI analysis of MEDI-545 treated patients with SLEDAI baseline scores of <6 as compared to placebo. Figure 14B summarizes the day 14, day 28 and day 56 BILAG analysis of MEDI-545 treated patients with BILAG baseline scores of <6 as compared to placebo.

**Figure 15****.** Combined Responses Tend to be More Frequent in MEDI-545 Treated Patients. This figure summarizes the combined responses to MEDI-545 as compared to placebo, with all five dose levels being included.

**Figure 16****.** Combined Responses in M1-CP-126 vs. Belimumab Phase II Trial. This figure summarizes the combined response rate of MEDI-545 treated patients as compared to patients receiving Belimumab, indicating the rapid response (as early as day 14) that MEDI-545 treated patients exhibit.

**Figures 17A** **and** **17B****.** Inactive Disease Tends to be More Frequent in MEDI-545 Treated Patients. These figures summarize the marked increase in inactive disease exhibited by MEDI-545 treated patients of all dose levels as compared to placebo using both the SLEDAI (Figure 17A) and the BILAG (Figure 17B) scores.

**Figure 18****.** Effect of MEDI-545 on Mean SLEDAI and BILAG Scores. This figure summarizes the mean SLEDAI and BILAG scores at day 0, 14, 28, and 56 of MEDI-545 treated patients as compared to placebo.

**Figure 19****.** Patients with Low Disease Activity Tend to Have Improvement with MEDI-545. This figure summarizes the mean SLEDAI and BILAG scores at day 0, 14, 28, and 56 of MEDI-545 treated patients having baseline scores of <6, as compared to placebo treated.

**Figure 20****.** No Effect of MEDI-545 on Mean SLEDAI and BILAG Scores in Patients with High Disease Activity. This figure summarizes the mean SLEDAI and BILAG scores at day 0, 14, 28, and 56 of MEDI-545 treated patients having baseline scores of ≥6, as compared to placebo treated.

**Figure 21****.** Changes in BILAG and SLEDAI Scores Correlate With Each Other. This figure summarizes how the changes in both the BILAG and SLEDAI scores correlate.

**Figure 22****.** Effect of MEDI-545 on SLEDAI Days 14, 28, and 56. This figure summarizes the SLEDAI scores of patients treated with the four different doses of MEDI-545 as compared to placebo.

**Figure 23****.** MEDI-545 and Low Disease Activity: SLEDAI = 0. This figure summarizes the number of MEDI-545 treated patients who reached a SLEDAI score of 0 at least once during the 56 monitored days post treatment, as compared to control.

**Figure 24****.** MEDI-545 and Low Disease Activity: BILAG = 0. This figure summarizes the number of MEDI-545 treated patients who reached a BILAG score of 0 at least once during the 56 monitored days post treatment, as compared to control.

**Figure 25****.** MEDI-545 and Low Disease Activity: PGA <0.5. This figure summarizes the number of MEDI-545 treated patients who reached a PGA score of <0.5 at any time during the 56 monitored days post treatment, as compared to control.

**Figure 26****.** MEDI-545 and SLEDAI Flares. This figure summarizes the drastic reduction in SLEDAI flares in the MEDI-545 treated group as compared to placebo over the 56 monitored days post treatment.

**Figure 27****.** MEDI-545 and Need forNew Immunomodulatory Therapy. This figure summarizes the drastic reduction in the need for new immunomodulatory therapy in the MEDI-545 treated group as compared to placebo over the 56 monitored days post treatment.

**Figure 28****.** Pharmacokinetic Characteristics of MEDI-545: Mean Cₘₐₓ Values for MEDI-545. This figure summarizes the mean Cmax values, with the Y-axis denoting Cₘₐₓ of MEDI-545 in µg/ml and the X-axis denoting the MEDI-545 dose in mg/kg.

**Figure 29****.** Pharmacokinetic Characteristics of MEDI-545: Mean AUC Values for MEDI-545. This figure summarizes the mean AUC values, with the Y-axis denoting AUC of MEDI-545 in µg x dl/ml and the X-axis denoting the MEDI-545 dose in mg/kg.

### DETAILED DESCRIPTION OF THE INVENTION

### Treatment of SLE

In one embodiment, the invention provides a method of treating moderate, severe, or very severe SLE in a human subject, said method comprising admistration of an anti-IFNα antibody.

In another embodiment, the invention provides a method of treating a human subject diagnosed with moderate, severe, or very severe SLE, said method comprising administration of an anti-IFNα antibody.

In a further embodiment, the invention provides a method of treating SLE in a human subject comprising administration of an anti-IFNα antibody to said human subject, wherein said human subject is a member of an indicated population composed predominantly of human subjects with moderate, severe, or very severe SLE.

In another embodiment, the invention provides a method of treating SLE in a human population composed predominantly of human subjects with moderate, severe, or very severe SLE, said method comprising administration of an anti-IFNα antibody.

In one embodiment, the invention provides a method of treating SLE in a human subject, wherein said human subject has a pre-treatment SLEDAI score of X, wherein X is equal to between and including 6 to 105, said method comprising administration of an anti-interferon α antibody to said subject, wherein said administration yields a post-treatment SLEDAI score of X minus between and including 1 to 105, wherein the lowest SLEDAI value that X minus between and including 1 to 105 can yield is 0.

In yet a further embodiment, the invention provides a method of treating SLE in a human subject comprising the administration of an anti-IFNα antibody to said human subject, wherein said human subject is a member of a population composed predominantly of human subjects with a first pre-treatment SLEDAI score indicative of moderate, severe, or very severe SLE, and wherein said SLEDAI score improves post-treatment. When referring to indicated patient populations or other specific patients populations, this is meant to include those patients that can be classified or grouped based an the multiple different SLE scoring systems that are known in the art and some of which are discussed herein.

In another embodiment, the invention provides, a method of reducing SLEDAI flares in a human subject with moderate, severe, or very severe SLE, said method comprising administering to said subject and anti-IFNα antibody. When referring to a SLEDAI flare, the skilled clinician will typically interpret this to mean an increase or spike in SLEDAI score by at least 3. However, there may be differing interpretations of flares by clinicians. Accordingly, in one embodiment, a SLEDAI flare is an increase in SLEDAI score of at least at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, at least 64, at least 65, at least 66, at least 67, at least 68, at least 69, at least 70, at least 71, at least 72, at least 73, at least 74, at least 75, at least 76, at least 77, at least 78, at least 79, at least 80, at least 81, at least 82, at least 83, at least 84, at least 85, at least 86, at least 87, at least 88, at least 89, at least 90, at least 91, at least 92, at least 93, at least 94, at least 95, at least 96, at least 97, at least 98, at least 99, at least 100, at least 101, at least 102, at least 103, at least 104, or 105.

In another embodiment, a SLEDAI flare is an increase in SLEDAI score of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, or 105.

As discussed herein above, the following categories of SLEDAI activity have been defined: no activity (SLEDAI = 0); mild activity (SLEDAI = 1-5); moderate activity (SLEDAI = 6-10); high activity (S LEDAI = 11-19); very high activity (SLEDAI = 20 or higher). (Griffiths, et al., Assessment of Patients with Systemic Lupus Erythematosus and the use of Lupus Disease Activity Indices).

In certain instances, moderate SLE is defined as patients with SLEDAI scores of between and including 6-10. Alternatively, moderate SLE may be defined as patients with SLEDAI scores of between and including 5-10, 5-11, 5-12, 6-11, or even 6-12.

In certain instances, severe SLE is defined as patients with SLEDAI scores of between and including 11-19. Alternatively, severe SLE may be defined as patients with SLEDAI scores of between and including 12-20, or even 13-20.

In certain instances, very severe SLE is defined as patients with SLEDAI scores of greater than 20.

As discussed herein above, the SLEDAI scoring system is one manner in which clinicians routinely evaluate SLE patients. In certain embodiments, the patients treated with the anti-IFNα antibodies of the invention have a pre-treatment SLEDAI score of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, or 105.

In certain embodiments, the patients treated with the anti-IFNα antibodies of the invention have a pre-treatment SLEDAI score of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, at least 64, at least 65, at least 66, at least 67, at least 68, at least 69, at least 70, at least 71, at least 72, at least 73, at least 74, at least 75, at least 76, at least 77, at least 78, at least 79, at least 80, at least 81, at least 82, at least 83, at least 84, at least 85, at least 86, at least 87, at least 88, at least 89, at least 90, at least 91, at least 92, at least 93, at least 94, at least 95, at least 96, at least 97, at least 98, at least 99, at least 100, at least 101, at least 102, at least 103, at least 104, or 105.

In one embodiment, the patients treated with the anti-IFNα antibodies of the invention have a pre-treatment SLEDAI score of between and including 6-10. In another embodiment, the patients treated with the anti-IFNα antibodies of the invention have a pre-treatment SLEDAI score of between and including 6-11. In a further embodiment, the patients treated with the anti-IFNα antibodies of the invention have a pre-treatment SLEDAI score of between and including 10-20. In another embodiment, the patients treated with the anti-IFNα antibodies of the invention have a pre-treatment SLEDAI score of between and including 11-20.

In certain embodiments, the patients treated with the anti-IFNα antibodies of the invention have a post-treatment SLEDAI score of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, or 104.

In certain embodiments, the patients treated with the anti-IFNα antibodies of the invention have a post-treatment SLEDAI score that is reduced by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, or 105, with 0 being lowest post-treatment SLEDAI score attainable.

In certain embodiments, the patients treated with the anti-IFNα antibodies of the invention have a post-treatment SLEDAI score that is reduced by at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63,.at least 64, at least 65, at least 66, at least 67, at least 68, at least 69, at least 70, at least 71, at least 72, at least 73, at least 74, at least 75, at least 76, at least 77, at least 78, at least 79, at least 80, at least 81, at least 82, at least 83, at least 84, at least 85, at least 86, at least 87, at least 88, at least 89, at least 90, at least 91, at least 92, at least 93, at least 94, at least 95, at least 96, at least 97, at least 98, at least 99, at least 100, at least 101, at least 102, at least 103, at least 104, or 105, with 0 being lowest post-treatment SLEDAI score attainable.

In a further embodiment, the reduction in SLEDAI score is evident at at least 14 days, at least 28 days, or at least 56 days post antibody administration. In yet a further embodiment, the reduction in SLEDAI score is evident 14 days, 28 days, or 56 days post antibody administration.

As discussed herein above, the BILAG scoring system is one manner in which clinicians routinely evaluate SLE patients. In certain embodiments, the patients treated with the anti-IFNα antibodies of the invention have a pre-treatment BILAG score of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72.

In certain embodiments, the patients treated with the anti-IFNα antibodies of the invention have a pre-treatment BILAG score of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, at least 64, at least 65, at least 66, at least 67, at least 68, at least 69, at least 70, at least 71, or at least 72.

In certain embodiments, the patients treated with the anti-IFNα antibodies of the invention have a post-treatment BILAG score of 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, or 71.

In certain embodiments, the patients treated with the anti-IFNα antibodies of the invention have a post-treatment BILAG score that is reduced by 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, or 72, with 0 being lowest post-treatment BILAG score attainable.

In certain embodiments, the patients treated with the anti-IFNα antibodies of the invention have a post-treatment BILAG score that is reduced by at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, at least 50, at least 51, at least 52, at least 53, at least 54, at least 55, at least 56, at least 57, at least 58, at least 59, at least 60, at least 61, at least 62, at least 63, at least 64, at least 65, at least 66, at least 67, at least 68, at least 69, at least 70, at least 71, or at least 72, with 0 being lowest post-treatment BILAG score attainable.

### PGA Score

As discussed herein above, the PGA scoring system is routinely used by clinicians to evaluate SLE patients. The PGA is the physician's overall evaluation of a patient's disease activity. It is performed by the physician marking their assessment of a patient's overall disease activity on a 3 inch visual analogue scale with anchors at 0 (none), I inch (mild), 2 inches (moderate), and 3 inches (severe). Improvement is measured by reduction in the PGA score from visit to visit.

In one embodiment, post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in PGA score at I month, at 3 months, at 6 months, at 1 year, or at a duration longer than 1 year.

In a specific embodiment, prior to treatment, the patients exhibit a PGA score indicative of moderate, severe, or very severe SLE, and post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in PGA score at 1 month, at 3 months, at 6 months, at 1 year, or at a duration longer than 1 year.

### Composite Responder Index (CRI)

Another system that can be used by the skilled clinician for evaluating and assessing patient response in SLE treatment is a Composite Responder Index (CRI). This can incorporate scoring from several different systems (e.g. SLEDAI, BILAG A, and BILAG B) to evaluate a patient's response to SLE treatment.

In one embodiment, post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in CRI score at 1 month, at 3 months, at 6 months, at 1 year, or at a duration longer than 1 year.

In a specific embodiment, prior to treatment, the patients exhibit a CRI score indicative of moderate, severe, or very severe SLE, and post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in PGA score at 1 month, at 3 months, at 6 months, at 1 year, or at a duration longer than 1 year.

### ANAM Score

The ANAM4™ test system consists of a library of tests designed for a broad spectrum of clinical and research applications. This library of computer-based tests was constructed to meet the need for precise measurement of cognitive processing efficiency in a variety of psychological assessment contexts that include neuropsychology, readiness to perform, neurotoxicology, pharmacology, and human factors research. The ANAM has been validated to assess human performance, is well established in clinical practice and has been linked to standards in clinical diagnosis. ANAM tests are significantly correlated with age, depression, SLE disease activity, steroid dose and antibodies believed to be involved with cognitive dysfunction in SLE. We have selected the Code Substitution, Immediate; Code Substitution, Delayed, Spatial Processing; Simple Reaction Time; and Memory Search (with a 6 character memory set) modules for the evaluation of patients with systemic lupus erythematosus. Each test module is an independent, self contained module designed to evaluate the particular domain. When taking the ANAM, the tests proceed sequentially until the battery has been completed. ANAM is designed for repeated measures applications as scores stabilize after a few practice administrations. It has been reliably used with individuals whose primary language is Spanish and individuals with low levels of formal education. Each ANAM test is computer scored for five measures: 1) lapses - failure to answer during the allotted response window; 2) median reaction time for correct responses; 3) accuracy as the percentage of correct responses; 4) standard deviation of reaction time; and 5) throughput as the number of correct responses per minute. Throughput combines response speed, accuracy and consistency and is considered the best ANAM measure of cognitive efficiency. ANAM scores can be examined individually by subtest or as summary scores across all tests.

In one embodiment, post-treatment with the anti-IFNa antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in ANAM score at 1 month, at 3 months, at 6 months, at 1 year, or at a duration longer than 1 year. In a specific embodiment, the patient exhibits at least a 20% improvement in ANAM score at 1, 2, 3, or 6 months post treatment with the anti-IFNα antibodies of the invention.

In a specific embodiment, priort to treatment the patients exhibit an ANAM score indicative of moderate, severe, or very severe SLE, and post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in ANAM score at 1 month, at 3 months, at 6 months, at 1 year, or at a duration longer than 1 year.

### Fatigue Severity Scale (FSS)

Another manner in which SLE patients can be evaluated during the course of treatment, and after treatment, is using the Fatigue Severity Scale (FSS). The FSS questionnaire is a 9-item scale of fatigue. Individuals indicate the extent to which they agree with each statement of fatigue's impact on activities of daily living on a scale of 1 ("strongly disagree") to 7 ("strongly agree"). The FSS total score is the mean of these 9 scores. This scale has demonstrated adequate reliability and validity in patients with SLE, multiple sclerosis, and chronic fatigue syndrome. Fatigue is strongly associated with psychological dysfunction and emotional disturbances in patients with SLE, and accordingly, improvement in the FSS score may correlate with overall patient improvement.

In one embodiment, post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement as read by FSS at I month, at 3 months, at 6 months, at 1 year, or at a duration longer than 1 year. In a specific embodiment, the patient exhibits at least a 20% improvement as read by FSS at 1, 2, 3, or 6 months post treatment with the anti-IFNα antibodies of the invention.

In a specific embodiment, prior to treatment, the patients exhibit an FSS score indicative of moderate, severe, or very severe SLE, and post-treatment with the anti-IFNα antibodies of the invention, a patient exhibits at least a 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 100%, 125%, 150%, 175%, 200%, 300%, 400%, or 500% improvement in FSS score at 1 month, at 3 months, at 6 months, at 1 year, or at a duration longer than 1 year.

### ACR Criteria

As discussed herein above, the ACR criteria are routinely used by clinicians to evaluate SLE patients. In one embodiment, the patients treated with the anti-IFNα antibodies of the invention exhibit at least 1, at least 2, at least 3, at least 4, at least 5, at least 6, at least 7, at least 8, at least 9, at least 10, at least 11, at least 12, at least 13, at least 14, at least 15, or 16 of the listed ACR criteria of Figure 3 prior to treatement. In another embodiment, the patients treated with the anti-IFNα antibodies of the invention exhibit 1, 2, 3, 4, 5, 6, 7, 8, 9, 10, 11, 12, 13, 14, 15, or 16 of the listed ACR criteria of Figure 3 prior to treatment.

In another embodiment, the patients treated with the anti-IFNα antibodies of the invention exhibit 1, 2, 3, 4, 5, 6, 7, 8, 9, 10, 11, 12, 13, 14, or 15 of the listed ACR criteria of Figure 3 post-treatment. In a further embodiment, the patients treated with the anti-IFNα antibodies of the invention exhibit 1, 2, 3, 4, 5, 6, 7, 8, 9, 10, 11, 12, 13, 14, 15, or 16 fewer of the listed ACR criteria of Figure 3 post-treatment.

In implementing the present invention and associated methods of treatment, it will be desirable to measure disease activity and clinical outcomes of patients. A useful piece of information for doing so is the "Draft Guidance for Industry: Systemic Lupus Erythematosus-Developing Drugs for Treatment" that was put forth by the U.S. Department of Health and Human Services, Food and Drug Administration, Center for Drug Evaluation and Research (CDER), March 2005. Although available to the skilled clinician since March of 2005, exerpts are included herein and follow.

### Disease Activity Indices

The clinical measurement of disease activity in SLE involves an assessment of the characteristic signs and symptoms of disease and the results of laboratory parameters. Academic and clinical investigators have identified those measures they believe are important for evaluation in clinical trials. These parameters include a measure of disease activity, a measure of disease-induced damage, a measure of therapy-induced damage, a measure of response as determined by the patient (i.e., a patient global response), and a measure of health-related quality of life (HRQL).

Although patterns of stable, increasing, or decreasing disease activity form the basis for initiating or adjusting treatment in SLE, the specific manifestations that characterize the level of disease activity vary considerably from patient to patient and at different points in time. Indices of disease activity have been developed that correlate with assessments of panels of expert clinicians. These indices score disease manifestations using predefined criteria based on the presence or absence of different aspects of the disease or, in the case of the BILAG, on the clinician's assessment of the need to change therapy. In clinical studies, these indices have been shown to be valid based on the concordance of scores with expert opinion, acceptable interobserver variability among trained evaluators, correlation between individual patients' scores on different indices, and correlation between increases in scores and clinical decisions to increase therapy. The SLE Disease Activity Index (SLEDAI and SELENA-SLEDAI), the BILAG, the SLE Activity Measure (SLAM), and the European Consensus Lupus Activity Measure (ECLAM) have been shown in cohort studies to be sensitive to change in disease activity (Strand 1999) and are used in clinical trials.

### Flares

The clinical course of SLE is generally characterized by periods of relatively stable disease followed by flares of disease activity. Studies that measure disease activity at fixed time points may miss flares in between study assessments. In one study, rates of flare were measured at an average of 0.6 flares per year (Petri 1991). A flare should reflect an episode of increased disease activity and should correlate with a need for increase in or change in treatment on clinical grounds. Criteria for major flare might include the need for or initiation of high dose glucocorticoid therapy, a change in dose of immunosuppressive therapy, hospitalization, or death. The frequency of flares may be affected by gender, menopausal status, treatment, and other patient characteristics.

### Damage

Patients suffering from lupus experience irreversible damage to internal organ systems. Accumulation of damage occurs over a period of years. Therapy-induced organ damage may also occur. An index of organ damage was proposed and validated as the Systemic Lupus Erythematosus International Collaborating Clinics/American College of Rheumatology (SLICC/ACR) Damage Index. Validation studies show that high scores on the SLICC/ACR Damage Index are predictive of increased mortality, and damage in the renal and pulmonary components are associated with poor outcomes (Stoll 1996). The prognostic information derived from SLICC/ACR Damage Index scores suggests they may be useful as stratification variables for clinical trials. The SLICC/ACR Damage Index measures only changes that have been present for at least six months; therefore, only longer-term clinical trials could demonstrate reduction in the rate of progression of damage using this measure. Some of the components of the SLICC/ACR Damage Index are measures of toxicity related to current treatment modalities.

### Organ-Specific Indices

Organ-specific measures of disease provide another approach to assessing disease activity in lupus. To measure organ-specific disease activity in a clinical trial, a responder analysis could be applied by measuring if subjects demonstrate improvement in the involved organ system using prespecified criteria, such as components of validated disease activity indices if these components can be shown to reflect disease activity. Examples of issues related to studies of renal and skin involvement are provided below.

Lupus nephritis is the most commonly studied organ-specific manifestation of lupus. The presence of diffuse proliferative (WHO class IV) and severe focal proliferative (WHO class III) glomerulonephritis in patients with SLE who have measures of inflammatory activity and damage is associated with increased long-term risk of progression to end-stage renal disease and mortality. Patients with severe lupus nephritis are often treated with high doses of immunosuppressive agents, including cyclophosphamide, and high doses of corticosteroids. These regimens are based on studies that suggest a decrease in the long-tenn risk of progression to end-stage renal disease. The outcome of lupus nephritis has improved markedly in recent years with 5-year survival rates of 90 percent or greater and 10-year survival rates of more than 80 percent reported (Urowitz 1999). However, there remains a need for additional regimens as current treatments can be highly toxic and not effective in all subjects.

After a diagnosis of lupus nephritis is established, disease activity is assessed clinically by examination of the urinary sediment and by measures of renal function. A variety of outcome measures have been used in clinical trials of lupus nephritis to assess organ-specific disease activity. Mortality is an important outcome measure, but low mortality rates and long observation times make it a relatively insensitive measure in clinical trials. Measures of renal function can be used as outcome measures, including progression to end-stage renal disease (ESRD), sustained doubling of serum creatinine, creatinine clearance, and iothalamate clearance, for full approval. Other measures may also be suitable and can be employed in therapeutic studies if sufficient data to support the proposed measure are available. The use of the doubling of serum creatinine is the best-validated of these measures as it has been shown to reliably predict long-term renal outcomes; however, it is insensitive to smaller changes that represent earlier signs of damage that are nonetheless clinically important. Changes in the urine protein/creatinine ratio may serve as an indicator of the need for further assessment with a 24-hour urine collection for quantitation of the extent of proteinuria and impairment in renal function as measured by creatinine clearance.

Changes in urinalysis can provide important information for the assessment of renal inflammation in lupus nephritis. The presence of cellular casts and hematuria, when measured accurately, is considered a sensitive indicator of the level of activity of lupus nephritis. However, central laboratories may be unreliable in assessing the presence of casts as they can break up during transport. There is no consensus on the appropriate evaluation of urine sediment. Local or central laboratories could be used if the chosen method is shown to be accurate and reproducible.

Major flares of lupus nephritis, as assessed by urinary sediment, proteinuria and renal function, have been used as outcome measures in clinical trials. Patients who experience nephritic flares characterized by nephritic sediment and an increase in serum creatinine or decrease in glomerular filtration rate (GFR) may be at increased risk of developing a persistent doubling of serum creatinine. Renal remission in response to therapy has been defined as a return to normal levels of an elevated creatinine and proteinuria and normalization of nephritic sediment. Patients who fail to normalize an elevated serum creatinine in response to therapy may have an increased risk of progression to renal failure (Levey 1992). Assessment of proteinuria is particularly important in patients with membranous glomerulonephritis; however, this is a less common form of lupus nephritis.

Skin is one of the organs most involved in SLE. The most common of the skin manifestations include discoid lupus, malar rash, subacute cutaneous lupus, and alopecia. Photosensitivity and oral ulcers are additional common manifestations. A variety of outcome measures can be used in clinical trials to assess the efficacy of new therapies on skin disease including erythema, induration, scaling, and physician and patient global assessment. In addition, outcomes such as involved surface area changes and skin biopsies can be considered.

### Health-Related Quality of Life and Fatigue

It is recommended that HRQL measures be studied in all trials of SLE. Instruments that assess health status and HRQL may measure aspects of SLE and its impact on patients that are not fully assessed by other outcome measures. It is important that trials showing improvement in a specific organ or in disease activity demonstrate no or minimal worsening in measures of HRQL. Patients with active SLE may have increased disability as assessed by the Health Assessment Questionnaire (HAQ) or Modified Health Assessment Questionnaire (MHAQ). Health-related quality of life has been assessed in lupus patients using a number of generic instruments including the HAQ, MHAQ, Arthritis Impact Measurement Scale (AIMS), the Medical Outcomes Survey Short Form-20 (SF-20), and Short Form-36 (SF-36). Differences compared to controls have been observed in several domains and subdomains. Some instruments do not adequately assess fatigue, an important symptom for many lupus patients. Specific instruments have been studied for assessment of fatigue (e.g., the Krupp Fatigue Severity Scale (KFSS)). As with any instrument, HRQL instruments used in clinical trials of SLE should undergo validation regarding content validity (inclusion of all relevant domains), construct validity, sensitivity to change, and other criteria. The use of these outcomes is critical to understanding both the efficacy of an agent as well as its potential adverse events. Even if the measure does not improve with a specific therapy, it should not worsen. Improvement in HRQL alone would not result in approval at this time.

### Serologies

Serologic markers play an important role in the assessment of disease activity in SLE, including assessment of anti-double-stranded DNA, complement levels, and others. Serologic markers are critical for understanding the pathogenesis of disease. Serologic markers have an imperfect correlation with disease activity and cannot substitute for a direct assessment of clinical benefit.

### Reduction in Disease Activity of SLE

This claim is intended to reflect clinical benefit associated with reductions in the signs and symptoms of SLE disease activity. SLE is a disease of long duration, with a waxing and waning course; therefore, this claim would ordinarily be established by trials of at least 1 year in duration. For products that may elicit the formation of antibodies, it is important that the clinical trials assess whether antibodies are formed and if they adversely affect efficacy and safety. We recommend using methods that assess the activity of disease over the duration of the study in conjunction with methods that measure disease activity at the beginning and end. As part of any trials in support of this claim, we also recommend studying measures of damage and HRQL, as well as determining a patient global assessment. A validated disease activity index (DAI) is an acceptable outcome measure to demonstrate a reduction in signs and symptoms of SLE.

In a randomized clinical trial, the SLEDAI, the SELENA-SLEDAI, the SLAM, the BILAG, the ECLAM, or other established index could be used to measure disease activity. To represent a clinical benefit, the change in DAI should be both statistically significant and clinically meaningful and prospectively defined. Since the BILAG evaluates patients based on the need for additional treatment, the clinical interpretation of a change in score is apparent. A success in a 1-year trial could be defined as a greater reduction in the BILAG score at 1 year along with supportive evidence of reduction in monthly measurements of the BILAG score compared to controls (see also Section V.B.I, Disease Activity Trials, for a discussion of landmark versus area under the curve (AUC) analyses). For other indices, deciding whether changes in score are clinically meaningful may be more complicated. If a disease activity measure other than the BILAG is chosen, confirmation of a positive result with two different DAIs would be important to confirm the findings.

### Effectiveness in the Treatment of a Specific Organ System Manifestation

In general, appropriate outcome measures in organ-specific trials are defined by the specific organ under study. For each organ studied, these include: (1) stabilization (no worsening of disease activity in the designated organ); (2) partial response; (3) complete response but still receiving medications; (4) complete remission (no ongoing treatments); (5) flares (time to flare and/or number of flares); and (6) ability to taper concomitant corticosteroids by clinically significant amounts.

For products being proposed for use in the manner of a specified short course of treatment leading to induction of a sustained remission, studies of 3-6 months duration may be acceptable with longer term follow-up for safety and durability of response. For products being proposed for chronic use, studies as short as 1 year may be considered.

Trials using the organ-specific approach may be either for the treatment of each organ studied (e.g., lupus nephritis) or for the treatment of lupus, depending on the number of patients and the type of organ impairment studied. Trials should show that there would be no worsening in terms of a patient global assessment as well as health-related quality of life. Trials intended to study clinical benefit for specific organ systems could enroll subjects with disease affecting a single organ system (e.g., lupus nephritis). Patients enrolled in studies evaluating multiple organ systems can be stratified according to the specific organ system involved for randomization and analysis. It is important that the definition of a response be prospectively specified for each organ system under study. Trials of patients with disease activity affecting specific organ systems can define success as an increase in the proportion of responders among patients receiving study drug compared to controls.

Trials designed to assess efficacy of a product for the treatment of lupus nephritis should demonstrate an improved outcome for patients with biopsy-proved severe glomerulonephritis (WHO grades III or IV), or membranous glomerulonephritis. Short-term benefits may not reliably predict long-term outcomes; therefore, it may be recommended that trials of lupus nephritis be at least 1 year in duration. The following outcome measures could establish efficacy in lupus nephritis:

Incidence of mortality and progression to end-stage renal disease. Mortality and ESRD (when clearly defined prospectively) are objective, reliably determined, and the endpoints of ultimate importance. However, studies using these as the endpoint will generally require longer duration and larger sample size than may be needed when other endpoints are used.

Sustained doubling in serum creatinine or other measure that has been validated including approximations of GFR such as iothalamate clearance or creatinine clearance studies. Doubling of serum creatinine has been shown to be associated with progression to ESRD. Thus, a decrease in the proportion of subjects meeting this endpoint in the treatment group compared to controls can be interpreted as demonstrating a patient benefit. Lesser degrees of change or changes in other measures may be considered but should be further justified. Similarly a significant change in GFR which has clinical importance may be considered.

A success in a trial utilizing this outcome measure would be defined as a decrease in the proportion of subjects whose serum creatinine attains a level double that of the baseline value and remains doubled for at least six months. Alternatively, a success in a trial could be defined as a reduction in the proportion of subjects experiencing a sustained fall in GFR of 50 percent or more.

3) An unvalidated surrogate marker for lupus nephritis reasonably likely to predict clinical benefit. FDA regulations for accelerated approval of new therapeutic agents (21 CFR 314, subpart H and 21 CFR 601, subpart E) provide an additional framework for FDA approval of drugs intended to treat serious or life-threatening diseases. One approach is to base approval on the effect on a surrogate marker, provided that specific criteria are met, and there is a commitment to verify the actual clinical benefit of the agent in studies completed after approval. Demonstration of marked and sustained improvement in renal function and renal inflammation in a seriously affected population of patients with lupus glomerulonephritis may qualify for consideration under these regulations. Data showing that the measure of improvement is associated with improved patient outcomes can contribute to supporting the conclusion that the surrogate is reasonably likely to predict clinical benefit.

Use of the accelerated approval pathway for a product for lupus nephritis, for example, would necessitate the timely completion of studies of long-term clinical outcomes postmarketing. The verification of clinical benefit can be a difficult task. It is important that the necessary studies be a clearly described part of the clinical development program at the time the studies of the surrogate endpoint are undertaken.

4) Induction of renal remission. Active lupus nephritis is associated with evidence of renal inflammation, including cellular casts, proteinuria, and decreases in renal function. Organ-threatening WHO class III and IV lupus nephritis is frequently treated with cyclophosphamide and high doses of corticosteroids, agents that are associated with significant toxicity. A treatment that induces a sustained remission in lupus nephritis would confer a clinical benefit. Clinical studies of lupus nephritis use varied definitions of renal remission, but generally specify decreases in hematuria and cellular casts, decreases in proteinuria, and stabilization or improvement in renal function. A clinical trial intended to demonstrate induction of renal remission would specify a definition of renal remission that includes all relevant parameters. It is recommended to provide evidence supporting an association with improved clinical outcome (e.g., decreased likelihood of developing end-stage renal disease or need for dialysis) to defend the selected definition of renal remission. Because of concerns that patients with an inactive urinary sediment may nonetheless progress to renal failure, it is recommended that studies using renal remission as an outcome measure include follow-up renal biopsies in at least a subset of patients.

Patients with renal remission may be expected to experience a clinical benefit to the extent that they are: (a) spared treatment with potentially toxic agents; and/or (b) spared from ultimate progression to end-stage renal disease. It is encouraged for clinicians proposing to use attainment of renal remission to demonstrate efficacy of a product for lupus nephritis to discuss their clinical development plans with the responsible reviewing division at the Agency. Proposals for clinical trials using renal remission as an endpoint should: (a) provide a clear definition for renal remission, and data supporting the choice of that definition; (b) provide evidence that attaining a renal remission would be expected to translate into a clinical benefit to the patient; and (c) assess the durability of the renal remissions.

5) Resolution of nephrotic syndrome. Patients with lupus nephritis may have high grade proteinuria with nephrotic syndrome. A clinical trial intended to demonstrate resolution of nephrotic syndrome would enroll patients with high grade proteinuria (e.g., ≥4 gm/d) and assess the proportion of patients who attain a prespecified, substantial reduction in proteinuria (e.g., to less than 500 mg per 24 hours). The trial should also collect data on the associated features of nephrotic syndrome (i.e., hypoalbuminemia, generalized edema, and hyperlipidemia) to assess whether changes in these parameters mirror improvements in proteinuria. It is encouraged that clinicians proposing to use resolution of nephrotic syndrome to demonstrate efficacy of a product for lupus nephritis to discuss their clinical development plans with the responsible review division at the FDA.

### Complete Clinical Response/Remission

A complete clinical response/remission claim would be approved for products that demonstrate the ability to induce a clinical response, characterized by the complete absence of disease activity at all sites for at least 6 consecutive months. This response is termed complete clinical response if the subjects continue to receive lupus-directed therapies. Remission occurs if subjects were receiving no ongoing therapy for their SLE. A trial in support of the claim of complete clinical response should be at least 12 months in duration and demonstrate an increase in the proportion of subjects in whom a disease activity measure achieves zero.

### Reduction in Flares

Reductions in the rate of flares of SLE or time to flare are considered to be clinically important outcomes. An increase in the frequency and severity of flares of lupus nephritis is correlated with worse outcomes. Thus, a reduction in the rate of flares of organ-specific disease (e.g., lupus nephritis) is also considered clinically important. If time-to-flare is evaluated as the efficacy endpoint, the study should be of sufficient duration to evaluate whether the flares are suppressed or only delayed in occurrence. Thus, a comparison of flare rate or incidence of flare-free at an appropriate time point will be a critical secondary endpoint. An established measure of flare may be considered in clinical trials studying flare as a primary outcome to demonstrate a decreased frequency of, or decreased severity of, flares. It is recommended to provide evidence that the chosen definition of flare accurately measures clinical flares. Proposals for clinical trials using renal flare as an endpoint should: (1) provide a clear and accepted definition for renal flare, and data supporting the choice of that definition; (2) provide evidence that reducing renal flare incidence by that definition of renal flare would be expected to translate into a clinical benefit to the patient; and (3) assess the durability of the renal benefit. A success in a clinical trial could be defined as an increase in the time-to-flare or as a decrease in the number or severity of flares over the course of a 1-year trial.

### Pharmacodynamic Markers

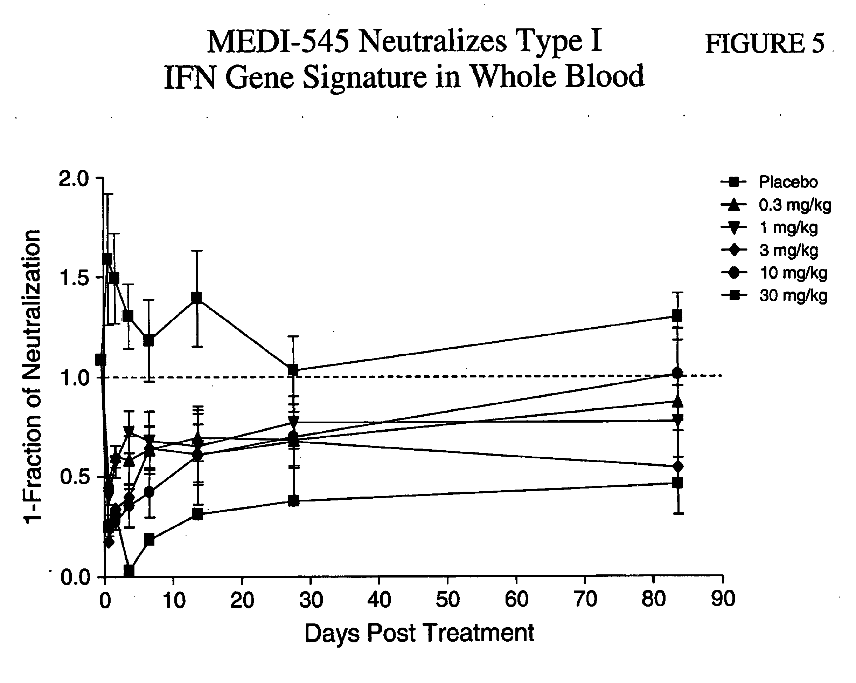

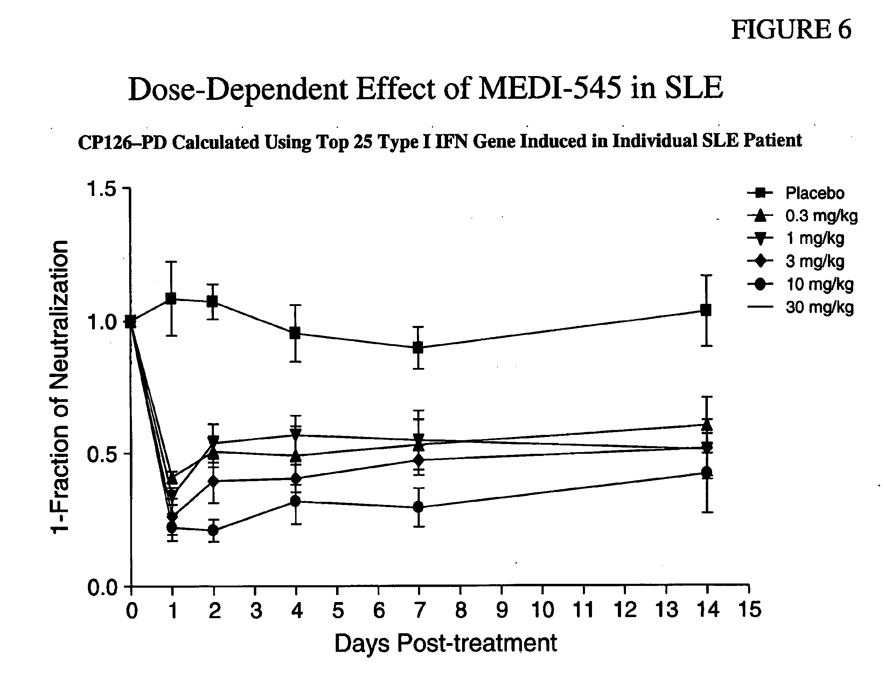

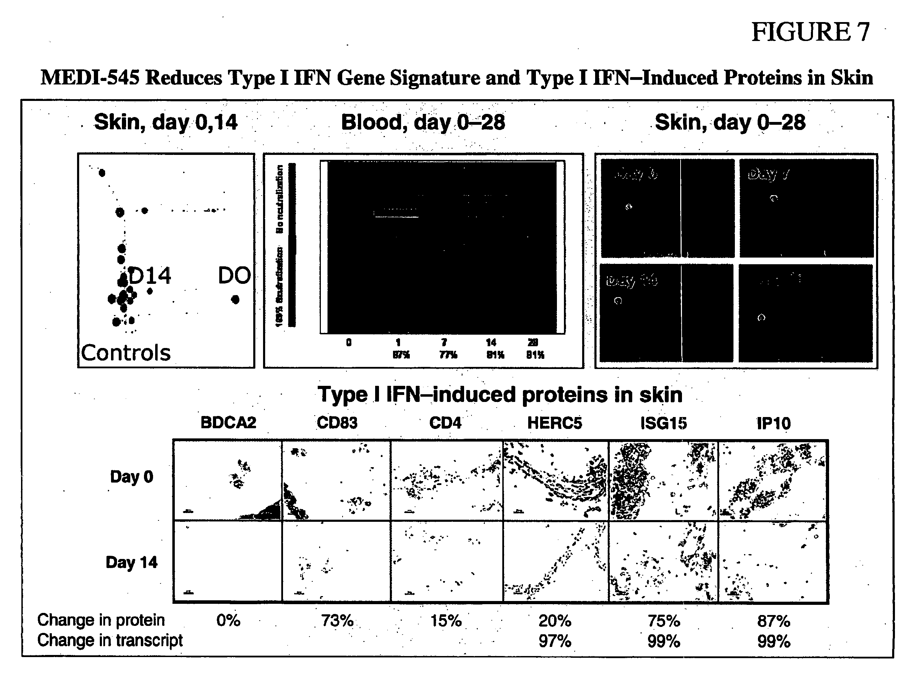

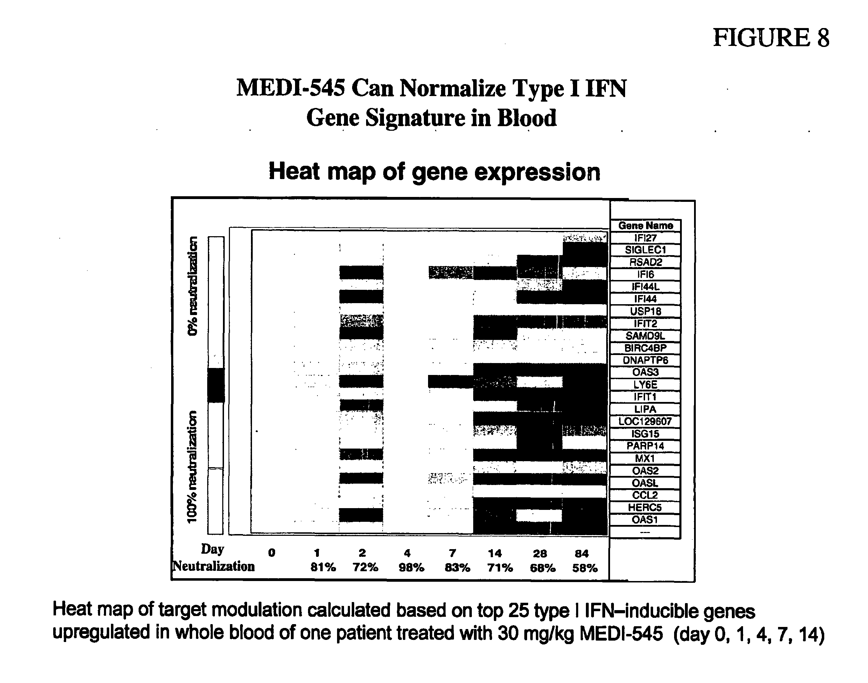

In additional to the scoring evaluation criteria discussed herein, it may also be desirable to monitor patients and assess the effects of treatment with the anti-interferon α antibodies of the invention using pharmacodynamic marker analyses. These markers may comprise sets of genes that are indicative of SLE, including gene signatures related to IFNα activity or reduction in activity. The markers may also comprise sets of autoantibodies that are indicative of the SLE phenotype. Examples of this type of analysis are summarized in Figures 5-8 herein, and further examples and descriptions of the methodologies used to generate the data in Figures 5-8 are available in, for example, but not limited to, U.S. Provisional Application Serial No. 60/873,008 filed December 6, 2006, U.S. Provisional Application Serial No. 60/907,762 filed April 16, 2007, U.S. Provisional Application Serial No. 60/924,219 filed May 3, 2007, U.S. Provisional Application Serial No. 60/924,584 filed May 21, 2007, U.S. Provisional Application Serial No. 60/960,187 filed September 19, 2007, U.S. Provisional Application Serial No. 60/996,176 filed November 5, 2007, International Patent Application No. PCT________ entitled "Interferon Alpha-induced Pharmacodynamic Markers" filed December 6, 2007 (Attorney docket no. IA200PCT), U.S. Provisional Application Serial No. 60/924,220 filed May 3, 2007, U.S. Provisional Application entitled "Auto-Antibody Markers of Autoimmune Disease" filed November 6, 2007 (Attorney docket no. IA201 P2), U.S. Provisional Application entitled "Auto-Antibody Markers of Autoimmune Disease" filed December 6, 2007 (Attorney docket no. IA201P3). For specific examples, see Tables 1, 19-24, 26-28, 30, 32, 33, Figures 72a, 74, 75, and 77, and as claimed in International Patent Application No. PCT_entitled "Interferon Alpha-induced Pharmacodynamic Markers" filed December 6, 2007 (Attorney docket no. IA200PCT). For further specific examples, see also Tables 2, 4, 5, and 9 in U.S. Provisional Application entitled "Auto-Antibody Markers of Autoimmune Disease" filed December 6, 2007 (Attorney docket no. IA201P3).

### Dosing and Administration

The amount of the composition of the invention which will be effective in the treatment, prevention or management of SLE can be determined by standard research techniques. Selection of the preferred effective dose can be determined (e.g., via clinical trials) by a skilled artisan based upon the consideration of several factors which will be known to one of ordinary skill in the art. Such factors include the symptoms involved, the patient's body mass, the patient's immune status and other factors known by the skilled artisan to reflect the accuracy of administered pharmaceutical compositions.

The precise dose to be employed in the formulation will also depend on the route of administration, and the seriousness of the SLE symptoms displayed (e.g., as scored by SLEDAI or BILAG), and should be decided according to the judgment of the practitioner and each patient's circumstances. Effective doses may be extrapolated from dose-response curves derived from in vitro or animal model test systems.

For antibodies, the dosage administered to a patient is typically 0.1 mg/kg to 100 mg/kg of the patient's body weight. In one embodiment, the dosage administered to a patient is between 0.1 mg/kg and 30 mg/kg of the patient's body weight. In another embodiment, the dosage administered to a patient is between 1 mg/kg and 10 mg/kg of the patient's body weight. In another embodiment, the dosage administered to a patient is between 0.1 mg/kg and 20 mg/kg of the patient's body weight. In another embodiment, the dosage administered to a patient is between 0.1 mg/kg and 10 mg/kg of the patient's body weight. In another embodiment, the dosage administered to a patient is between 1 mg/kg and 50 mg/kg of the patient's body weight. In another embodiment, the dosage administered to a patient is between 1 mg/kg and 40 mg/kg of the patient's body weight. In another embodiment, the dosage administered to a patient is between 1 mg/kg and 30 mg/kg of the patient's body weight. In another embodiment, the dosage administered to a patient is between I mg/kg and 20 mg/kg of the patient's body weight. In another embodiment, the dosage administered to a patient is between 1 mg/kg and 10 mg/kg of the patient's body weight. In another embodiment, the dosage administered to a patient is between 1 mg/kg and 5 mg/kg of the patient's body weight. In another embodiment, the dosage administered to a patient is between 5 mg/kg and 10 mg/kg of the patient's body weight. In another embodiment, the dosage administered to a patient is between 0.3 mg/kg and 30 mg/kg of the patient's body weight. In another embodiment, the dosage administered to a patient is 0.1 mg/kg, 0.2 mg/kg, 0.3 mg/kg, 0.4 mg/kg, 0.5 mg/kg, 1.0 mg/kg, 1.5 mg/kg, 2.0 mg/kg, 2.5 mg/kg, 3.0 mg/kg, 3.5 mg/kg, 4.0 mg/kg, 4.5 mg/kg, 5.0 mg/kg, 5.5 mg/kg, 6.0 mg/kg, 6.5 mg/kg, 7.0 mg/kg, 7.5 mg/kg, 8.0 mg/kg, 8.5 mg/kg, 9.0 mg/kg, 9.5 mg/kg, 10.0 mg/kg, 11.0 mg/kg, 12.0 mg/kg, 13.0 mg/kg, 14.0 mg/kg, 15.0 mg/kg, 16.0 mg/kg, 17.0 mg/kg, 18.0 mg/kg, 19.0 mg/kg, 20.0 mg/kg, 25 mg/kg, 30 mg/kg, 35 mg/kg, 40 mg/kg, 45 mg/kg, or 50 mg/kg of the patient's body weight. In a specific embodiment, the dosage administered to a patient is selected from the group consisting of 0.3 mg/kg, 1.0 mg/kg, 3.0 mg/kg, 10 mg/kg, and 30 mg/kg of the patient's body weight.

Generally, human and humanized antibodies have a longer half-life within the human body than antibodies from other species due to the immune response to the foreign polypeptides. Thus, lower dosages of human antibodies and less frequent administration is often possible.

Various delivery systems are known and can be used to administer an anti-IFNα antibody of the invention or the combination of an anti-IFNα antibody of the invention and an additional prophylactic agent or therapeutic agent useful for preventing or treating SLE, e.g., encapsulation in liposomes, microparticles, microcapsules, recombinant cells capable of expressing the antibody or antibody fragment, receptor-mediated endocytosis (see, e.g., Wu and Wu, 1987, J. Biol. Chem. 262:4429-4432), construction of a nucleic acid as part of a retroviral or other vector, etc. Methods of administering a prophylactic or therapeutic agent of the invention include, but are not limited to, parenteral administration (e.g., intradermal, intramuscular, intraperitoneal, intravenous and subcutaneous), epidural, and mucosal (e.g., intranasal, inhaled, and oral routes). In a specific embodiment, prophylactic or therapeutic agents of the invention are administered intramuscularly, intravenously, or subcutaneously. The prophylactic or therapeutic agents may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local.

### Anti-Interferon α Antibodies

Antibodies of the invention include, but are not limited to, synthetic antibodies, monoclonal antibodies, polyclonal antibodies, recombinantly produced antibodies, intrabodies, multispecific antibodies (including bi-specific antibodies), human antibodies, humanized antibodies, chimeric antibodies, synthetic antibodies, single-chain Fvs (scFv) (including bi-specific scFvs), BiTE molecules, single chain antibodies Fab fragments, F(ab') fragments, disulfide-linked Fvs (sdFv), and anti-idiotypic (anti-Id) antibodies, and epitope-binding fragments of any of the above. In particular, antibodies of the present invention include immunoglobulin molecules and immunologically active portions of immunoglobulin molecules. Furthermore, the antibodies of the invention can be of any isotype. In one embodiment, antibodies of the invention are of the IgG1, IgG2, IgG3 or IgG4 isotype. The antibodies of the invention can be full-length antibodies comprising variable and constant regions, or they can be antigen-binding fragments thereof, such as a single chain antibody, or a Fab or Fab'2 fragment.

The invention also provides an immunoconjugate comprising an antibody of the invention, or antigen-binding portion thereof, linked to a therapeutic agent, such as a cytotoxin or a radioactive isotope. The invention also provides a bispecific molecule comprising an antibody, or antigen-binding portion thereof, of the invention, linked to a second functional moiety having a different binding specificity than said antibody, or antigen binding portion thereof.

Compositions comprising an antibody, or antigen-binding portion thereof, or immunoconjugate or bispecific molecule of the invention and a pharmaceutically acceptable carrier are also provided.

Antibodies that bind multiple subtypes of interferon α are known in the art. Nonlimiting examples of these antibodies can be found in, for example, U.S. Patent No. 7,087,726 and U.S. Patent Appl. Publ. No. 2007/0014724. For example, in one embodiment, the anti-IFNα antibodies that may be used in the invention are any of those disclosed in Example 1 and Example 2 of U.S. Patent No. 7,087,726, including, for example, those disclosed in Table 3 and Table 4, and/or those disclosed in the table entitled "Deposit of Material" on lines 25-54, column 56, and may comprise SEQ ID Nos 1, 2, 3, 4, 5, 6, 7, 8, 9, 10, 11, 12, 13, and/or 14 as disclosed in U.S. Patent No. 7, 087,726, and may further include chimeric, humanized, or human versions of these antibodies (if not already a chimeric, humanized, or human version), and may further include fragments or derivatives thereof.

In certain embodiments, it may be desirable to alter the activity of specific subtypes, or combinations of subtypes, of interferon α. Accordingly, in one embodiment, the anti-interferon α antibodies selectively bind to at least interferon α subtype α2, which may include the α2a and/or α2b subtypes. In another embodiment, the anti-interferon α antibodies selectively bind to at least interferon α subtype α1, α2, α4, α5, α6, α7, α8, α10, α13, α14, α16, α17, or α21.

In one embodiment, the anti-interferon α antibodies selectively bind to at least interferon α subtype α1, α2, α4, α5, α6, α7, α8, α10, α13, α14, α16, α17, and α21. In one embodiment, the anti-interferon α antibodies selectively bind to at least interferon α subtype α1, α2, α4, α5, α6, α7, α8, α10, α13, α14, α16, and α17. In one embodiment, the anti-interferon α antibodies selectively bind to at least interferon α subtype α1, α2, α4, α5, α6, α7, α8, α10, α13, α14, and α16. In one embodiment, the anti-interferon α antibodies selectively bind to at least interferon α subtype α1, α2, α4, α5, α6, α7, α8, α10, α13, and α14. In one embodiment, the anti-interferon α antibodies selectively bind to at least interferon α subtype α1, α2, α4, α5, α6, α7, α8, α10, and α13. In one embodiment, the anti-interferon α antibodies selectively bind to at least interferon α subtype α1, α2, α4, α5, α6, α7, α8, and α10. In one embodiment, the anti-interferon α antibodies selectively bind to at least interferon α subtype α1, α2, α4, α5, α6, α7, and α8. In one embodiment, the anti-interferon α antibodies selectively bind to at least interferon α subtype α1, α2, α4, α5, α6, and α7. In one embodiment, the anti-interferon α antibodies selectively bind to at least interferon α subtype α1, α2, α4, α5, and α6. In one embodiment, the anti-interferon α antibodies selectively bind to at least interferon α subtype α1, α2, α4, and α5. In one embodiment, the anti-interferon α antibodies selectively bind to at least interferon α subtype α1, α2, and α4. In one embodiment, the anti-interferon α antibodies selectively bind to at least interferon α subtype α1 and α2. In one embodiment, the anti-interferon α antibodies selectively bind to at least interferon α subtype and α1.

In one embodiment, the anti-interferon α antibodies selectively bind to at least interferon α subtype α2, α4, α5, α6, α7, α8, α10, α13, α14, α16, α17, and α21. In one embodiment, the anti-interferon α antibodies selectively bind to at least interferon α subtype α4, α5, α6, α7, α8, α10, α13, α14, α16, α17, and α21. In one embodiment, the anti-interferon α antibodies selectively bind to at least interferon α subtype α5, α6, α7, α8, α10, α13, α14, α16, α17, and α21. In one embodiment, the anti-interferon α antibodies selectively bind to at least interferon α subtype α6, α7, α8, α10, α13, α14, α16, α17, and α21. In one embodiment, the anti-interferon α antibodies selectively bind to at least interferon α subtype α7, α8, α10, α13, α14, α16, α17, and α21. In one embodiment, the anti-interferon α antibodies selectively bind to at least interferon α subtype α8, α10, α13, α14, α16, α17, and α21. In one embodiment, the anti-interferon α antibodies selectively bind to at least interferon α subtype α10, α13, α14, α16, α17, and α21. In one embodiment, the anti-interferon α antibodies selectively bind to at least interferon α subtype α13, α14, α16, α17, and α21. In one embodiment, the anti-interferon α antibodies selectively bind to at least interferon α subtype α14, α16, α17, and α21. In one embodiment, the anti-interferon α antibodies selectively bind to at least interferon α subtype α16, α17, and α21. In one embodiment, the anti-interferon α antibodies selectively bind to at least interferon α subtype α17 and α21. In one embodiment, the anti-interferon α antibodies selectively bind to at least interferon α subtype α21.

In one embodiment, the anti-interferon α antibodies selectively bind to at least interferon α subtype α1, α4, α5, α6, α7, α8, α10, α13, α14, α16, α17, and α21. In one embodiment, the anti-interferon α antibodies selectively bind to at least interferon α subtype α1, α2, α5, α6, α7, α8, α10, α13, α14, α16, α17, and α21. In one embodiment, the anti-interferon α antibodies selectively bind to at least interferon α subtype α1, α2, α4, α6, α7, α8, α10, α13, α14, α16, α17, and α21. In one embodiment, the anti-interferon α antibodies selectively bind to at least interferon α subtype α1, α2, α4, α5, α7, α8, α10, α13, α14, α16, α17, and α21. In one embodiment, the anti-interferon α antibodies selectively bind to at least interferon α subtype α1, α2, α4, α5, α6, α8, α10, α13, α14, α16, α17, and α21. In one embodiment, the anti-interferon α antibodies selectively bind to at least interferon α subtype α1, α2, α4, α5, α6, α7, α10, α13, α14, α16, α17, and α21. In one embodiment, the anti-interferon α antibodies selectively bind to at least interferon α subtype α1, α2, α4, α5, α6, α7, α8, α13, α14, α16, α17, and α21. In one embodiment, the anti-interferon α antibodies selectively bind to at least interferon α subtype α1, α2, α4, α5, α6, α7, α8, α10, α14, α16, α17, and α21. In one embodiment, the anti-interferon α antibodies selectively bind to at least interferon α subtype α1, α2, α4, α5, α6, α7, α8, α10, α13, α16, α17, and α21. In one embodiment, the anti-interferon α antibodies selectively bind to at least interferon α subtype α1, α2, α4, α5, α6, α7, α8, α10, α13, α14, α17, and α21. In one embodiment, the anti-interferon α antibodies selectively bind to at least interferon α subtype α1, α2, α4, α5, α6, α7, α8, α10, α13, α14, α16, and α21.

In one embodiment, the anti-interferon α antibodies selectively bind to at least interferon α subtype α1, α4, α5, α6, α7, α8, α10, α13, α14, α16, α17, and α21. In one embodiment, the anti-interferon α antibodies selectively bind to at least interferon α subtype α1, α5, α6, α7, α8, α10, α13, α14, α16, α17, and α21. In one embodiment, the anti-interferon α antibodies selectively bind to at least interferon α subtype α1, α6, α7, α8, α10, α13, α14, α16, α17, and α21. In one embodiment, the anti-interferon α antibodies selectively bind to at least interferon α subtype α1, α7, α8, α10, α13, α14, α16, α17, and α21. In one embodiment, the anti-interferon α antibodies selectively bind to at least interferon α subtype α1, α8, α10, α13, α14, α16, α17, and α21. In one embodiment, the anti-interferon α antibodies selectively bind to at least interferon α subtype α1, α10, α13, α14, α16, α17, and α21. In one embodiment, the anti-interferon α antibodies selectively bind to at least interferon α subtype α1, α13, α14, α16, α17, and α21. In one embodiment, the anti-interferon α antibodies selectively bind to at least interferon α subtype α1, α14, α16, α17, and α21. In one embodiment, the anti-interferon α antibodies selectively bind to at least interferon α subtype α1, α16, α17, and α21. In one embodiment, the anti-interferon α antibodies selectively bind to at least interferon α subtype α1, α17, and α21. In one embodiment, the anti-interferon α antibodies selectively bind to at least interferon α subtype α1 and α21.

In one embodiment, the anti-interferon α antibodies selectively bind to at least interferon α subtype α1, α2, α6, α7, α8, α10, α13, α14, α16, α17, and α21. In one embodiment, the anti-interferon α antibodies selectively bind to at least interferon α subtype α1, α2, α7, α8, α10, α13, α14, α16, α17, and α21. In one embodiment, the anti-interferon α antibodies selectively bind to at least interferon α subtype α1, α2, α8, α10, α13, α14, α16, α17, and α21. In one embodiment, the anti-interferon α antibodies selectively bind to at least interferon α subtype α1, α2, α10, α13, α14, α16, α17, and α21. In one embodiment, the anti-interferon α antibodies selectively bind to at least interferon α subtype α1, α2, α13, α14, α16, α17, and α21. In one embodiment, the anti-interferon α antibodies selectively bind to at least interferon α subtype α1, α2, α14, α16, α17, and α21. In one embodiment, the anti-interferon α antibodies selectively bind to at least interferon α subtype α1, α2, α16, α17, and α21. In one embodiment, the anti-interferon α antibodies selectively bind to at least interferon α subtype α1, α2, α17, and α21. In one embodiment, the anti-interferon α antibodies selectively bind to at least interferon α subtype α1, α2, and α21. In one embodiment, the anti-interferon α antibodies selectively bind to at least interferon α subtype α and α2.

In one embodiment, the anti-interferon α antibodies selectively bind to at least interferon α subtype α1, α2, α4, α6, α7, α8, α10, α13, α14, α16, α17, and α21. In one embodiment, the anti-interferon α antibodies selectively bind to at least interferon α subtype α1, α2, α4, α7, α8, α10, α13, α14, α16, α17, and α21. In one embodiment, the anti-interferon α antibodies selectively bind to at least interferon α subtype α1, α2, α4, α8, α10, α13, α14, α16, α17, and α21. In one embodiment, the anti-interferon α antibodies selectively bind to at least interferon α subtype α1, α2, α4, α10, α13, α14, α16, α17, and α21. In one embodiment, the anti-interferon α antibodies selectively bind to at least interferon α subtype α1, α2, α4, α13, α14, α16, α17, and α21. In one embodiment, the anti-interferon α antibodies selectively bind to at least interferon α subtype α1, α2, α4, α14, α16, α17, and α21. In one embodiment, the anti-interferon α antibodies selectively bind to at least interferon α subtype α1, α2, α4, α16, α17, and α21. In one embodiment, the anti-interferon α antibodies selectively bind to at least interferon α subtype α1, α2, α4, α17, and α21. In one embodiment, the anti-interferon α antibodies selectively bind to at least interferon α subtype α1, α2, α4, and α21. In one embodiment, the anti-interferon α antibodies selectively bind to at least interferon α subtype α1, α2, and α4.

In one embodiment, the anti-interferon α antibodies selectively bind to at least interferon α subtype α1, α2, α4, α5, α7, α8, α10, α13, α14, α16, α17, and α21. In one embodiment, the anti-interferon α antibodies selectively bind to at least interferon α subtype α1, α2, α4, α5, α8, α10, α13, α14, α16, α17, and α21. In one embodiment, the anti-interferon α antibodies selectively bind to at least interferon α subtype α1, α2, α4, α5, α10, α13, α14, α16, α17, and α21. In one embodiment, the anti-interferon α antibodies selectively bind to at least interferon α subtype α1, α2, α4, α5, α13, α14, α16, α17, and α21. In one embodiment, the anti-interferon α antibodies selectively bind to at least interferon α subtype α1, α2, α4, α5, α14, α16, α17, and α21. In one embodiment, the anti-interferon α antibodies selectively bind to at least interferon α subtype α1, α2, α4, α5, α16, α17, and α21. In one embodiment, the anti-interferon α antibodies selectively bind to at least interferon α subtype α1, α2, α4, α5, α17, and α21. In one embodiment, the anti-interferon α antibodies selectively bind to at least interferon α subtype α1, α2, α4, α5, and α21. In one embodiment, the anti-interferon α antibodies selectively bind to at least interferon α subtype α1, α2, α4, and α5.

In one embodiment, the anti-interferon α antibodies selectively bind to at least interferon α subtype α1, α2, α4, α5, α6, α8, α10, α13, α14, α16, α17, and α21. In one embodiment, the anti-interferon α antibodies selectively bind to at least interferon α subtype α1, α2, α4, α5, α6, α10, α13, α14, α16, α17, and α21. In one embodiment, the anti-interferon α antibodies selectively bind to at least interferon α subtype α1, α2, α4, α5, α6, α13, α14, α16, α17, and α21. In one embodiment, the anti-interferon α antibodies selectively bind to at least interferon α subtype α1, α2, α4, α5, α6, α14, α16, α17, and α21. In one embodiment, the anti-interferon α antibodies selectively bind to at least interferon α subtype α1, α2, α4, α5, α6, α16, α17, and α21. In one embodiment, the anti-interferon α antibodies selectively bind to at least interferon α subtype α1, α2, α4, α5, α6, α17, and α21. In one embodiment, the anti-interferon α antibodies selectively bind to at least interferon α subtype α1, α2, α4, α5, α6, and α21. In one embodiment, the anti-interferon α antibodies selectively bind to at least interferon α subtype α1, α2, α4, α5, and α6.

In one embodiment, the anti-interferon α antibodies selectively bind to at least interferon α subtype α1, α2, α4, α5, α6, α7, α10, α13, α14, α16, α17, and α21. In one embodiment, the anti-interferon α antibodies selectively bind to at least interferon α subtype α1, α2, α4, α5, α6, α7, α13, α14, α16, α17, and α21. In one embodiment, the anti-interferon α antibodies selectively bind to at least interferon α subtype α1, α2, α4, α5, α6, α7, α14, α16, α17, and α21. In one embodiment, the anti-interferon α antibodies selectively bind to at least interferon α subtype α1, α2, α4, α5, α6, α7, α16, α17, and α21. In one embodiment, the anti-interferon α antibodies selectively bind to at least interferon α subtype α1, α2, α4, α5, α6, α7, α17, and α21. In one embodiment, the anti-interferon α antibodies selectively bind to at least interferon α subtype α1, α2, α4, α5, α6, α7, and α21. In one embodiment, the anti-interferon α antibodies selectively bind to at least interferon α subtype α1, α2, α4, α5, α6, and α7.

In one embodiment, the anti-interferon α antibodies selectively bind to at least interferon α subtype α1, α2, α4, α5, α6, α7, α8, α13, α14, α16, α17, and α21. In one embodiment, the anti-interferon α antibodies selectively bind to at least interferon α subtype α1, α2, α4, α5, α6, α7, α8, α14, α16, α17, and α21. In one embodiment, the anti-interferon α antibodies selectively bind to at least interferon α subtype α1, α2, α4, α5, α6, α7, α8, α16, α17, and α21. In one embodiment, the anti-interferon α antibodies selectively bind to at least interferon α subtype α1, α2, α4, α5, α6, α7, α8, α17, and α21. In one embodiment, the anti-interferon α antibodies selectively bind to at least interferon α subtype α1, α2, α4, α5, α6, α7, α8, and α21. In one embodiment, the anti-interferon α antibodies selectively bind to at least interferon α subtype α1, α2, α4, α5, α6, α7, and α8.

In one embodiment, the anti-interferon α antibodies selectively bind to at least interferon α subtype α1, α2, α4, α5, α6, α7, α8, α10, α14, α16, α17, and α21. In one embodiment, the anti-interferon α antibodies selectively bind to at least interferon α subtype α1, α2, α4, α5, α6, α7, α8, α10, α16, α17, and α21. In one embodiment, the anti-interferon α antibodies selectively bind to at least interferon α subtype α1, α2, α4, α5, α6, α7, α8, α10, α17, and α21. In one embodiment, the anti-interferon α antibodies selectively bind to at least interferon α subtype α1, α2, α4, α5, α6, α7, α8, α10, and α21. In one embodiment, the anti-interferon α antibodies selectively bind to at least interferon α subtype α1, α2, α4, α5, α6, α7, α8, and α10.

In one embodiment, the anti-interferon α antibodies selectively bind to at least interferon α subtype α1, α2, α4, α5, α6, α7, α8, α10, α13, α16, α17, and α21. In one embodiment, the anti-interferon α antibodies selectively bind to at least interferon α subtype α1, α2, α4, α5, α6, α7, α8, α10, α13, α17, and α21. In one embodiment, the anti-interferon α antibodies selectively bind to at least interferon α subtype α1, α2, α4, α5, α6, α7, α8, α 10, α13, and α21. In one embodiment, the anti-interferon α antibodies selectively bind to at least interferon α subtype α1, α2, α4, α5, α6, α7, α8, α10, and α13.

In one embodiment, the anti-interferon α antibodies selectively bind to at least interferon α subtype α1, α2, α4, α5, α6, α7, α8, α10, α13, α14, α16, α17, and α21. In one embodiment, the anti-interferon α antibodies selectively bind to at least interferon α subtype α1, α2, α4, α5, α6, α7, α8, α10, α13, α14, α17, and α21. In one embodiment, the anti-interferon α antibodies selectively bind to at least interferon α subtype α1, α2, α4, α5, α6, α7, α8, α10, α13, α14, and α21. In one embodiment, the anti-interferon α antibodies selectively bind to at least interferon α subtype α1, α2, α4, α5, α6, α7, α8, α10, α13, and α14. In one embodiment, the anti-interferon α antibodies selectively bind to at least interferon α subtype α1, α2, α4, α5, α6, α7, α8, α10, α13, α14, α16, and α21. In one embodiment, the anti-interferon α antibodies selectively bind to at least interferon α subtype α1, α2, α4, α5, α6, α7, α8, α10, α13, α14, and α16.

In one embodiment, the anti-interferon α antibodies selectively bind to at least interferon α subtype α1, α2, α4, α5, α8, α10, and α21.

In one embodiment, the anti-interferon α antibodies selectively bind to at least interferon α subtype α1. In one embodiment, the anti-interferon α antibodies selectively bind to at least interferon α subtype α2. In one embodiment, the anti-interferon α antibodies selectively bind to at least interferon α subtype α4. In one embodiment, the anti-interferon α antibodies selectively bind to at least interferon α subtype α5. In one embodiment, the anti-interferon α antibodies selectively bind to at least interferon α subtype α6. In one embodiment, the anti-interferon α antibodies selectively bind to at least interferon α subtype α7. In one embodiment, the anti-interferon α antibodies selectively bind to at least interferon α subtype α8. In one embodiment, the anti-interferon α antibodies selectively bind to at least interferon α subtype α10. In one embodiment, the anti-interferon α antibodies selectively bind to at least interferon α subtype α13. In one embodiment, the anti-interferon α antibodies selectively bind to at least interferon α subtype α14. In one embodiment, the anti-interferon α antibodies selectively bind to at least interferon α subtype α16. In one embodiment, the anti-interferon α antibodies selectively bind to at least interferon α subtype α17. In one embodiment, the anti-interferon α antibodies selectively bind to at least interferon α subtype α21.

In certain embodiments, it will be desirable to have the anti-IFNα antibodies of the invention selectively bind to and neutralize specific IFNα subtypes, or combinations of IFNα subtypes. Interferon neutralization assays are well known in the art. In addition the assay disclosed in Example 1 herein, see also, for example, assays disclosed in U.S. Patent No. 7,087,726 (for example, columns 29-32), U.S. Patent No. 7,179,465, U.S. Patent Application Publication No. 2006/0029601, or in U.S. Patent Application Publication No. 2007/0014724 A1 (for example, paragraphs 315-316).

Accordingly, in one embodiment, the anti-interferon α antibodies selectively bind to and neutralize at least interferon α subtype α2, which may include the α2a and/or α2b subtypes. In another embodiment, the anti-interferon α antibodies selectively bind to and neutralize at least interferon α subtype α1, α2, α4, α5, α6, α7, α8, α10, α13, α14, α16, α17, or α21.

In one embodiment, the anti-interferon α antibodies selectively bind to and neutralize at least interferon α subtype α1, α2, α4, α5, α6, α7, α8, α10, α13, α14, α16, α17, and α21. In one embodiment, the anti-interferon α antibodies selectively bind to and neutralize at least interferon α subtype α1, α2, α4, α5, α6, α7, α8, α 10, α13, α14, α16, and α17. In one embodiment, the anti-interferon α antibodies selectively bind to and neutralize at least interferon α subtype α1, α2, α4, α5, α6, α7, α8, α10, α13, α14, and α16. In one embodiment, the anti-interferon α antibodies selectively bind to and neutralize at least interferon α subtype α1, α2, α4, α5, α6, α7, α8, α10, α13, and α14. In one embodiment, the anti-interferon α antibodies selectively bind to and neutralize at least interferon α subtype α1, α2, α4, α5, α6, α7, α8, α10, and α13. In one embodiment, the anti-interferon α antibodies selectively bind to and neutralize at least interferon α subtype α1, α2, α4, α5, α6, α7, α8, and α10. In one embodiment, the anti-interferon α antibodies selectively bind to and neutralize at least interferon α subtype α1, α2, α4, α5, α6, α7, and α8. In one embodiment, the anti-interferon α antibodies selectively bind to and neutralize at least interferon α subtype α1, α2, α4, α5, α6, and α7. In one embodiment, the anti-interferon α antibodies selectively bind to and neutralize at least interferon α subtype α1, α2, α4, α5, and α6. In one embodiment, the anti-interferon α antibodies selectively bind to and neutralize at least interferon α subtype α1, α2, α4, and α5. In one embodiment, the anti-interferon α antibodies selectively bind to and neutralize at least interferon α subtype α1, α2, and α4. In one embodiment, the anti-interferon α antibodies selectively bind to and neutralize at least interferon α subtype α1 and α2. In one embodiment, the anti-interferon α antibodies selectively bind to and neutralize at least interferon α subtype and α1.

In one embodiment, the anti-interferon α antibodies selectively bind to and neutralize at least interferon α subtype α2, α4, α5, α6, α7, α8, α10, α13, α14, α16, α17, and α21. In one embodiment, the anti-interferon α antibodies selectively bind to and neutralize at least interferon α subtype α4, α5, α6, α7, α8, α10, α13, α14, α16, α17, and α21. In one embodiment, the anti-interferon α antibodies selectively bind to and neutralize at least interferon α subtype α5, α6, α7, α8, α10, α13, α14, α16, α17, and α21. In one embodiment, the anti-interferon α antibodies selectively bind to and neutralize at least interferon α subtype α6, α7, α8, α10, α13, α14, α16, α17, and α21. In one embodiment, the anti-interferon α antibodies selectively bind to and neutralize at least interferon α subtype α7, α8, α10, α13, α14, α16, α17, and α21. In one embodiment, the anti-interferon α antibodies selectively bind to and neutralize at least interferon α subtype α8, α10, α13, α14, α16, α17, and α21. In one embodiment, the anti-interferon α antibodies selectively bind to and neutralize at least interferon α subtype α10, α13, α14, α16, α17, and α21. In one embodiment, the anti-interferon α antibodies selectively bind to and neutralize at least interferon α subtype α13, α14, α16, α17, and α21. In one embodiment, the anti-interferon α antibodies selectively bind to and neutralize at least interferon α subtype α14, α16, α17, and α21. In one embodiment, the anti-interferon α antibodies selectively bind to and neutralize at least interferon α subtype α16, α17, and α21. In one embodiment, the anti-interferon α antibodies selectively bind to and neutralize at least interferon α subtype α17 and α21. In one embodiment, the anti-interferon α antibodies selectively bind to and neutralize at least interferon α subtype α21.

In one embodiment, the anti-interferon α antibodies selectively bind to and neutralize at least interferon α subtype α1, α4, α5, α6, α7, α8, α10, α13, α14, α16, α17, and α21. In one embodiment, the anti-interferon α antibodies selectively bind to and neutralize at least interferon α subtype α1, α2, α5, α6, α7, α8, α10, α13, α14, α16, α17, and α21. In one embodiment, the anti-interferon α antibodies selectively bind to and neutralize at least interferon α subtype α1, α2, α4, α6, α7, α8, α10, α13, α14, α16, α17, and α21. In one embodiment, the anti-interferon α antibodies selectively bind to and neutralize at least interferon α subtype α1, α2, α4, α5, α7, α8, α10, α13, α14, α16, α17, and α21. In one embodiment, the anti-interferon α antibodies selectively bind to and neutralize at least interferon α subtype α1, α2, α4, α5, α6, α8, α10, α13, α14, α16, α17, and α21. In one embodiment, the anti-interferon α antibodies selectively bind to and neutralize at least interferon α subtype α1, α2, α4, α5, α6, α7, α10, α13, α14, α16, α17, and α21. In one embodiment, the anti-interferon α antibodies selectively bind to and neutralize at least interferon α subtype α1, α2, α4, α5, α6, α7, α8, α13, α14, α16, α17, and α21. In one embodiment, the anti-interferon α antibodies selectively bind to and neutralize at least interferon α subtype α1, α2, α4, α5, α6, α7, α8, α10, α14, α16, α17, and α21. In one embodiment, the anti-interferon α antibodies selectively bind to and neutralize at least interferon α subtype α1, α2, α4, α5, α6, α7, α8, α10, α13, α16, α17, and α21. In one embodiment, the anti-interferon α antibodies selectively bind to and neutralize at least interferon α subtype α1, α2, α4, α5, α6, α7, α8, α10, α13, α14, α17, and α21. In one embodiment, the anti-interferon α antibodies selectively bind to and neutralize at least interferon α subtype α1, α2, α4, α5, α6, α7, α8, α10, α13, α14, α16, and α21.

In one embodiment, the anti-interferon α antibodies selectively bind to and neutralize at least interferon α subtype α1, α4, α5, α6, α7, α8, α10, α13, α14, α16, α17, and α21. In one embodiment, the anti-interferon α antibodies selectively bind to and neutralize at least interferon α subtype α1, α5, α6, α7, α8, α10, α13, α14, α16, α17, and α21. In one embodiment, the anti-interferon α antibodies selectively bind to and neutralize at least interferon α subtype α1, α6, α7, α8, α10, α13, α14, α16, α17, and α21. In one embodiment, the anti-interferon α antibodies selectively bind to and neutralize at least interferon α subtype α1, α7, α8, α10, α13, α14, α16, α17, and α21. In one embodiment, the anti-interferon α antibodies selectively bind to and neutralize at least interferon α subtype α1, α8, α10, α13, α14, α16, α17, and α21. In one embodiment, the anti-interferon α antibodies selectively bind to and neutralize at least interferon α subtype α1, α10, α13, α14, α16, α17, and α21. In one embodiment, the anti-interferon α antibodies selectively bind to and neutralize at least interferon α subtype α1, α13, α14, α16, α17, and α21. In one embodiment, the anti-interferon α antibodies selectively bind to and neutralize at least interferon α subtype α1, α14, α16, α17, and α21. In one embodiment, the anti-interferon α antibodies selectively bind to and neutralize at least interferon α subtype α1, α16, α17, and α21. In one embodiment, the anti-interferon α antibodies selectively bind to and neutralize at least interferon α subtype α1, α17, and α21. In one embodiment, the anti-interferon α antibodies selectively bind to and neutralize at least interferon α subtype α1 and α21.

In one embodiment, the anti-interferon α antibodies selectively bind to and neutralize at least interferon α subtype α1, α2, α6, α7, α8, α10, α13, α14, α16, α17, and α21. In one embodiment, the anti-interferon α antibodies selectively bind to and neutralize at least interferon α subtype α1, α2, α7, α8, α10, α13, α14, α16, α17, and α21. In one embodiment, the anti-interferon α antibodies selectively bind to and neutralize at least interferon α subtype α1, α2, α8, α10, α13, α14, α16, α17, and α21. In one embodiment, the anti-interferon α antibodies selectively bind to and neutralize at least interferon α subtype α1, α2, α10, α13, α14, α16, α17, and α21. In one embodiment, the anti-interferon α antibodies selectively bind to and neutralize at least interferon α subtype α1, α2, α13, α14, α16, α17, and α21. In one embodiment, the anti-interferon α antibodies selectively bind to and neutralize at least interferon α subtype α1, α2, α14, α16, α17, and α21. In one embodiment, the anti-interferon α antibodies selectively bind to and neutralize at least interferon α subtype α1, α2, α16, α17, and α21. In one embodiment, the anti-interferon α antibodies selectively bind to and neutralize at least interferon α subtype α1, α2, α17, and α21. In one embodiment, the anti-interferon α antibodies selectively bind to and neutralize at least interferon α subtype α1, α2, and α21. In one embodiment, the anti-interferon α antibodies selectively bind to and neutralize at least interferon α subtype α1 and α2.

In one embodiment, the anti-interferon α antibodies selectively bind to and neutralize at least interferon α subtype α1, α2, α4, α6, α7, α8, α10, α13, α14, α16, α17, and α21. In one embodiment, the anti-interferon α antibodies selectively bind to and neutralize at least interferon α subtype α1, α2, α4, α7, α8, α10, α13, α14, α16, α17, and α21. In one embodiment, the anti-interferon α antibodies selectively bind to and neutralize at least interferon α subtype α1, α2, α4, α8, α10, α13, α14, α16, α17, and α21. In one embodiment, the anti-interferon α antibodies selectively bind to and neutralize at least interferon α subtype α1, α2, α4, α10, α13, α14, α16, α17, and α21. In one embodiment, the anti-interferon α antibodies selectively bind to and neutralize at least interferon α subtype α1, α2, α4, α13, α14, α16, α17, and α21. In one embodiment, the anti-interferon α antibodies selectively bind to and neutralize at least interferon α subtype α1, α2, α4, α14, α16, α17, and α21. In one embodiment, the anti-interferon α antibodies selectively bind to and neutralize at least interferon α subtype α1, α2, α4, α16, α17, and α21. In one embodiment, the anti-interferon α antibodies selectively bind to and neutralize at least interferon α subtype α1, α2, α4, α17, and α21. In one embodiment, the anti-interferon α antibodies selectively bind to and neutralize at least interferon α subtype α1, α2, α4, and α21. In one embodiment, the anti-interferon α antibodies selectively bind to and neutralize at least interferon α subtype α1, α2, and α4.

In one embodiment, the anti-interferon α antibodies selectively bind to and neutralize at least interferon α subtype α1, α2, α4, α5, α7, α8, α10, α13, α14, α16, α17, and α21. In one embodiment, the anti-interferon α antibodies selectively bind to and neutralize at least interferon α subtype α1, α2, α4, α5, α8, α10, α13, α14, α16, α17, and α21. In one embodiment, the anti-interferon α antibodies selectively bind to and neutralize at least interferon α subtype α1, α2, α4, α5, α10, α13, α14, α16, α17, and α21. In one embodiment, the anti-interferon α antibodies selectively bind to and neutralize at least interferon α subtype α1, α2, α4, α5, α13, α14, α16, α17, and α21. In one embodiment, the anti-interferon α antibodies selectively bind to and neutralize at least interferon α subtype α1, α2, α4, α5, α14, α16, α17, and α21. In one embodiment, the anti-interferon α antibodies selectively bind to and neutralize at least interferon α subtype α1, α2, α4, α5, α16, α17, and α21. In one embodiment, the anti-interferon α antibodies selectively bind to and neutralize at least interferon α subtype α1, α2, α4, α5, α17, and α21. In one embodiment, the anti-interferon α antibodies selectively bind to and neutralize at least interferon α subtype α1, α2, α4, α5, and α21. In one embodiment, the anti-interferon α antibodies selectively bind to and neutralize at least interferon α subtype α1, α2, α4, and α5.

In one embodiment, the anti-interferon α antibodies selectively bind to and neutralize at least interferon α subtype α1, α2, α4, α5, α6, α8, α10, α13, α14, α16, α17, and α21. In one embodiment, the anti-interferon α antibodies selectively bind to and neutralize at least interferon α subtype α1, α2, α4, α5, α6, α10, α13, α14, α16, α17, and α21. In one embodiment, the anti-interferon α antibodies selectively bind to and neutralize at least interferon α subtype α1, α2, α4, α5, α6, α13, α14, α16, α17, and α21. In one embodiment, the anti-interferon α antibodies selectively bind to and neutralize at least interferon α subtype α1, α2, α4, α5, α6, α14, α16, α17, and α21. In one embodiment, the anti-interferon α antibodies selectively bind to and neutralize at least interferon α subtype α1, α2, α4, α5, α6, α16, α17, and α21. In one embodiment, the anti-interferon α antibodies selectively bind to and neutralize at least interferon α subtype α1, α2, α4, α5, α6, α17, and α21. In one embodiment, the anti-interferon α antibodies selectively bind to and neutralize at least interferon α subtype α1, α2, α4, α5, α6, and α21. In one embodiment, the anti-interferon α antibodies selectively bind to and neutralize at least interferon α subtype α1, α2, α4, α5, and α6.

In one embodiment, the anti-interferon α antibodies selectively bind to and neutralize at least interferon α subtype α1, α2, α4, α5, α6, α7, α10, α13, α14, α16, α17, and α21. In one embodiment, the anti-interferon α antibodies selectively bind to and neutralize at least interferon α subtype α1, α2, α4, α5, α6, α7, α13, α14, α16, α17, and α21. In one embodiment, the anti-interferon α antibodies selectively bind to and neutralize at least interferon α subtype α1, α2, α4, α5, α6, α7, α14, α16, α17, and α21. In one embodiment, the anti-interferon α antibodies selectively bind to and neutralize at least interferon α subtype α1, α2, α4, α5, α6, α7, α16, α17, and α21. In one embodiment, the anti-interferon α antibodies selectively bind to and neutralize at least interferon α subtype α1, α2, α4, α5, α6, α7, α17, and α21. In one embodiment, the anti-interferon α antibodies selectively bind to and neutralize at least interferon α subtype α1, α2, α4, α5, α6, α7, and α21. In one embodiment, the anti-interferon α antibodies selectively bind to and neutralize at least interferon α subtype α1, α2, α4, α5, α6, and α7.

In one embodiment, the anti-interferon α antibodies selectively bind to and neutralize at least interferon α subtype α1, α2, α4, α5, α6, α7, α8, α13, α14, α16, α17, and α21. In one embodiment, the anti-interferon α antibodies selectively bind to and neutralize at least interferon α subtype α1, α2, α4, α5, α6, α7, α8, α14, α16, α17, and α21. In one embodiment, the anti-interferon α antibodies selectively bind to and neutralize at least interferon α subtype α1, α2, α4, α5, α6, α7, α8, α16, α17, and α21. In one embodiment, the anti-interferon α antibodies selectively bind to and neutralize at least interferon α subtype α1, α2, α4, α5, α6, α7, α8, α17, and α21. In one embodiment, the anti-interferon α antibodies selectively bind to and neutralize at least interferon α subtype α1, α2, α4, α5, α6, α7, α8, and α21. In one embodiment, the anti-interferon α antibodies selectively bind to and neutralize at least interferon α subtype α1, α2, α4, α5, α6, α7, and α8.

In one embodiment, the anti-interferon α antibodies selectively bind to and neutralize at least interferon α subtype α1, α2, α4, α5, α6, α7, α8, α10, α14, α16, α17, and α21. In one embodiment, the anti-interferon α antibodies selectively bind to and neutralize at least interferon α subtype α1, α2, α4, α5, α6, α7, α8, α10, α16, α17, and α21. In one embodiment, the anti-interferon α antibodies selectively bind to and neutralize at least interferon α subtype α1, α2, α4, α5, α6, α7, α8, α10, α17, and α21. In one embodiment, the anti-interferon α antibodies selectively bind to and neutralize at least interferon α subtype α1, α2, α4, α5, α6, α7, α8, α10, and α21. In one embodiment, the anti-interferon α antibodies selectively bind to and neutralize at least interferon α subtype α1, α2, α4, α5, α6, α7, α8, and α10.

In one embodiment, the anti-interferon α antibodies selectively bind to and neutralize at least interferon α subtype α1, α2, α4, α5, α6, α7, α8, α10, α13, α16, α17, and α21. In one embodiment, the anti-interferon α antibodies selectively bind to and neutralize at least interferon α subtype α1, α2, α4, α5, α6, α7, α8, α10, α13, α17, and α21. In one embodiment, the anti-interferon α antibodies selectively bind to and neutralize at least interferon α subtype α1, α2, α4, α5, α6, α7, α8, α10, α13, and α21. In one embodiment, the anti-interferon α antibodies selectively bind to and neutralize at least interferon α subtype α1, α2, α4, α5, α6, α7, α8, α10, and α13.

In one embodiment, the anti-interferon α antibodies selectively bind to and neutralize at least interferon α subtype α1, α2, α4, α5, α6, α7, α8, α10, α13, α14, α16, α17, and α21. In one embodiment, the anti-interferon α antibodies selectively bind to and neutralize at least interferon α subtype α1, α2, α4, α5, α6, α7, α8, α10, α13, α14, α17, and α21. In one embodiment, the anti-interferon α antibodies selectively bind to and neutralize at least interferon α subtype α1, α2, α4, α5, α6, α7, α8, α10, α13, α14, and α21. In one embodiment, the anti-interferon α antibodies selectively bind to and neutralize at least interferon α subtype α1, α2, α4, α5, α6, α7, α8, α10, α13, and α14. In one embodiment, the anti-interferon α antibodies selectively bind to and neutralize at least interferon α subtype α1, α2, α4, α5, α6, α7, α8, α10, α13, α14, α16, and α21. In one embodiment, the anti-interferon α antibodies selectively bind to and neutralize at least interferon α subtype α1, α2, α4, α5, α6, α7, α8, α10, α13, α14, and α16.

In one embodiment, the anti-interferon α antibodies selectively bind to and neutralize at least interferon α subtype α1, α2, α4, α5, α8, α10, and α21.

In one embodiment, the anti-interferon α antibodies selectively bind to and neutralize at least interferon α subtype α1. In one embodiment, the anti-interferon α antibodies selectively bind to and neutralize at least interferon α subtype α2. In one embodiment, the anti-interferon α antibodies selectively bind to and neutralize at least interferon α subtype α4. In one embodiment, the anti-interferon α antibodies selectively bind to and neutralize at least interferon α subtype α5. In one embodiment, the anti-interferon α antibodies selectively bind to and neutralize at least interferon α subtype α6. In one embodiment, the anti-interferon α antibodies selectively bind to and neutralize at least interferon α subtype α7. In one embodiment, the anti-interferon α antibodies selectively bind to and neutralize at least interferon α subtype α8. In one embodiment, the anti-interferon α antibodies selectively bind to and neutralize at least interferon α subtype α10. In one embodiment, the anti-interferon α antibodies selectively bind to and neutralize at least interferon α subtype α13. In one embodiment, the anti-interferon α antibodies selectively bind to and neutralize at least interferon α subtype α14. In one embodiment, the anti-interferon α antibodies selectively bind to and neutralize at least interferon α subtype α16. In one embodiment, the anti-interferon α antibodies selectively bind to and neutralize at least interferon α subtype α17. In one embodiment, the anti-interferon α antibodies selectively bind to and neutralize at least interferon α subtype α21.

### MEDI-545

MEDI-545 is a fully human, 147,000 Dalton IgG1k monoclonal antibody (Mab) that binds to a majority of interferon-alpha (IFN-α) subtypes. MEDI-545 is made from 100% human protein sequences, thereby making it a fully human monoclonal antibody. Fully human monoclonal antibodies may have advantages over other forms of monoclonal antibodies, such as chimeric and humanized antibodies, as they may have a more favorable safety profile and may be eliminated less rapidly from the human body, thereby possibly reducing the frequency of dosing. MEDI-545 was derived from an IgG4κ antibody, 13H5, which was selected based on functional assays as having the most desirable properties for a potential therapeutic agent. 13H5 was subsequently converted to an IgG1 antibody isotype, produced in CHO cells, and selected for further characterization and preclinical development with an initial designation of MDX-1103, now referred to as MEDI-545. See also U.S. Patent Application Publication No. 2007/0014724, U.S. Provisional Application No. 60/909,232 entitled "Antibodies with Decreased Deamidation Profiles," U.S. Provisional Application No. 60/909,117 entitled "Antibody Formulation," U.S. Provisional Application Serial No. 60/873,008 filed December 6, 2006, U.S. Provisional Application Serial No. 60/907,762 filed April 16, 2007, U.S. Provisional Application Serial No. 60/924,219 filed May 3, 2007, U.S. Provisional Application Serial No. 60/924,584 filed May 21, 2007, U.S. Provisional Application Serial No. 60/960,187 filed September 19, 2007, U.S. Provisional Application Serial No. 60/996,176 filed November 5, 2007, International Patent Application No. PCT________entitled "Interferon Alpha-induced Pharmacodynamic Markers" filed December 6, 2007 (Attorney docket no. IA200PCT), U.S. Provisional Application Serial No. 60/924,220 filed May 3, 2007, U.S. Provisional Application entitled "Auto-Antibody Markers of Autoimmune Disease" filed November 6, 2007 (Attorney docket no. IA201P2), U.S. Provisional Application entitled "Auto-Antibody Markers of Autoimmune Disease" filed December 6, 2007 (Attorney docket no. IA201P3) and U.S. Provisional Application No. ______ filed on November 5, 2007 (Attorney docket no. IA220P1) and entitled "Methods of Treating Scleroderma," each of which is hereby incorporated herein by reference.

MEDI-545 was initially evaluated in systemic lupus erythematosus (SLE) patients. SLE is a prototypic systemic autoimmune disease with protean manifestations in which current treatment remains unsatisfactory. A body of evidence suggests that SLE patients have elevated levels of IFN-α that appear to be related to clinical activity.

In one embodiment, the anti-inteferon α antibody to be used in the invention is not MEDI-545.

In certain embodiments, it may be desirable to alter the half-life of the anti-interferon α antibodies. In one embodiment, it may be desirable to decrease the in vivo half-life of the antibodies. In another embodiment, it may be desirable to increase the in vivo half-life of the antibodies. See, for example, U.S. Patent Application Publication No. 2006/0198840 A1.

### Methods of Producing Antibodies

The antibodies or fragments thereof can be produced by any method known in the art for the synthesis of antibodies, in particular, by chemical synthesis or by recombinant expression techniques.

Polyclonal antibodies to IFN alpha can be produced by various procedures well known in the art. For example, IFN alpha or immunogenic fragments thereof can be administered to various host animals including, but not limited to, rabbits, mice, rats, etc. to induce the production of sera containing polyclonal antibodies specific for IFN alpha. Various adjuvants may be used to increase the immunological response, depending on the host species, and include but are not limited to, Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (bacille Calmette-Guerin) and corynebacterium parvum. Such adjuvants are also well known in the art.

Monoclonal antibodies can be prepared using a wide variety of techniques known in the art including the use of hybridoma, recombinant, and phage display technologies, or a combination thereof. For example, monoclonal antibodies can be produced using hybridoma techniques including those known in the art and taught, for example, in Harlow et al., Antibodies: A Laboratory Manual, (Cold Spring Harbor Laboratory Press, 2nd ed. 1988); Hammerling, et al., in: Monoclonal Antibodies and T-Cell Hybridomas 563-681 (Elsevier, N.Y., 1981) (said references incorporated by reference in their entireties). The term "monoclonal antibody" as used herein is not limited to antibodies produced through hybridoma technology. The term "monoclonal antibody" refers to an antibody that is derived from a single clone, including any eukaryotic, prokaryotic, or phage clone, and not the method by which it is produced.

Methods for producing and screening for specific antibodies using hybridoma technology are routine and well known in the art. Briefly, mice can be immunized with IFN alpha and once an immune response is detected, for example, antibodies specific for IFN alpha are detected in the mouse serum, the mouse spleen is harvested and splenocytes isolated. The splenocytes are then fused by well known techniques to any suitable myeloma cells, for example cells from cell line SP20 available from the ATCC. Hybridomas are selected and cloned by limited dilution. The hybridoma clones are then assayed by methods known in the art for cells that secrete antibodies capable of binding a polypeptide of the invention. Ascites fluid, which generally contains high levels of antibodies, can be generated by immunizing mice with positive hybridoma clones.

Accordingly, monoclonal antibodies can be generated by culturing a hybridoma cell secreting an antibody of the invention wherein, preferably, the hybridoma is generated by fusing splenocytes isolated from a mouse immunized with IFN alpha with myeloma cells and then screening the hybridomas resulting from the fusion for hybridoma clones that secrete an antibody able to bind IFN alpha.

Antibody fragments which recognize specific IFN alpha epitopes may be generated by any technique known to those of skill in the art. For example, Fab and F(ab')2 fragments of the invention may be produced by proteolytic cleavage of immunoglobulin molecules, using enzymes such as papain (to produce Fab fragments) or pepsin (to produce F(ab')2 fragments). F(ab')2 fragments contain the variable region, the light chain constant region and the CH1 domain of the heavy chain. Further, the antibodies of the present invention can also be generated using various phage display methods known in the art.

In phage display methods, functional antibody domains are displayed on the surface of phage particles which carry the polynucleotide sequences encoding them. In particular, DNA sequences encoding VH and VL domains are amplified from animal cDNA libraries (for example, human or murine cDNA libraries of lymphoid tissues). The DNA encoding the VH and VL domains are recombined together with an scFv linker by PCR and cloned into a phagemid vector (for example, p CANTAB 6 or pcomb 3 HSS). The vector is electroporated in E. coli and the E. coli is infected with helper phage. Phage used in these methods are typically filamentous phage including fd and M13 and the VH and VL domains are usually recombinantly fused to either the phage gene III or gene VIII. Phage expressing an antigen binding domain that binds to the IFN alpha epitope of interest can be selected or identified with antigen, for example, using labeled antigen or antigen bound or captured to a solid surface or bead. Examples of phage display methods that can be used to make the antibodies of the present invention include those disclosed in Brinkman et al., 1995, J. Immunol. Methods 182:41-50; Ames et al., 1995, J. Immunol. Methods 184:177-186; Kettleborough et al., 1994, Eur. J. Immunol. 24:952-958; Persic et al., 1997, Gene 187:9-18; Burton et al., 1994, Advances in Immunology 57:191-280; International Application No. PCT/GB91/01134; International Publication Nos. WO 90/02809, WO 91/10737, WO 92/01047, WO 92/18619, WO 93/11236, WO 95/15982, WO 95/20401, and WO97/13844; and U.S. Pat. Nos. 5,698,426, 5,223,409, 5,403,484, 5,580,717, 5,427,908, 5,750,753, 5,821,047, 5,571,698, 5,427,908, 5,516,637, 5,780,225, 5,658,727, 5,733,743 and 5,969,108; each of which is incorporated herein by reference in its entirety.

As described in the above references, after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, for example, as described below. Techniques to recombinantly produce Fab, Fab' and F(ab')2 fragments can also be employed using methods known in the art such as those disclosed in International Publication No. WO 92/22324; Mullinax et al., 1992, BioTechniques 12(6):864-869; Sawai et al., 1995, AJRI 34:26-34; and Better et al., 1988, Science 240:1041-1043 (said references incorporated by reference in their entireties).

To generate whole antibodies, PCR primers including VH or VL nucleotide sequences, a restriction site, and a flanking sequence to protect the restriction site can be used to amplify the VH or VL sequences in scFv clones. Utilizing cloning techniques known to those of skill in the art, the PCR amplified VH domains can be cloned into vectors expressing a VH constant region, for example the human gamma 4 constant region, and the PCR amplified VL domains can be cloned into vectors expressing a VL constant region, for example, human kappa or lamba constant regions. Preferably, the vectors for expressing the VH or VL domains comprise an EF-1α promoter, a secretion signal, a cloning site for the variable domain, constant domains, and a selection marker such as neomycin. The VH and VL domains may also be cloned into one vector expressing the necessary constant regions. The heavy chain conversion vectors and light chain conversion vectors are then co-transfected into cell lines to generate stable or transient cell lines that express full-length antibodies, for example, IgG, using techniques known to those of skill in the art.

For some uses, including in vivo use of antibodies in humans and in vitro detection assays, it may be preferable to use human or chimeric antibodies. Completely human antibodies are particularly desirable for therapeutic treatment of human subjects. Human antibodies can be made by a variety of methods known in the art including phage display methods described above using antibody libraries derived from human immunoglobulin sequences. See also U.S. Pat. Nos. 4,444,887 and 4,716,111; and International Publication Nos. WO 98/46645, WO 98/50433, WO 98/24893, WO98/16654, WO 96/34096, WO 96/33735, and WO 91/10741; each of which is incorporated herein by reference in its entirety.

Human antibodies can also be produced using transgenic mice which are incapable of expressing functional endogenous immunoglobulins, but which can express human immunoglobulin genes. For example, the human heavy and light chain immunoglobulin gene complexes may be introduced randomly or by homologous recombination into mouse embryonic stem cells. Alternatively, the human variable region, constant region, and diversity region may be introduced into mouse embryonic stem cells in addition to the human heavy and light chain genes. The mouse heavy and light chain immunoglobulin genes may be rendered non-functional separately or simultaneously with the introduction of human immunoglobulin loci by-homologous recombination. In particular, homozygous deletion of the JH region prevents endogenous antibody production. The modified embryonic stem cells are expanded and microinjected into blastocysts to produce chimeric mice. The chimeric mice are then bred to produce homozygous offspring which express human antibodies. The transgenic mice are immunized in the normal fashion with a selected antigen, for example, all or a portion of a polypeptide of the invention. Monoclonal antibodies directed against the antigen can be obtained from the immunized, transgenic mice using conventional hybridoma technology. The human immunoglobulin transgenes harbored by the transgenic mice rearrange during B cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg and Huszar (1995, Int. Rev. Immunol. 13:65-93). For a detailed discussion of this technology for producing human antibodies and human monoclonal antibodies and protocols for producing such antibodies, see, for example, International Publication Nos. WO 98/24893, WO 96/34096, and WO 96/33735; and U.S. Pat. Nos. 5,413,923, 5,625,126, 5,633,425, 5,569,825, 5,661,016, 5,545,806, 5,814,318, and 5,939,598, which are incorporated by reference herein in their entirety. In addition, companies such as Abgenix, Inc. (Freemont, Calif.), Genpharm (San Jose, Calif.) and Medarex (Princeton, N.J.) can be engaged to provide human antibodies directed against a selected antigen using technology similar to that described above.

A chimeric antibody is a molecule in which different portions of the antibody are derived from different immunoglobulin molecules. Methods for producing chimeric antibodies are known in the art. See for example, Morrison, 1985, Science 229:1202; Oi et al., 1986, BioTechniques 4:214; Gillies et al., 1989, J. Immunol. Methods 125:191-202; and U.S. Pat. Nos. 5,807,715, 4,816,567, 4,816,397, and 6,311,415, which are incorporated herein by reference in their entirety.

A humanized antibody is an antibody or its variant or fragment thereof which is capable of binding to a predetermined antigen and which comprises a framework region having substantially the amino acid sequence of a human immunoglobulin and a CDR having substantially the amino acid sequence of a non-human immuoglobulin. A humanized antibody comprises substantially all of at least one, and typically two, variable domains (Fab, Fab', F(ab')2, Fabc, Fv) in which all or substantially all of the CDR regions correspond to those of a non-human immunoglobulin (donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. Ordinarily, the antibody will contain both the light chain as well as at least the variable domain of a heavy chain. The antibody also may include the CH1, hinge, CH2, CH3, and CH4 regions of the heavy chain. The humanized antibody can be selected from any class of immunoglobulins, including IgM, IgG, IgD; IgA and IgE, and any isotype, including IgG1, IgG2, IgG3 and IgG4. Usually the constant domain is a complement fixing constant domain where it is desired that the humanized antibody exhibit cytotoxic activity, and the class is typically IgG1. Where such cytotoxic activity is not desirable, the constant domain may be of the IgG2 class. The humanized antibody may comprise sequences from more than one class or isotype, and selecting particular constant domains to optimize desired effector functions is within the ordinary skill in the art. The framework and CDR regions of a humanized antibody need not correspond precisely to the parental sequences, for example, the donor CDR or the consensus framework may be mutagenized by substitution, insertion or deletion of at least one residue so that the CDR or framework residue at that site does not correspond to either the consensus or the import antibody. Such mutations, however, will not be extensive. Usually, at least 75% of the humanized antibody residues will correspond to those of the parental framework region (FR) and CDR sequences, more often 90%, and most preferably greater than 95%. Humanized antibody can be produced using variety of techniques known in the art, including but not limited to, CDR-grafting (European Patent No. EP 239,400; International Publication No. WO 91/09967; and U.S. Pat. Nos. 5,225,539, 5,530,101, and 5,585,089), veneering or resurfacing (European Patent Nos. EP 592,106 and EP 519,596; Padlan, 1991, Molecular Immunology 28(4/5):489-498; Studnicka et al., 1994, Protein Engineering 7(6):805-814; and Roguska et al., 1994, PNAS 91:969-973), chain shuffling (U.S. Pat. No. 5,565,332), and techniques disclosed in, for example, U.S. Pat. Nos. 6,407,213, 5,766,886, WO 9317105, Tan et al., J. Immunol. 169:1119-25 (2002), Caldas et al., Protein Eng. 13(5):353-60 (2000), Morea et al., Methods 20(3):267-79 (2000), Baca et al., J. Biol. Chem. 272(16):10678-84 (1997), Roguska et al., Protein Eng. 9(10):895-904 (1996), Couto et al., Cancer Res. 55 (23 Supp):5973s-5977s (1995), Couto et al., Cancer Res. 55(8):1717-22 (1995), Sandhu J S, Gene 150(2):409-10 (1994), and Pedersen et al., J. Mol. Biol. 235(3):959-73 (1994). Often, framework residues in the framework regions will be substituted with the corresponding residue from the CDR donor antibody to alter, preferably improve, antigen binding. These framework substitutions are identified by methods well known in the art, for example, by modeling of the interactions of the CDR and framework residues to identify framework residues important for antigen binding and sequence comparison to identify unusual framework residues at particular positions. (See, for example, Queen et al., U.S. Pat. No. 5,585,089; and Riechmann et al., 1988, Nature 332:323, which are incorporated herein by reference in their entireties).

### Polynucleotides Encoding an Antibody

The methods of the invention also encompass polynucleotides that hybridize under high stringency, intermediate or lower stringency hybridization conditions, to polynucleotides that encode an antibody of the invention.

The polynucleotides may be obtained, and the nucleotide sequence of the polynucleotides determined, by any method known in the art. Since the amino acid sequences of the antibodies are known, nucleotide sequences encoding these antibodies can be determined using methods well known in the art, such as, nucleotide codons known to encode particular amino acids are assembled in such a way to generate a nucleic acid that encodes the antibody or fragment thereof of the invention. Such a polynucleotide encoding the antibody maybe assembled from chemically synthesized oligonucleotides (for example, as described in Kutmejer et al., 1994, BioTechniques 17:242), which, briefly, involves the synthesis of overlapping oligonucleotides containing portions of the sequence encoding the antibody, annealing and ligating of those oligonucleotides, and then amplification of the ligated oligonucleotides by PCR.

Alternatively, a polynucleotide encoding an antibody may be generated from nucleic acid from a suitable source. If a clone containing a nucleic acid encoding a particular antibody is not available, but the sequence of the antibody molecule is known, a nucleic acid encoding the immunoglobulin may be chemically synthesized or obtained from a suitable source (for example, an antibody cDNA library, or a cDNA library generated from, or nucleic acid, preferably poly A+RNA, isolated from, any tissue or cells expressing the antibody, such as hybridoma cells selected to express an antibody of the invention) by PCR amplification using synthetic primers hybridizable to the 3' and 5' ends of the sequence or by cloning using an oligonucleotide probe specific for the particular gene sequence to identify, for example, a cDNA clone from a cDNA library that encodes the antibody. Amplified nucleic acids generated by PCR may then be cloned into replicable cloning vectors using any method well known in the art.

Once the nucleotide sequence of the antibody is determined, the nucleotide sequence of the antibody may be manipulated using methods well known in the art for the manipulation of nucleotide sequences, for example, recombinant DNA techniques, site directed mutagenesis, PCR, etc. (see, for example, the techniques described in Sambrook et al., 1990, Molecular Cloning, A Laboratory Manual, 2d Ed., Cold Spring Harbor Laboratory, Cold Spring Harbor, N.Y. and Ausubel et al., eds., 1998, Current Protocols in Molecular Biology, John Wiley & Sons, NY, which are both incorporated by reference herein in their entireties), to generate antibodies having a different amino acid sequence, for example to create amino acid substitutions, deletions, and/or insertions.

In a specific embodiment, one or more of the CDRs is inserted within framework regions using routine recombinant DNA techniques. The framework regions may be naturally occurring or consensus framework regions, and preferably human framework regions (see, for example, Chothia et al., 1998, J. Mol. Biol. 278: 457-479 for a listing of human framework regions). Preferably, the polynucleotide generated by the combination of the framework regions and CDRs encodes an antibody that specifically binds to IFN alpha. Additionally, one or more amino acid substitutions may be made within the framework regions, and, the amino acid substitutions may improve binding of the antibody to its antigen. Additionally, such methods may be used to make amino acid substitutions or deletions of one or more variable region cysteine residues participating in an intrachain disulfide bond to generate antibody molecules lacking one or more intrachain disulfide bonds. Other alterations to the polynucleotide are encompassed by the present invention and within the skill of the art.

### Recombinant Expression of an Antibody

Recombinant expression of an antibody of the invention, derivative, analog or fragment thereof, (for example, a heavy or light chain of an antibody of the invention or a portion thereof or a single chain antibody of the invention), requires construction of an expression vector containing a polynucleotide that encodes the antibody. Once a polynucleotide encoding an antibody molecule or a heavy or light chain of an antibody, or portion thereof, of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, in vitro recombinant DNA techniques, synthetic techniques, and in vivo genetic recombination. The invention, thus, provides replicable vectors comprising a nucleotide sequence encoding an antibody molecule of the invention, a heavy or light chain of an antibody, a heavy or light chain variable domain of an antibody or a portion thereof, or a heavy or light chain CDR, operably linked to a promoter. Such vectors may include the nucleotide sequence encoding the constant region of the antibody molecule (see, for example, International Publication No. WO 86/05807; International Publication No. WO 89/01036; and U.S. Pat. No. 5,122,464) and the variable domain of the antibody maybe cloned into such a vector for expression of the entire heavy, the entire light chain, or both the entire heavy and light chains.

The expression vector is transferred to a host cell by conventional techniques and the transfected cells are then cultured by conventional techniques to produce an antibody of the invention. Thus, the invention includes host cells containing a polynucleotide encoding an antibody of the invention or fragments thereof, or a heavy or light chain thereof, or portion thereof, or a single chain antibody of the invention, operably linked to a heterologous promoter. In other embodiments for the expression of double-chained antibodies, vectors encoding both the heavy and light chains may be co-expressed in the host cell for expression of the entire immunoglobulin molecule, as detailed below.

A variety of host-expression vector systems may be utilized to express the antibody molecules of the invention (see, for example, U.S. Pat. No. 5,807,715). Such host-expression systems represent vehicles by which the coding sequences of interest may be produced and subsequently purified, but also represent cells which may, when transformed or transfected with the appropriate nucleotide coding sequences, express an antibody molecule of the invention in situ. These include but are not limited to microorganisms such as bacteria (for example, E. coli and B. subtilis) transformed with recombinant bacteriophage DNA, plasmid DNA or cosmid DNA expression vectors containing antibody coding sequences; yeast (for example, Saccharomyces Pichia) transformed with recombinant yeast expression vectors containing antibody coding sequences; insect cell systems infected with recombinant virus expression vectors (for example, baculovirus) containing antibody coding sequences; plant cell systems infected with recombinant virus expression vectors (for example, cauliflower mosaic virus, CaMV; tobacco mosaic virus, TMV) or transformed with recombinant plasmid expression vectors (for example Ti plasmid) containing antibody coding sequences; or mammalian cell systems (for example, COS, CHO, BHK, 293, NS0, and 3T3 cells) harboring recombinant expression constructs containing promoters derived from the genome of mammalian cells (for example, metallothionein promoter) or from mammalian viruses (for example, the adenovirus late promoter; the vaccinia virus 7.5K promoter). For example bacterial cells such as Escherichia coli, and eukaryotic cells, especially for the expression of whole recombinant antibody molecule, are used for the expression of a recombinant antibody molecule. For example, mammalian cells such as Chinese hamster ovary cells (CHO), in conjunction with a vector such as the major intermediate early gene promoter element from human cytomegalovirus is an effective expression system for antibodies (Foecking et al., 1986, Gene 45:101; and Cockett et al., 1990, Bio/Technology 8:2). In a specific embodiment, the expression of nucleotide sequences encoding antibodies or fragments thereof which specifically bind to IFN alpha is regulated by a constitutive promoter, inducible promoter or tissue specific promoter.

In bacterial systems, a number of expression vectors may be advantageously selected depending upon the use intended for the antibody molecule being expressed. For example, when a large quantity of such a protein is to be produced, for the generation of pharmaceutical compositions of an antibody molecule, vectors which direct the expression of high levels of fusion protein products that are readily purified may be desirable. Such vectors include, but are not limited to, the E. coli expression vector pUR278 (Ruther et al., 1983, EMBO 12:1791), in which the antibody coding sequence may be ligated individually into the vector in frame with the lac Z coding region so that a fusion protein is produced; pIN vectors (Inouye & Inouye, 1985, Nucleic Acids Res. 13:3101-3109; Van Heeke & Schuster, 1989, J. Biol. Chem. 24:5503-5509); and the like. pGEX vectors may also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system, Autographa californica nuclear polyhedrosis virus (AcNPV) is used as a vector to express foreign genes. The virus grows in Spodoptera frugiperda cells. The antibody coding sequence may be cloned individually into non-essential regions (for example the polyhedrin gene) of the virus and placed under control of an AcNPV promoter (for example the polyhedrin promoter).

In mammalian host cells, a number of viral-based expression systems may be utilized. In cases where an adenovirus is used as an expression vector, the antibody coding sequence of interest may be ligated to an adenovirus transcription/translation control complex, for example, the late promoter and tripartite leader sequence. This chimeric gene may then be inserted in the adenovirus genome by in vitro or in vivo recombination. Insertion in a non-essential region of the viral genome (for example, region E1 or E3) will result in a recombinant virus that is viable and capable of expressing the antibody molecule in infected hosts (for example, see Logan & Shenk, 1984, Proc. Natl. Acad. Sci. USA 8 1:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see, for example, Bittner et al., 1987, Methods in Enzymol. 153:516-544).

In addition, a host cell strain may be chosen which modulates the expression of the inserted sequences, or modifies and processes the gene product in the specific fashion desired. Such modifications (for example, glycosylation) and processing (for example, cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, W138, BT483, Hs578T, HTB2, BT20 and T47D, NS0, CRL7O3O and HsS78Bst cells.

For long-term, high-yield production of recombinant proteins, stable expression is preferred. For example, cell lines which stably express the antibody molecule may be engineered. Rather than using expression vectors which contain viral origins of replication, host cells can be transformed with DNA controlled by appropriate expression control elements (for example, promoter, enhancer, sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the antibody molecule. Such engineered cell lines may be particularly useful in screening and evaluation of compositions that interact directly or indirectly with the antibody molecule.

A number of selection systems may be used, including but not limited to, the herpes simplex virus thymidine kinase (Wigler et al., 1977, Cell 11:223), hypoxanthineguanine phosphoribosyltransferase (Szybalska & Szybalski, 1992, Proc.Natl. Acad. Sci. USA 48:202), and adenine phosphoribosyltransferase (Lowy et al., 1980, Cell 22:8-17) genes can be employed in tk-, hgprt- or aprt- cells, respectively. Also, antimetabolite resistance can be used as the basis of selection for the following genes: dhfr, which confers resistance to methotrexate (Wigler et al., 1980, Natl. Acad. Sci. USA 77:357; O'Hare et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62: 191-217; May, 1993, TIB TECH 11(5):155-2 15); and hygro, which confers resistance to hygromycin (Santerre et al., 1984, Gene 30:147). Methods commonly known in the art of recombinant DNA technology may be routinely applied to select the desired recombinant clone, and such methods are described, for example, in Ausubel et al. (eds.), Current Protocols in Molecular Biology, John Wiley & Sons, NY (1993); Kriegler, Gene Transfer and Expression, A Laboratory Manual, Stockton Press, NY (1990); and in Chapters 12 and 13, Dracopoli et al. (eds), Current Protocols in Human Genetics, John Wiley & Sons, NY (1994); Colberre-Garapin et al., 1981, J. Mol. Biol. 150: 1, which are incorporated by reference herein in their entireties.

The expression levels of an antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse et al., 1983, Mol. Cell. Biol. 3:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes, and is capable of expressing, both heavy and light chain polypeptides. In such situations, the light chain should be placed before the heavy chain to avoid an excess of toxic free heavy chain (Proudfoot, 1986, Nature 322:52; and Kohler, 1980, Proc. Natl. Acad. Sci. USA 77:2 197). The coding sequences for the heavy and light chains may comprise cDNA or genomic DNA.

Once an antibody molecule of the invention has been produced by recombinant expression, it may be purified by any method known in the art for purification of an immunoglobulin molecule, for example, by chromatography (for example, ion exchange, affinity, particularly by affinity for the specific antigen after Protein A, and sizing column chromatography), centrifugation, differential solubility, or by any other standard technique for the purification of proteins. Further, the antibodies of the present invention or fragments thereof may be fused to heterologous polypeptide sequences described herein or otherwise known in the art to facilitate purification.

### Combination Therapies

In certain embodiments, it may be desired to administer the anti-IFNα antibodies of the invention in combination with additional therapeutics. In one embodiment, the additional therapeutics may be administered prior to administration of the anti-IFNα antibodies of the invention. In another embodiment, the additional therapeutics may be administered after administration of the anti-IFNα antibodies of the invention. In yet another embodiment, the additional therapeutics may be administered at the same time as the administration of the anti-IFNα antibodies of the invention. In certain embodiments, any of the below mentioned additional thereapeutics may be employed and administered in combination with each other and in combination with the anti-IFNα antibodies of the present invention.

### Corticosteroids

In one embodiment, the anti-IFNα antibodies are administered in combination with systemic corticosteroids. Systemic steroids are typically taken by mouth or given by injection as opposed to topical corticosteroids, which are applied directly to the skin. Systemic steroids include, but are not limited to, prednisone, prednisolone, methylprednisolone, betamethasone, dexamethasone, triamcinolone and hydrocortisone. Dosing of systemic corticosteroids can be readily determined by skilled clinicians. For example, in dosing prednisone, a low dose regimen typically consists of, e.g., <10mg/day of prednisone. A medium dose dose prednisone regimen typically consists of, e.g., 10-20 mg/day of prednisone. A high dose prednisone regimen typically consists of, e.g., >20mg/day of prednisone, and sometimes more than 100mg/day. In one embodiment, the anti-IFNα antibodies are administered in combination with <20mg/day of prednisone. In another embodiment, the anti-IFNα antibodies are administered in combination with 20mg/day of prednisone. In another embodiment, the anti-IFNα, antibodies are administered in combination with 10mg/day of prednisone. In another embodiment, the anti-IFNα antibodies are administered in combination with 10mg/day of prednisone.

In another embodiment, the anti-IFNα antibodies are administered in combination with topical corticosteroids. Skin rashes are sometimes treated with topical corticosteroids. These creams are applied to the affected area to reduce the inflammation in your skin cells. Topical corticosteroids are frequently classified as very potent, potent, moderately potent, and mildly potent (sometimes with overlapping classification). Examples of very potent topical corticosteroids include, but are not limited to, betamethasone dipropionate (Diprolene), Clobetasol 17-Propionate 0.05% (Dermovate), Halobetasolpropionate (Ultravate), and Halcinonide 0.1% (Halog). Examples of potent topical corticosteroids include, but are not limited to, Amcinonide 0.1% (Cyclocort), Betamethasone dipropionate 0.5 mg (Diprolene, generics), Betamethasone valerate 0.05% (Betaderm, Celestoderm, Prevex), Desoximetasone 0.25% (Desoxi, Topicort), Diflucortolone valerate 0.1% (Nerisone), Fluocinonlone acetonide 0.25% (Derma, Fluoderm, Synalar), Fluocinonide 0.05% (Lidemol, Lidex, Tydenn, Tiamol, Topsyn), luticasone Propionate (Cutivate), Halcinonide (Halog), and Mometasone furoate 0.1 % (Elocom). Examples of moderately potent topical corticosteroids include, but are not limited to, Betamethasone valerate (Betnovate), Betamethasone valerate (Celestoderm), Clobetasone 17-Butyrate 0.05% (Eumovate), Desonide 0.05% (Desocort), Hydrocortisone acetate 1.0% (Cortef, Hyderm), Hydrocortisone valerate 0.2% (Westcort, Hydroval), Prednicarbate 0.1% (Dermatop), and Triamcinolone Acetonide 0.1% (Kenalog,Traiderm). Examples of mildly potent topical corticosteroids include, but are not limited to, Desonide (Desocort), Hydrocortisone 0.5% (Cortate, Claritin, Cortoderm), and Hydrocortisone Acetate 0.5% (Cortef, Hyderm).

### Nonsteroidal Anti-Inflammatory Drugs (NSAIDS)

In other embodiments, the anti-IFNα antibodies of the invention may be administered in combination with nonsteroidal anti-inflammatory drugs (NSAIDS). These medications are prescribed for a variety of rheumatic diseases, including lupus. Examples of such compounds include acetylsalicylic acid (e.g., aspirin), ibuprofen (Motrin), naproxen (Naprosyn), indomethacin (Indocin), nabumetone (Relafen), tolmetin (Tolectin), and a large number of others readily known to skilled clinicians. These drugs are usually recommended for muscle and joint pain, and arthritis.

In other embodiments, the anti-IFNα antibodies of the invention may be administered in combination with acetaminophen. Although not an NSAID, acetaminophen (e.g., Tylenol) is a mild analgesic that can often be used for pain in the place of NSAIDS. It has the advantage of less stomach irritation than aspirin, but it is not nearly as effective at suppressing inflammation as aspirin.

### Antimalarials

In other embodiments, the anti-IFNα antibodies of the invention may be administered in combination with antimalarials. Although there's no known relationship between lupus and malaria, these medications have proved useful in treating signs and symptoms of lupus. Chloroquine (Aralen) or hydroxychloroquine (Plaquenil), commonly used in the treatment of malaria, may also be very useful in some individuals with lupus. They are most often prescribed for skin and joint symptoms of lupus. Dosing regimens for antimalarials are readily available to the skilled clinician.

### Immunomodulating Agents

In other embodiments, the anti-IFNα antibodies of the invention may be administered in combination with immunomodulating agents. Azathioprine (Imuran), mycophenolate mofetil (CellCept), and cyclophosphamide (Cytoxan) are in a group of agents known as cytotoxic or immunosuppressive drugs. These drugs act in a similar manner to the corticosteroid drugs in that they suppress inflammation and tend to suppress the immune system.

In other embodiments, the anti-IFNα antibodies of the invention may be administered in combination with other agents like methotrexate and cyclosporin to control the symptoms of lupus. Both are immunomodulating drugs which have their own side effects. These drugs are still in the investigational phase for lupus. Some of these agents are used in conjunction with apheresis, a blood filtering treatment. Apheresis has been tried by itself in an effect to remove specific antibodies from the blood but the results have not been promising.

In other embodiments, the anti-IFNα antibodies of the invention may be administered in combination with newer agents directed toward specific cells of the immune system. These include agents which block the production of specific antibodies like those against DNA, or agents which act to suppress the manufacture of antibodies through other mechanisms. Examples of this are intravenous immunoglobulin injections which are given on a regular basis to increase platelets (particles important to coagulation).

In one embodiment, the anti-IFNα antibodies of the invention may be administered in combination with Rituximab (Rituxan). Rituximab decreases the number of B cells, a type of white blood cell, in a patient's body and has shown some promise in treating lupus in people who haven't responded to other immunosuppressants.

In another embodiment, the anti-IFNα antibodies of the invention may be administered in combination with LymphoStat-B® (belimumab). LymphoStat-B is a human monoclonal antibody that specifically recognizes and inhibits the biological activity of B-lymphocyte stimulator, or BlyS.

In another embodiment, the anti-IFNα antibodies of the invention may be administered in combination with Abatacept (Orencia - anti-CTLA-4 IgG). In yet a further embodiment, the anti-IFNα antibodies of the invention may be administered in combination with and tocilizumab (anti-IL-6).

### Anticoagulants

In other embodiments, the anti-IFNα antibodies of the invention may be administered in combination with anticoagulants. These drugs are employed to thin the blood, or in actuality to prevent blood from clotting rapidly. They range from aspirin at very low dose which prevents platelets from sticking, to heparin/coumadin which actually prevent the blood from clotting. The latter requires careful monitoring to insure that the patient is in the "therapeutic range" or that the blood is not excessively "thin". Generally, such therapy is life-long in people with lupus and follows an actual episode of clotting (embolus or thromboses).

### Stem Cell Transplant

In one embodiment, the anti-IFNα antibodies of the invention may be administered in combination with a stem cell transplant. A stem cell transplant uses a patient's own adult stem cells to rebuild their immune system. Before a stem cell transplant you're given a drug that coaxes the adult stem cells out of your bone marrow and into your bloodstream. The stem cells are then filtered from your blood and frozen for later use. Strong immunosuppressive drugs are administered to wipe out your immune system, sometimes in combination with radiation treatments to further ablate the cells of the immune system. The adult stem cells are then administered back into the patient's body where they can rebuild the immune system.

### Dehydroepiandrosterone (DHEA)

(DHEA). Some clinical trials have shown that a synthetic form of the hormone DHEA may improve quality of life in people with lupus. Accordingly, in one embodiment, the anti-IFNα antibodies of the invention may be administered in combination with DHEA.

### Dapsone

Dapsone (diamino-diphenyl sulphone) is an anti-bacterial that inhibits bacterial synthesis of dihydrofolic acid. Dapsone also exhibits anti-inflammatory properties and is used in the treatment of cutaneous lupus. Accordingly, in one embodiment, the anti-IFNα antibodies of the invention may be used in combination with dapsone.

### Pharmaceutical Compositions

Examples of pharmaceutical compositions comprising anti-interferon α antibodies can be found in U.S. patent application no. 60/909,117 entitled "Antibody Formulation", which is hereby incorporated by reference herein in its entirety.

In one embodiment, a formulation of the invention is for parenteral administration. In one embodiment, a formulation of the invention is an injectable formulation. In one embodiment, a formulation of the invention is for intravenous, subcutaneous, or intramuscular administration. In a specific embodiment, a formulation of the invention comprises an anti-interferon alpha antibody wherein said formulation is for subcutaneous injection.

In one embodiment, a formulation of the invention is for intravenous administration wherein said formulation comprises between about 20 mg/ml and about 40 mg/ml anti-interferon alpha antibody or a fragment thereof. In a specific embodiment, a formulation of the invention is for intravenous administration wherein said formulation comprises between about 20 mg/ml and about 40 mg/ml of an anti-interferon alpha antibody.

In one embodiment, a formulation of the invention is for subcutaneous administration wherein said formulation comprises between about 70 mg/ml and about 250 mg/ml of an anti-interferon alpha antibody or a fragment thereof. In a specific embodiment, a formulation of the invention is for subcutaneous administration wherein said formulation comprises between about 70 mg/ml and about 250 mg/ml of an anti-interferon alpha antibody.

In one embodiment, a formulation of the invention is for aerosol administration.

The present invention also provides a pharmaceutical unit dosage form suitable for parenteral administration to a human which comprises an anti-interferon alpha antibody formulation in a suitable container. In one embodiment, a pharmaceutical unit dosage of the invention comprises an anti-interferon alpha antibody. In one embodiment, a pharmaceutical unit dosage of the invention comprises an intravenously, subcutaneously, or intramuscularly delivered of an anti-interferon alpha antibody formulation. In another embodiment, a pharmaceutical unit dosage of the invention comprises aerosol delivered anti-interferon alpha antibody formulation. In a specific embodiment, a pharmaceutical unit dosage of the invention comprises a subcutaneously delivered anti-interferon alpha antibody formulation. In another embodiment, a pharmaceutical unit dosage of the invention comprises an aerosol delivered anti-interferon alpha antibody formulation. In a further embodiment, a pharmaceutical unit dosage of the invention comprises an intranasally administered anti-interferon alpha antibody formulation.

In one embodiment, a formulation of the invention is provided in a sealed container.

The present invention further provided a kit comprising an anti-interferon alpha antibody formulation of the invention.

Certain additional embodiments of the invention are detailed in the following numbered embodiments:
1. A method of treating SLE in a human subject, wherein said human subject has a pre-treatment SLEDAI score of X, wherein X is equal to between and including 1 to 105, said method comprising administration of an anti-interferon α antibody to said subject, wherein said administration yields a post-treatment SLEDAI score of X minus between and including 1 to 105, wherein the lowest SLEDAI value that X minus between and including 1 to 105 can yield is 0.
2. The method of embodiment 1, wherein said pre-treatment SLEDAI score is in the range of 1-50.
3. The method of embodiment 1, wherein said pre-treatment SLEDAI score is in the range of 1-25.
4. The method of embodiment 1, wherein said pre-treatment SLEDAI score is in the range of 1-10.
5. The method of embodiment 1, wherein said pre-treatment SLEDAI score is in the range of 1-9.
6. The method of embodiment 1, wherein said pre-treatment SLEDAI score is in the range of 1-8.
7. The method of embodiment 1, wherein said pre-treatment SLEDAI score is in the range of 1-7.
8. The method of embodiment 1, wherein said pre-treatment SLEDAI score is in the range of 1-6.
9. The method of embodiment 1, wherein said pre-treatment SLEDAI score is in the range of 1-5.
10. The method of embodiment 1, wherein said pre-treatment SLEDAI score is in the range of 1-4.
11. The method of embodiment 1, wherein said pre-treatment SLEDAI score is in the range of 1-3.
12. The method of embodiment 1, wherein said pre-treatment SLEDAI score is in the range of 1-2.
13. The method of embodiment 1, wherein said pre-treatment SLEDAI score is in the range of 5-25.
14. The method of embodiment 1, wherein said pre-treatment SLEDAI score is in the range of 5-20.
15. The method of embodiment 1, wherein said pre-treatment SLEDAI score is in the range of 5-15.
16. The method of embodiment 1, wherein said pre-treatment SLEDAI score is in the range of 5-10.
17. The method of embodiment 1, wherein said pre-treatment SLEDAI score is in the range of 5-9.
18. The method of embodiment 1, wherein said pre-treatment SLEDAI score is in the range of 5-8.
19. The method of embodiment 1, wherein said pre-treatment SLEDAI score is in the range of 5-7.
20. The method of embodiment 1, wherein said pre-treatment SLEDAI score is in the range of 5-6.
21. The method of embodiment 1, wherein said pre-treatment SLEDAI score is 1.
22. The method of embodiment 1, wherein said pre-treatment SLEDAI score is 2.
23. The method of embodiment 1, wherein said pre-treatment SLEDAI score is 3.
24. The method of embodiment 1, wherein said pre-treatment SLEDAI score is 4.
25. The method of embodiment 1, wherein said pre-treatment SLEDAI score is 5.
26. The method of embodiment 1, wherein said pre-treatment SLEDAI score is 6.
27. The method of embodiment 1, wherein said pre-treatment SLEDAI score is 7.
28. The method of embodiment 1, wherein said pre-treatment SLEDAI score is 8.
29. The method of embodiment 1, wherein said pre-treatment SLEDAI score is 9.
30. The method of embodiment 1, wherein said pre-treatment SLEDAI score is 10.
31. The method of embodiment 1, wherein said pre-treatment SLEDAI score is 11.
32. The method of embodiment 1, wherein said pre-treatment SLEDAI score is 12.
33. The method of embodiment 1, wherein said pre-treatment SLEDAI score is 13.
34. The method of embodiment 1, wherein said pre-treatment SLEDAI score is 14.
35. The method of embodiment 1, wherein said pre-treatment SLEDAI score is 15.
36. The method of embodiment 1, wherein said pre-treatment SLEDAI score is 16.
37. The method of embodiment 1, wherein said pre-treatment SLEDAI score is 17.
38. The method of embodiment 1, wherein said pre-treatment SLEDAI score is 18.
39. The method of embodiment 1, wherein said pre-treatment SLEDAI score is 19.
40. The method of embodiment I, wherein said pre-treatment SLEDAI score is 20.
41. The method of embodiment 1, wherein said pre-treatment SLEDAI score is 21.
42. The method of embodiment 1, wherein said pre-treatment SLEDAI score is 22.
43. The method of embodiment 1, wherein said pre-treatment SLEDAI score is 23.
44. The method of embodiment 1, wherein said pre-treatment SLEDAI score is 24.
45. The method of embodiment 1, wherein said pre-treatment SLEDAI score is 25.
46. The method of any of embodiments 1-45, wherein said'post-treatment SLEDAI score is equal to the pre-treatment SLEDAI score minus 1, with 0 being lowest post-treatment SLEDAI score attainable.
47. The method of any of embodiments 1-45, wherein said post-treatment SLEDAI score is equal to the pre-treatment SLEDAI score minus 2, with 0 being lowest post-treatment SLEDAI score attainable.
48. The method of any of embodiments 1-45, wherein said post-treatment SLEDAI score is equal to the pre-treatment SLEDAI score minus 3, with 0 being lowest post-treatment SLEDAI score attainable.
49. The method of any of embodiments 1-45, wherein said post-treatment SLEDAI score is equal to the pre-treatment SLEDAI score minus 4, with 0 being lowest post-treatment SLEDAI score attainable.
50. The method of any of embodiments 1-45, wherein said post-treatment SLEDAI score is equal to the pre-treatment SLEDAI score minus 5, with 0 being lowest post-treatment SLEDAI score attainable.
51. The method of any of embodiments 1-45, wherein said post-treatment SLEDAI score is equal to the pre-treatment SLEDAI score minus 6, with 0 being lowest post-treatment SLEDAI score attainable.
52. The method of any of embodiments 1-45, wherein said post-treatment SLEDAI score is equal to the pre-treatment SLEDAI score minus 7, with 0 being lowest post-treatment SLEDAI score attainable.
53. The method of any of embodiments 1-45, wherein said post-treatment SLEDAI score is equal to the pre-treatment SLEDAI score minus 8, with 0 being lowest post-treatment SLEDAI score attainable.
54. The method of any of embodiments 1-45, wherein said post-treatment SLEDAI score is equal to the pre-treatment SLEDAI score minus 9, with 0 being lowest post-treatment SLEDAI score attainable.
55. The method of any of embodiments 1-45, wherein said post-treatment SLEDAI score is equal to the pre-treatment SLEDAI score minus 10, with 0 being lowest post-treatment SLEDAI score attainable.
56. The method of any of embodiments 1-45, wherein said post-treatment SLEDAI score is equal to the pre-treatment SLEDAI score minus 11, with 0 being lowest post-treatment SLEDAI score attainable.
57. The method of any of embodiments 1-45, wherein said post-treatment SLEDAI score is equal to the pre-treatment SLEDAI score minus 12, with 0 being lowest post-treatment SLEDAI score attainable.
58. The method of any of embodiments 1-45, wherein said post-treatment SLEDAI score is equal to the pre-treatment SLEDAI score minus 13, with 0 being lowest post-treatment SLEDAI score attainable.
59. The method of any of embodiments 1-45, wherein said post-treatment SLEDAI score is equal to the pre-treatment SLEDAI score minus 14, with 0 being lowest post-treatment SLEDAI score attainable.
60. The method of any of embodiments 1-45, wherein said post-treatment SLEDAI score is equal to the pre-treatment SLEDAI score minus 15, with 0 being lowest post-treatment SLEDAI score attainable.
61. The method of any of embodiments 1-45, wherein said post-treatment SLEDAI score is equal to the pre-treatment SLEDAI score minus 16, with 0 being lowest post-treatment SLEDAI score attainable.
62. The method of any of embodiments 1-45, wherein said post-treatment SLEDAI score is equal to the pre-treatment SLEDAI score minus 17, with 0 being lowest post-treatment SLEDAI score attainable.
63. The method of any of embodiments 1-45, wherein said post-treatment SLEDAI score is equal to the pre-treatment SLEDAI score minus 18, with 0 being lowest post-treatment SLEDAI score attainable.
64. The method of any of embodiments 1-45, wherein said post-treatment SLEDAI score is equal to the pre-treatment SLEDAI score minus 19, with 0 being lowest post-treatment SLEDAI score attainable.
65. The method of any of embodiments 1-45, wherein said post-treatment SLEDAI score is equal to the pre-treatment SLEDAI score minus 20, with 0 being lowest post-treatment SLEDAI score attainable.
66. The method of any of embodiments 1-45, wherein said post-treatment SLEDAI score is equal to the pre-treatment SLEDAI score minus 21, with 0 being lowest post-treatment SLEDAI score attainable.
67. The method of any of embodiments 1-45, wherein said post-treatment SLEDAI score is equal to the pre-treatment SLEDAI score minus 22, with 0 being lowest post-treatment SLEDAI score attainable.
68. The method of any of embodiments 1-45, wherein said post-treatment SLEDAI score is equal to the pre-treatment SLEDAI score minus 23, with 0 being lowest post-treatment SLEDAI score attainable.
69. The method of any of embodiments 1-45, wherein said post-treatment SLEDAI score is equal to the pre-treatment SLEDAI score minus 24, with 0 being lowest post-treatment SLEDAI score attainable.
70. The method of any of embodiments 1-45, wherein said post-treatment SLEDAI score is equal to the pre-treatment SLEDAI score minus 25, with 0 being lowest post-treatment SLEDAI score attainable.
67. The method of any of embodiments 1-45, wherein said post-treatment SLEDAI score is equal to the pre-treatment SLEDAI score minus at least 1, at least 3, at least 5, at least 10, at least 15, or at least 20, with 0 being lowest post-treatment SLEDAI score attainable.
68. The method of any of embodiments 1-45, wherein said post-treatment SLEDAI score is equal to the pre-treatment SLEDAI score minus at least 1, with 0 being lowest post-treatment SLEDAI score attainable.
69. The method of any of embodiments 1-45, wherein said post-treatment SLEDAI score is equal to the pre-treatment SLEDAI score minus at least 2, with 0 being lowest post-treatment SLEDAI score attainable.
70. The method of any of embodiments 1-45, wherein said post-treatment SLEDAI score is equal to the pre-treatment SLEDAI score minus at least 3, with 0 being lowest post-treatment SLEDAI score attainable.
71. The method of any of embodiments 1-45, wherein said post-treatment SLEDAI score is equal to the pre-treatment SLEDAI score minus at least 4, with 0 being lowest post-treatment SLEDAI score attainable.
72. The method of any of embodiments 1-45, wherein said post-treatment SLEDAI score is equal to the pre-treatment SLEDAI score minus at least 5, with 0 being lowest post-treatment SLEDAI score attainable.
73. The method of any of embodiments 1-45, wherein said post-treatment SLEDAI score is equal to the pre-treatment SLEDAI score minus at least 6, with 0 being lowest post-treatment SLEDAI score attainable.
74. The method of any of embodiments 1-45, wherein said post-treatment SLEDAI score is equal to the pre-treatment SLEDAI score minus at least 7, with 0 being lowest post-treatment SLEDAI score attainable.
75. The method of any of embodiments 1-45, wherein said post-treatment SLEDAI score is equal to the pre-treatment SLEDAI score minus at least 8, with 0 being lowest post-treatment SLEDAI score attainable.
76. The method of any of embodiments 1-45, wherein said post-treatment SLEDAI score is equal to the pre-treatment SLEDAI score minus at least 9, with 0 being lowest post-treatment SLEDAI score attainable.
77. The method of any of embodiments 1-45, wherein said post-treatment SLEDAI score is equal to the pre-treatment SLEDAI score minus at least 10, with 0 being lowest post-treatment SLEDAI score attainable.
78. The method of any of embodiments 1-45, wherein said post-treatment SLEDAI score is equal to the pre-treatment SLEDAI score minus at least 11, with 0 being lowest post-treatment SLEDAI score attainable.
79. The method of any of embodiments 1-45, wherein said post-treatment SLEDAI score is equal to the pre-treatment SLEDAI score minus at least 12, with 0 being lowest post-treatment SLEDAI score attainable.
80. The method of any of embodiments 1-45, wherein said post-treatment SLEDAI score is equal to the pre-treatment SLEDAI score minus at least 13, with 0 being lowest post-treatment SLEDAI score attainable.
81. The method of any of embodiments 1-45, wherein said post-treatment SLEDAI score is equal to the pre-treatment SLEDAI score minus at least 14, with 0 being lowest post-treatment SLEDAI score attainable.
82. The method of any of embodiments 1-45, wherein said post-treatment SLEDAI score is equal to the pre-treatment SLEDAI score minus at least 15, with 0 being lowest post-treatment SLEDAI score attainable.
83. The method of any of embodiments 1-45, wherein said post-treatment SLEDAI score is equal to the pre-treatment SLEDAI score minus at least 16, with 0 being lowest post-treatment SLEDAI score attainable.
84. The method of any of embodiments 1-45, wherein said post-treatment SLEDAI score is equal to the pre-treatment SLEDAI score minus at least 17, with 0 being lowest post-treatment SLEDAI score attainable.
85. The method of any of embodiments 1-45, wherein said post-treatment SLEDAI score is equal to the pre-treatment SLEDAI score minus at least 18, with 0 being lowest post-treatment SLEDAI score attainable.
86. The method of any of embodiments 1-45, wherein said post-treatment SLEDAI score is equal to the pre-treatment SLEDAI score minus at least 19, with 0 being lowest post-treatment SLEDAI score attainable.
87. The method of any of embodiments 1-45, wherein said post-treatment SLEDAI score is equal to the pre-treatment SLEDAI score minus at least 20, with 0 being lowest post-treatment SLEDAI score attainable.
88. The method of any of embodiments 1-45, wherein said post-treatment SLEDAI score is equal to the pre-treatment SLEDAI score minus at least 21, with 0 being lowest post-treatment SLEDAI score attainable.
89. The method of any of embodiments 1-45, wherein said post-treatment SLEDAI score is equal to the pre-treatment SLEDAI score minus at least 22, with 0 being lowest post-treatment SLEDAI score attainable.
90. The method of any of embodiments 1-45, wherein said post-treatment SLEDAI score is equal to the pre-treatment SLEDAI score minus at least 23, with 0 being lowest post-treatment SLEDAI score attainable.
91. The method of any of embodiments 1-45, wherein said post-treatment SLEDAI score is equal to the pre-treatment SLEDAI score minus at least 24, with 0 being lowest post-treatment SLEDAI score attainable.
92. The method of any of embodiments 1-45, wherein said post-treatment SLEDAI score is equal to the pre-treatment SLEDAI score minus at least 25, with 0 being lowest post-treatment SLEDAI score attainable.
93. The method of any of embodiments 1-92, wherein said post-treatment SLEDAI score is 0.
94. The method of any of embodiments 1-92, wherein said post-treatment SLEDAI score is 1.
95. The method of any of embodiments 1-92, wherein said post-treatment SLEDAI score is 2.
96. The method of any of embodiments 1-92, wherein said post-treatment SLEDAI score is 3.
97. The method of any of embodiments 1-92, wherein said post-treatment SLEDAI score is 4.
98. The method of any of embodiments 1-92, wherein said post-treatment SLEDAI score is 5.
99. The method of any of embodiments 1-92, wherein said post-treatment SLEDAI score is 6.
100. The method of any of embodiments 1-92, wherein said post-treatment SLEDAI score is 7.
101. The method of any of embodiments 1-92, wherein said post-treatment SLEDAI score is 8.
102. The method of any of embodiments 1-92, wherein said post-treatment SLEDAI score is 9.
103. The method of any of embodiments 1-92, wherein said post-treatment SLEDAI score is 10.
104. The method of any of embodiments 1-92, wherein said post-treatment SLEDAI score is 11.
105. The method of any of embodiments 1-92, wherein said post-treatment SLEDAI score is 12.
106. The method of any of embodiments 1-92, wherein said post-treatment SLEDAI score is 13.
107. The method of any of embodiments 1-92, wherein said post-treatment SLEDAI score is 14.
108. The method of any of embodiments 1-92, wherein said post-treatment SLEDAI score is 15.
109. The method of any of embodiments 1-92, wherein said post-treatment SLEDAI score is 16.
110. The method of any of embodiments 1-92, wherein said post-treatment SLEDAI score is 17.
111. The method of any of embodiments 1-92, wherein said post-treatment SLEDAI score is 18.
112. The method of any of embodiments 1-92, wherein said post-treatment SLEDAI score is 19.
113. The method of any of embodiments 1-92, wherein said post-treatment SLEDAI score is 20.
114. The method of any of embodiments 1-92, wherein said post-treatment SLEDAI score is 21.
115. The method of any of embodiments 1-92, wherein said post-treatment SLEDAI score is 22.
116. The method of any of embodiments 1-92, wherein said post-treatment SLEDAI score is 23.
117. The method of any of embodiments 1-92, wherein said post-treatment SLEDAI score is 24.
118. The method of any of embodiments 1-92, wherein said post-treatment SLEDAI score is 25.
119. A method of treating SLE in a human subject, wherein said human subject has a pre-treatment SLEDAI score of between and including 1 to 105, said method comprising administration of an anti-interferon α antibody to said subject, wherein said administration yields a post-treatment SLEDAI score that is reduced by at least about 1%, about 2%, about 3%, about 4%, about 5%, about 6%, about 7%, about 8%, about 9%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100% as compared to the pre-treatment SLEDAI score.
120. A method of reducing SLEDAI flares in a human subject, said method comprising administering to said subject an anti-interferon α antibody.
121. A method of treating SLE in a human subject comprising administering to said human subject an anti-interferon α antibody.
122. The method of any of embodiments 1-121, wherein said reduction in SLEDAI score is evident at at least 14 days, at least 28 days, or at least 56 days post antibody administration.
123. The method of any of embodiments 1-122, wherein prior to treatment said human subject has at least 1, at least 2, at least 3, at least 4, at least 5, at least 6, at least 7, at least 8, at least 9, at least 10, at least 11, at least 12, at least 13, at least 14, at least 15, or 16 of the listed ACR criteria of Figure 3, said method comprising administering to said subject an anti-interferon α antibody.
124. The method of any of embodiments 1-123, wherein said anti-interferon α antibody is administered at a dose selected from the group consisting of 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg, 10.0 mg/kg, 11.0 mg/kg, 12.0 mg/kg, 13.0 mg/kg, 14.0 mg/kg, 15.0 mg/kg, 16.0 mg/kg, 17.0 mg/kg, 18.0 mg/kg, 19.0 mg/kg, 20.0 mg/kg, 25 mg/kg, and 30 mg/kg.
125. The method of any of embodiments 1-124, wherein said anti-interferon α antibody selectively binds to at least the following interferon α subtype: α2a.
126. The method of any of embodiments 1-124, wherein said anti-interferon α antibody selectively binds to at least the following interferon α subtype: α2b.
127. The method of any of embodiments 1-124, wherein said anti-interferon α antibody selectively binds to at least the following interferon α subtype: α6.
128. The method of any of embodiments 1-124, wherein said anti-interferon α antibody selectively binds to at least the following interferon α subtype: α16.
129. The method of any of embodiments 1-124, wherein said anti-interferon α antibody selectively binds to at least the following interferon α subtype: α10.
130. The method of any of embodiments 1-124, wherein said anti-interferon α antibody selectively binds to at least the following interferon α subtype: α5.
131. The method of any of embodiments 1-124, wherein said anti-interferon α antibody selectively binds to at least the following interferon α subtype: α8.
132. The method of any of embodiments 1-124, wherein said anti-interferon α antibody selectively binds to at least the following interferon α subtype: α7.
133. The method of any of embodiments 1-124, wherein said anti-interferon α antibody selectively binds to at least the following interferon α subtype: α17.
134. The method of any of embodiments 1-124, wherein said anti-interferon α antibody selectively binds to at least the following interferon α subtype: α21.
135. The method of any of embodiments 1-124, wherein said anti-interferon α antibody selectively binds to at least the following interferon α subtype: α14.
136. The method of any of embodiments 1-124, wherein said anti-interferon α antibody selectively binds to at least the following interferon α subtype: αa1.
137. The method of any of embodiments 1-124, wherein said anti-interferon α antibody selectively binds to at least the following interferon α subtype: α4.
138. The method of any of embodiments 1-124, wherein said anti-interferon α antibody selectively binds to at least the following interferon α subtypes: α1 and α2.
139. The method of any of embodiment 1-124, wherein said anti-interferon α antibody selectively binds to at least the following interferon α subtypes: α1, α2, and α4.
140. The method of any of embodiments 1-124, wherein said anti-interferon α antibody selectively binds to at least the following interferon α subtypes: α1, α2, α4, and α5.
141. The method of any of embodiments 1-124, wherein said anti-interferon α antibody selectively binds to at least the following interferon α subtypes: α1, α2, α4, α5, α8, and α10.
142. The method of any of embodiments 1-124, wherein said anti-interferon α antibody selectively binds to at least the following interferon α subtypes: α1, α2, α4, α5, α8, α10, and α21.
143. The method of any of embodiments 1-124, wherein said anti-interferon α antibody selectively binds to at least the following interferon α subtypes: α1, α2, α4, α5, α6, α8, α10, and α21.
144. The method of any of embodiments 1-124, wherein said anti-interferon α antibody selectively binds to at least the following interferon α subtypes: α1, α2, α4, α5, α6, α7, α8, α10, and α21.
145. The method of any of embodiments 1-124, wherein said anti-interferon α antibody selectively binds to at least the following interferon α subtypes: α1, α2, α4, α5, α6, α7, α8, α10, α14, α17, and α21.
146. The method of any of embodiments 1-124, wherein said anti-interferon α antibody selectively binds to at least the following interferon α subtypes: α1, α2, α4, α5, α6, α7, α8, α10, α14, α16, α17, and α21.
147. The method of any of embodiments 1-146, wherein said anti-interferon α antibody selectively binds to all interferon α subtypes.
148. The method of any of embodiments 1-147, wherein said anti-inteferon α antibody is not MEDI-545.

### EXAMPLES

The invention is now described with reference to the following examples. These examples are provided for the purpose of illustration only and the invention should in no way be construed as being limited to these examples but rather should be construed to encompass any and all variations which become evident as a result of the teachings provided herein.

### EXAMPLE 1

### NEUTRALIZATION OF TYPE I IFN ACTIVITY IN SLE PATIENT PLASMA BY MEDI-545

SLE patient plasma has been shown to have elevated concentrations of Type I IFN. It was hypothesized that an antibody that has the capacity to neutralize the biological activity of IFN-α in SLE patient plasma could be an effective therapy for the treatment of SLE. To address this question we determined whether MEDI-545 could inhibit Type I IFN activity in the sera of individuals using luciferase reporter assays.

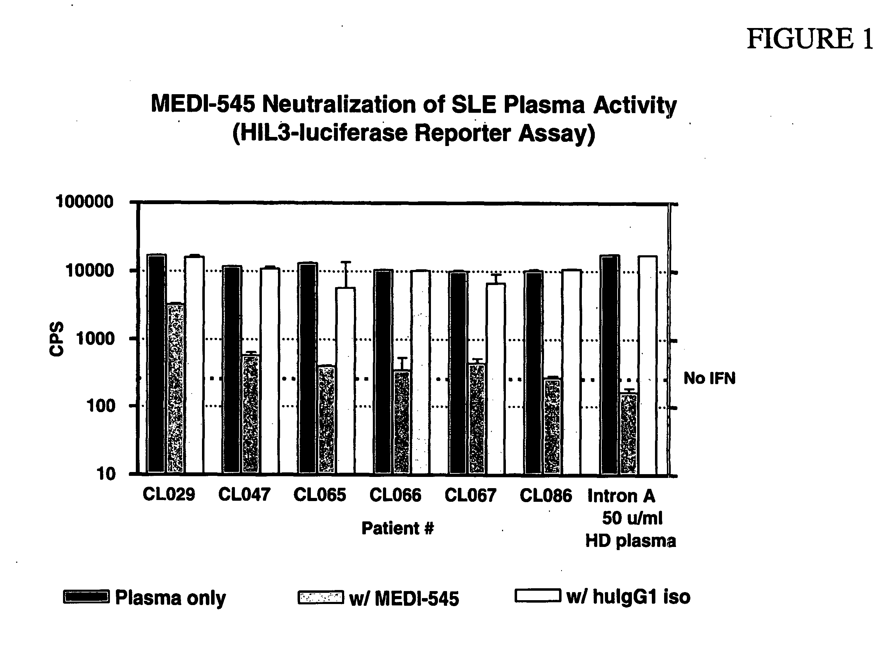

Figure 1 summarizes data from six SLE donors. IFN-α activity was neutralized in the presence of MEDI-545 resulting in blockade of luciferase induction (counts per second). The IFN activity in 5 of 6 patient samples was completely inhibited. The IFN signaling in the remaining patient sample was significantly reduced; the residual activity may represent Type I IFNs, such as IFN-β or IFN-ω, which are not neutralized by MEDI-545.

To confirm the previous observation on SLE patient plasma, the neutralizing potency of MEDI-545 on 1FN-α, activity was extended to 20 SLE serum samples using a different cell line than was used in the previously described experiment. In these experiments the PIL-5 monocytic cell line transfected with ISRE-luciferase was used. PIL-5 cells were used in this experiment because their growth rate is quite robust, and similar to the HIL-3 cells, they respond well to low levels of Type I IFN.

Table 1 includes leukocyte-IFN equivalent activity values for 20 SLE patients' serum samples treated with MEDI-545, anti-IFNAR or R3-47 antibodies. Compared with the R3-47 negative control group, MEDI-545 showed good activity against all donor sera; the minimum inhibition of activity observed was about 72% (in donor 417). Overall, treatment with MEDI-545 resulted in a 95.6 ± 7 neutralization of leukocyte-IFN activity. With the anti-IFNAR mAb, the inhibition was 99.8 ± 1.3%.

In summary, MEDI-545 specifically inhibits leukocyte-IFN equivalent activities of serum samples of SLE patients by > 90%, with an approximate IC₅₀ in the subnanomolar range.

**Table 1 Neutralization of Interferon Activity (Leukocyte-like) of SLE Patient Serum**

| **Donor** | **Negative Control** | **MEDI-545** | | **Anti-IFNAR** | |
|---|---|---|---|---|---|
| | | **Leukocytes IFN Activity (IU/mL)** | **Neutralization relative to control (%)** | **Leukocytes IFN Activity (IU/mL)** | **Neutralization relative to control (%)** |
| **1030** | **79.6** | **1.7** | **98** | **< 0.5** | **100** |
| **948** | **39.9** | **1.6** | **96** | **< 0.5** | **100** |
| **751** | **14.5** | **< 0.5** | **100** | **< 0.5** | **100** |
| **429** | **28.3** | **2.2** | **92** | **< 0.5** | **100** |
| **348** | **53.8** | **4.9** | **91** | **< 0.5** | **100** |
| **347** | **53.8** | **< 0.5** | **100** | **< 0.5** | **100** |
| **761** | **9.8** | **< 0.5** | **100** | **< 0.5** | **100** |
| **567** | **6.2** | **< 0.5** | **100** | **< 0.5** | **100** |
| **641** | **8** | **< 0.5** | **100** | **< 0.5** | **100** |
| **463** | **12.6** | **< 0.5** | **100** | **< 0.5** | **100** |
| **1367** | **17** | **< 0.5** | **100** | **< 0.5** | **100** |
| **525** | **5** | **< 0.5** | **100** | **< 0.5** | **100** |
| **340** | **49** | **< 0.5** | **100** | **< 0.5** | **100** |
| **450** | **34** | **1.7** | **95** | **< 0.5** | **100** |
| **325** | **39** | **4.4** | **89** | **< 0.5** | **100** |
| **603** | **115.1** | **12.3** | **89** | **< 0.5** | **100** |
| **502** | **98** | **5.6** | **94** | **< 0.5** | **100** |
| **417** | **83.4** | **23.5** | **72** | **4.8** | **94** |
| **362** | **6.5** | **0** | **100** | **< 0.5** | **100** |
| **329** | **107.4** | **5.1** | **95** | **< 0.5** | **100** |
| **Average** | **43.05** | **3.15** | **95.6** | **0.24** | **99.7** |
| **St. Dev.** | **36.13** | **5.71** | **6.8** | **1.07** | **1.3** |

### Example 2: MEDI-545 CLINICAL TRIAL

### Summary of the Clinical Program with MEDI-545

The goal of the clinical development program for MEDI-545 is to study the safety and efficacy of MEDI-545 in SLE. Initial clinical trials were conducted to establish the safety, tolerability, immunogenicity, and serum pharmacokinetic profile of single and repeat escalating doses of MEDI-545 in SLE patients with cutaneous manifestations. Patients with both mild SLE and prominent skin involvement were selected for this study because (1) patients with milder disease and less systemic end-organ involvement will facilitate the interpretation of safety data and (2) patients with skin manifestations have been shown to have elevated levels of IFN-a in both the blood and skin.

The dose range for MEDI-545 was chosen based on the following considerations. In vitro studies showed that 10 µg/mL of MEDI-545 was sufficient to prevent lupus sera (containing IFN-α) from inducing monocyte differentiation into dendritic cells. This concentration is therefore considered to be a biologically active concentration that is sufficient to neutralize IFN-α in lupus sera. It is hypothesized that evaluation of single IV doses of MEDI-545 between 0.3 and 30 mg/kg will yield serum levels that are equal to or exceed a concentration of 10 µg/mL with an assumption that the pharmacokinetics of MEDI-545 will be similar to other IgG monoclonal antibodies. Based on a cynomolgus monkey toxicity study that evaluated a single dose of MEDI-545 at 100 mg/kg IV, the planned starting dose in humans of 0.3 mg/kg IV is more than 100-fold lower than the highest dose to be tested in cynomolgus monkeys. The highest proposed clinical dose is less than a third of the 100 mg/kg dose in monkeys.

### Derivation of MEDI-545

MEDI-545 is a fully human, 147,000 Dalton IgG1k monoclonal antibody (Mab) that binds to a majority of IFN-a subtypes. MEDI-545 was generated by immunizing the Medarex transgenic HuMab-Mouseâ with human multiple IFN-a subtypes, in complete/incomplete Freund's adjuvant. Hybridomas were obtained by fusing immune splenocytes with the SP-2/0-AgI4 myeloma cell line. The 13H5 monoclonal antibody (IgG4k isotype) was selected based on functional assays as having the most desirable properties for a potential anti-IFN-a therapeutic agent. 13H5 was subsequently converted to an IgG1 antibody isotype, produced in CHO cells, and selected for further characterization and preclinical development with an initial designation of MDX-1103, now referred to as MEDI-545.

### Production and Purification Methods

MEDI-545 was expressed in Chinese Hamster Ovary (CHO) cells and was purified using a two chromatography step process that includes a low pH viral inactivation step and a nanofiltration viral removal step. Cell banks were prepared and have undergone industry accepted characterization.

Manufacturing of clinical supplies of MEDI-545 was conducted in accordance with current Good Manufacturing Practices (cGMP). Cell culture was performed in stirred tank bioreactors to produce crude MEDI-545. The cell-free conditioned medium was purified through various steps (e.g., chromatography, filtration), including viral inactivation and removal steps. The finished product underwent lot release testing before release and distribution of the product for clinical use. The lot release assays and internal tests were performed to ensure the identity, purity, potency, stability and safety of the finished product. All test results for released product were within the set of specifications for the final MEDI-545 drug product.

### Formulation

MEDI-545 was formulated at 5 mg/mL in 20 mM citric acid, 100 mM sodium chloride, 1.5% mannitol, 50 mM Diethylenetriamine Pentacetic Acid (DTPA), and 0.02% plant-derived Polysorbate 80 (Tweenä 80), pH 6.0. MEDI-545 was filled into 10 mL Type I borosilicate 20-mm clear glass serum vials and closed with 20-mm stoppers and seals.

### Preparation of MEDI-545

Research personnel at the sites ordered study medication in a blinded fashion from the pharmacy according to normal study site procedures. The order form must contain at a minimum the patient's current body weight as well as the patient's identification number (PID). Once the order for study medication was received by the pharmacist, the automated randomization system was contacted to randomize the patient to the active or placebo treatment regimen. A confirmatory fax with this information was sent to the pharmacist (or designee). Based on the patient's weight supplied on the study medication order provided by the investigator and on the confirmatory fax received from the automated randomization system, the pharmacist/designee prepared the dose of medication. All study drug doses were prepared using aseptic technique. In order to avoid foaming, vials of MEDI-545 or placebo were not to be shaken.

The dose for each patient was calculated by the pharmacist (as described below) based on the patient's actual weight (to the nearest 0.1 kg) on the day MEDI-545 is administered. The dose should have been rounded to the nearest 0.1 mL.

The required dose of MEDI-545 was to be calculated using the following formula:

Dose (m.L) = Pt. Wt (kg) x Dose (mg/kg)

5 mg/mL of MEDI-545

For example: For an 80 kg patient dosed at 3 mg/kg MEDI-545, the volume of MEDI-545 required for IV infusion would be 48 mL.

The pharmacist/designee was to prepare the study medication according to the instructions provided in the Clinical Trial Material Manual (or equivalent document provided by the sponsor). After preparing the correct dose, the pharmacist was to label the dose according to normal institutional policy but was to include at a minimum the patient's initials, the PID, the total dose (mg) and volume prepared (mL).

Study medication was dispensed for the patient in such a way that it did not unblind the patient, the investigator or any other study personnel.

### Storage and Handling

MEDI-545 was stored at 2 - 8°C until prepared. To avoid foaming, the vial should not have been shaken. If study drug was not infused within 1 hour after preparation, it should have been stored at 2 - 8° C and used within 24 hours of preparation. In the event that the prepared study drug remained at room temperature, it should have been infused within 8 hours.

### Administration

Study drug was administered by IV administration.

Doses of MEDI-545 were calculated prior to study drug administration. MEDI-545 was injected or infused intravenously through a 0.22 micron in-line filter using an established IV access with 0.9% Sodium Chloride as the IV fluid at a rate and duration as specified in the clinical trial material manual. Once the patient's dose was prepared, it must have been administered within 8 hours. If it was not administered within this interval, a new dose must have been prepared since there are no bacteriostatic agents in the preparation.

Monitoring of vital signs, observation of patients during and after IV administration for acute and delayed reactions, and their treatment were described in the protocol, and would be considered routine for a skilled physician.

### Pharmacokinetics

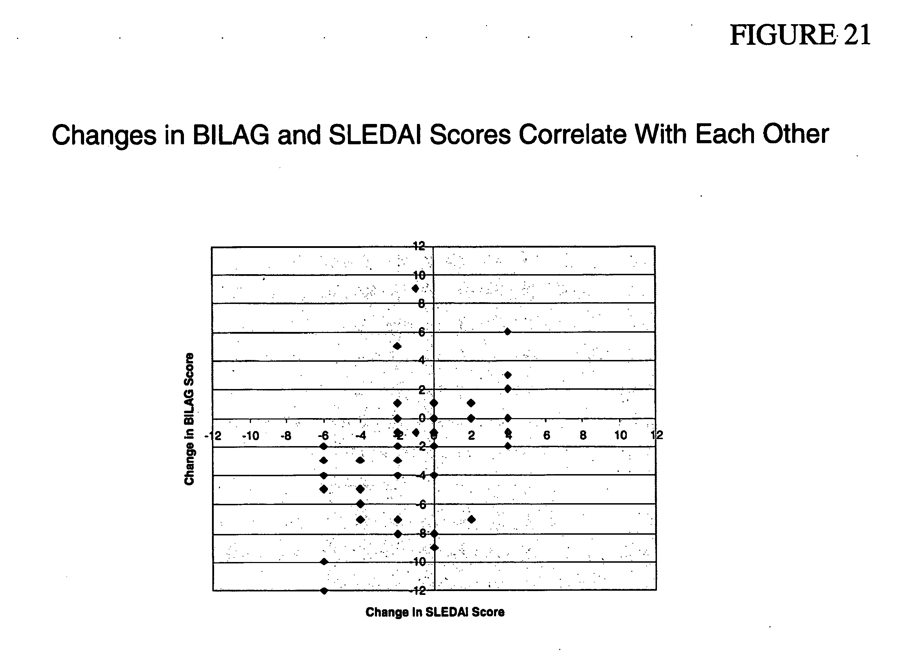

Serum concentrations of MEDI-545 were measured by Cₘₐₓ (maximum concentration) and AUC (area under the curve), and indicated that MEDI-545 concentrations increased in a linear, dose-proportional manner (see Figures 21A-B herein). Serum half-life of MEDI-545 was approximately 20 days.

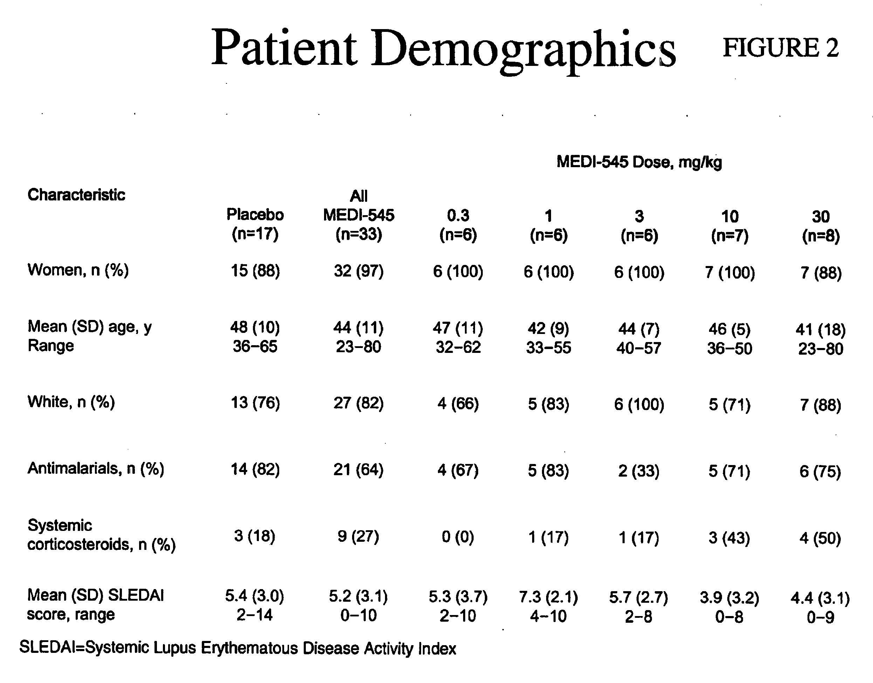

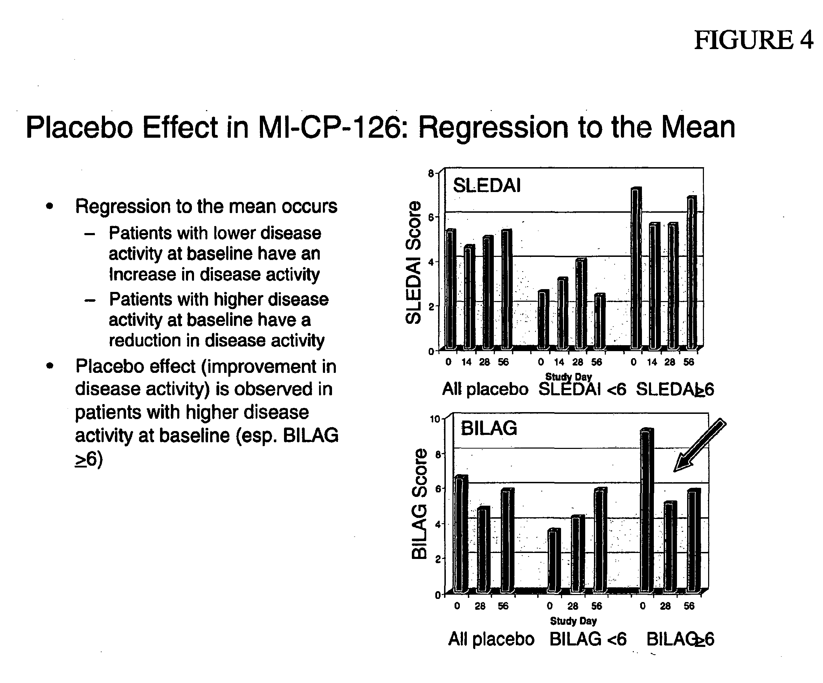

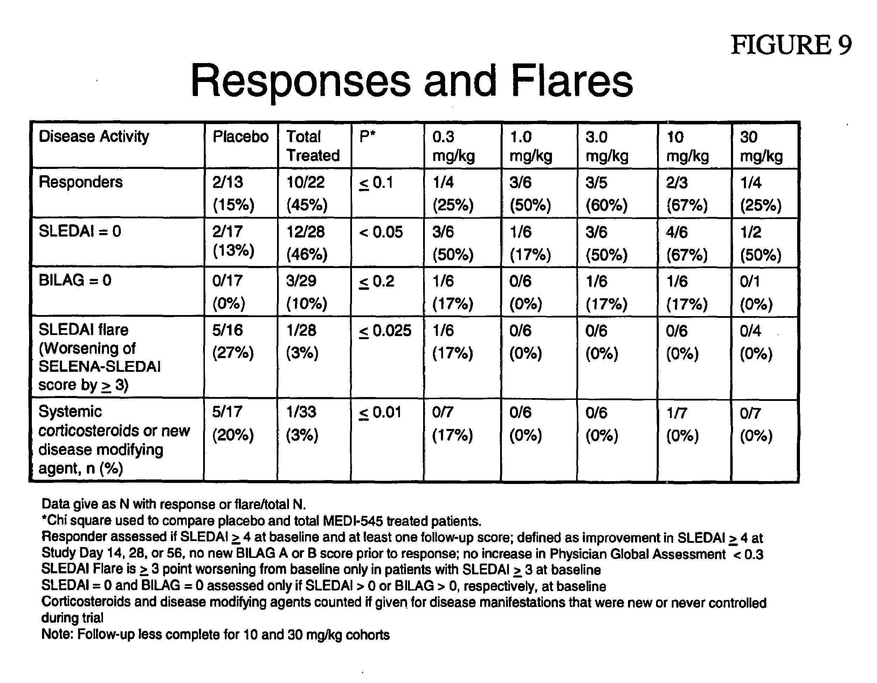

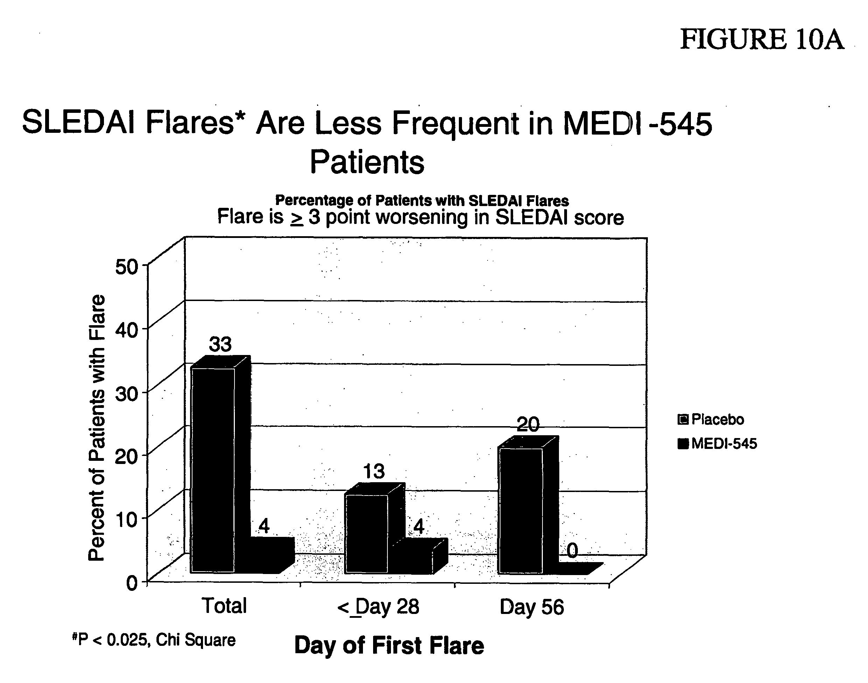

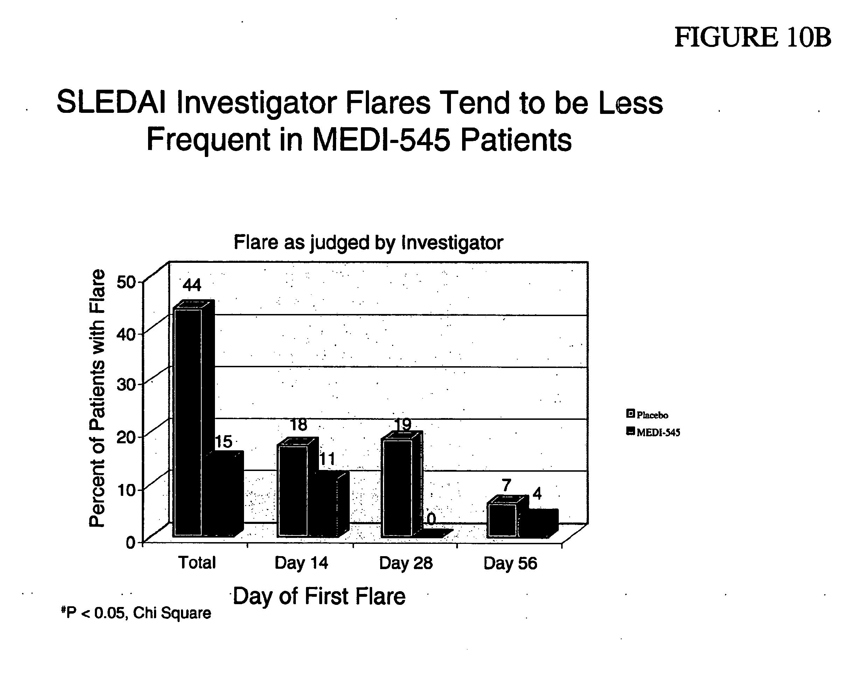

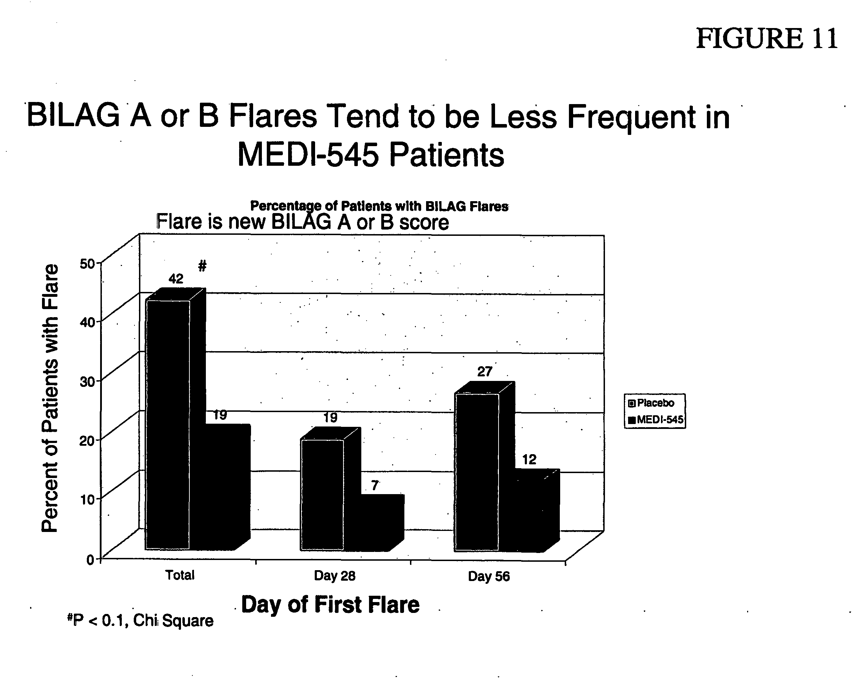

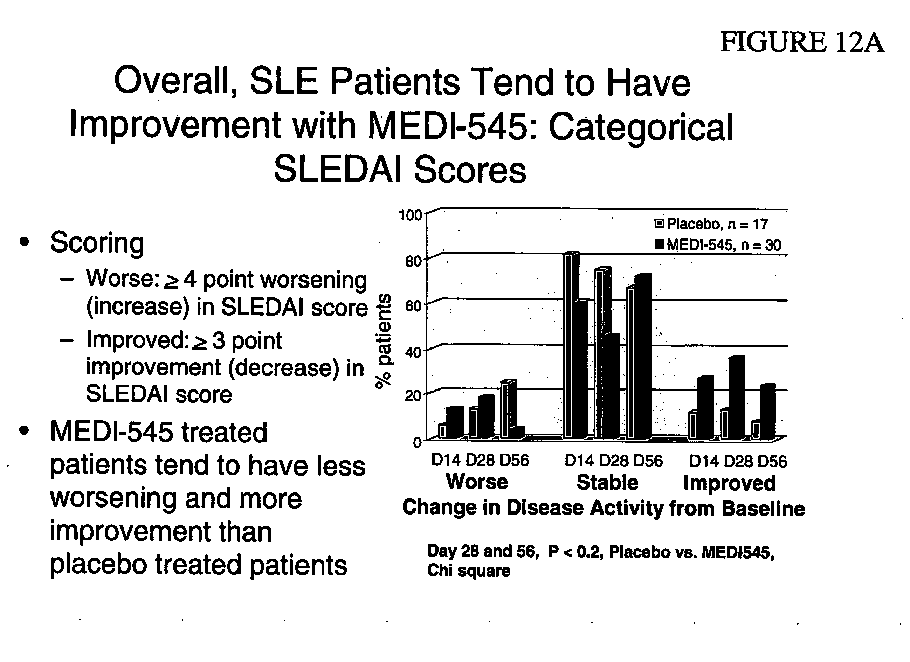

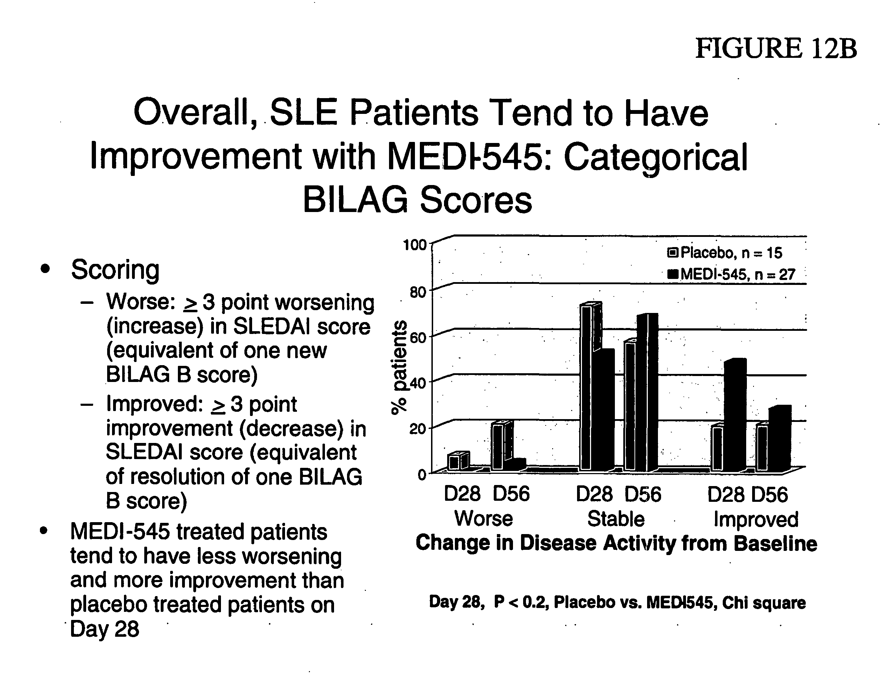

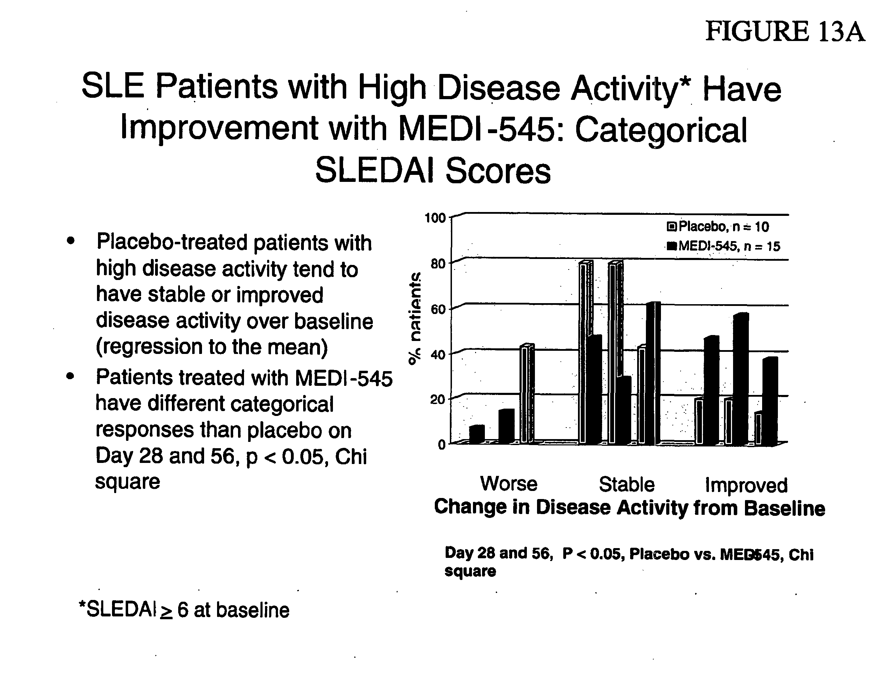

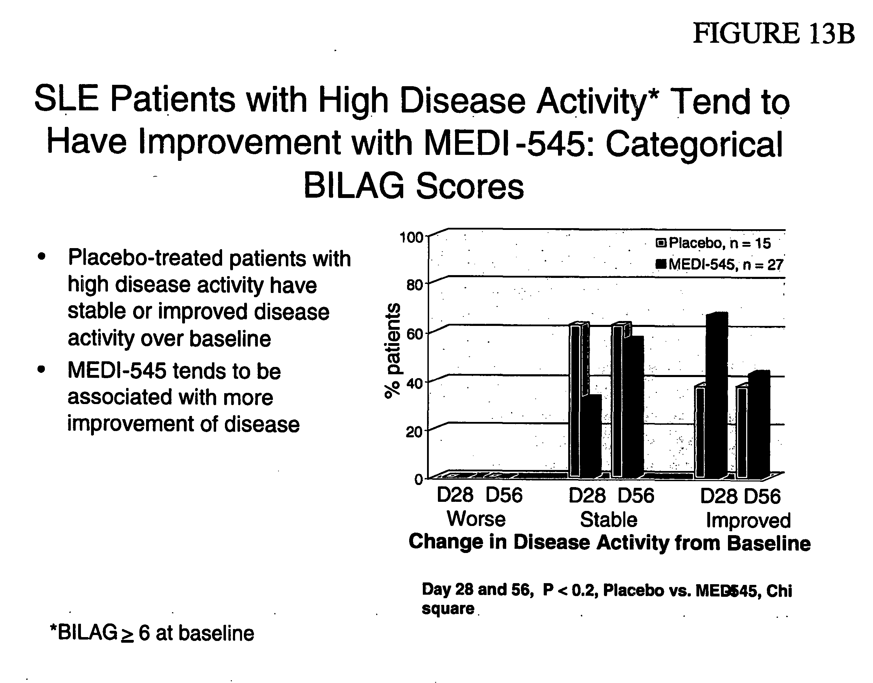

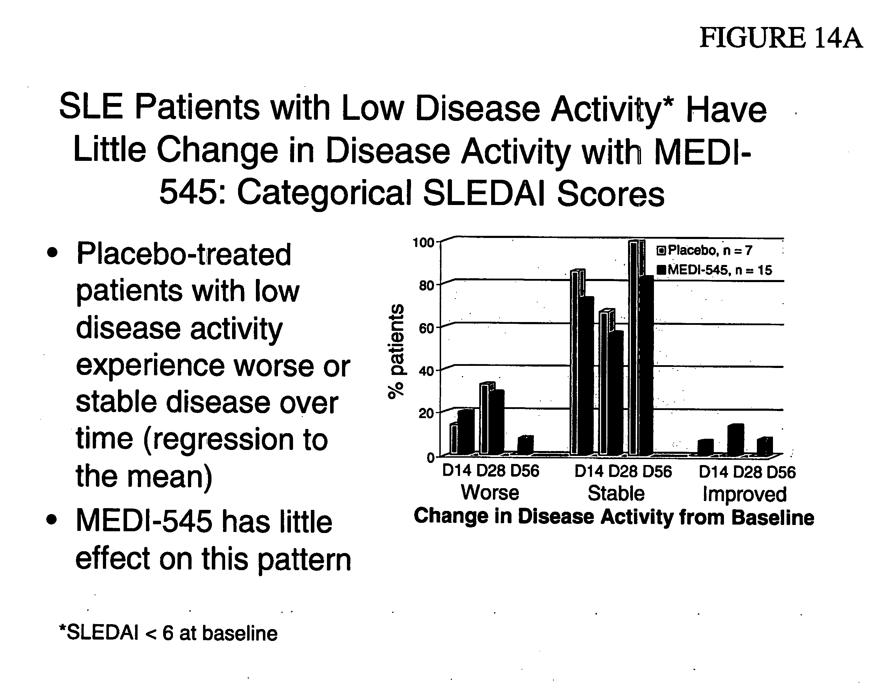

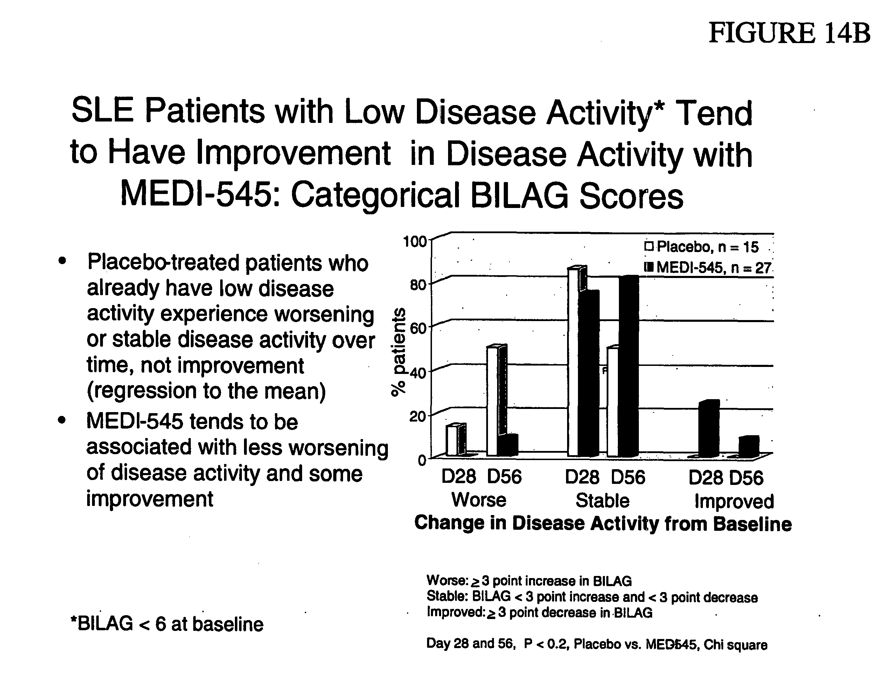

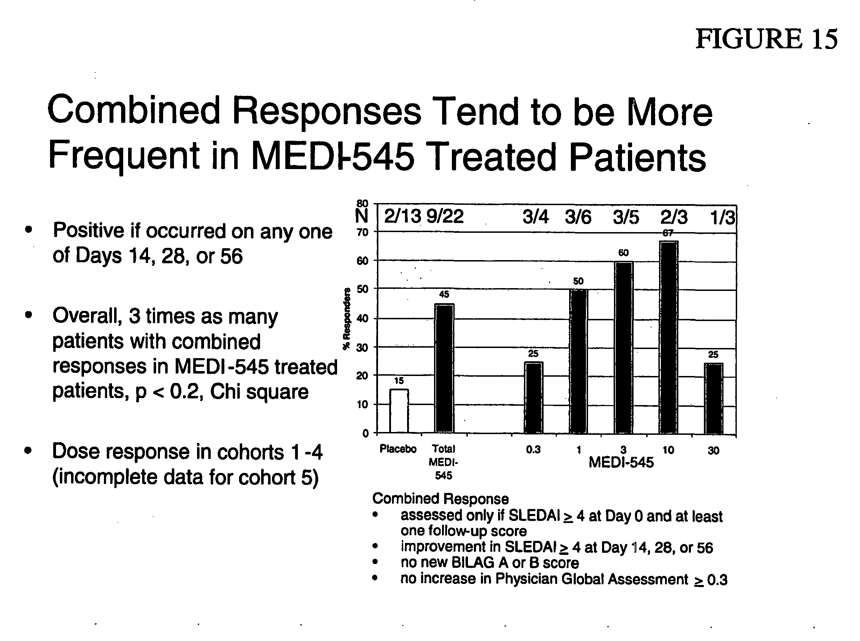

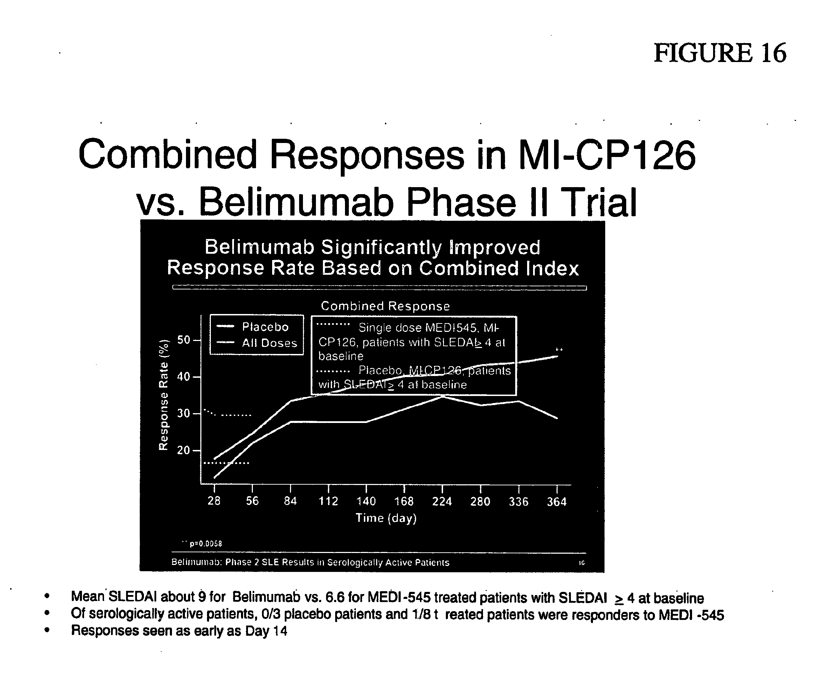

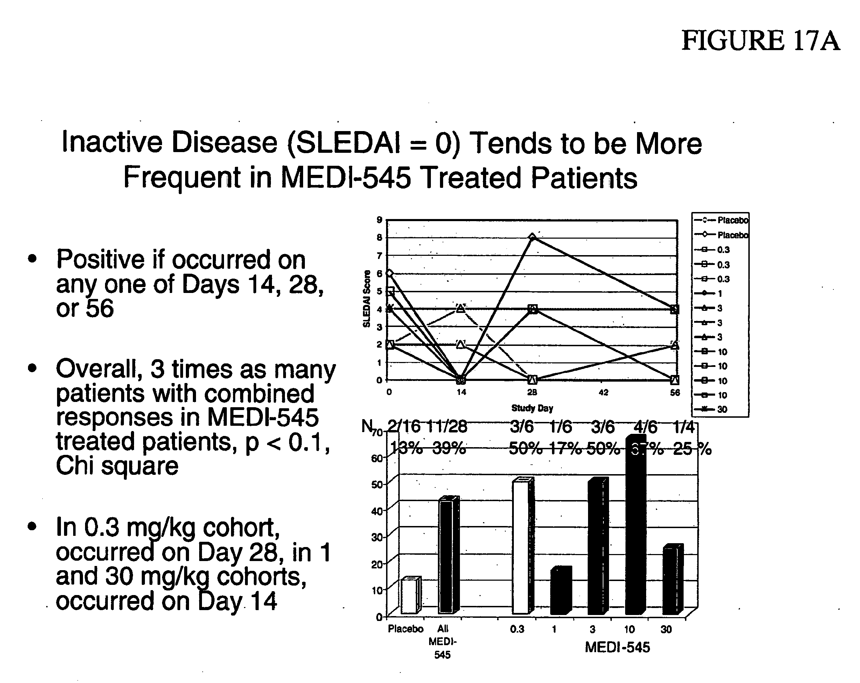

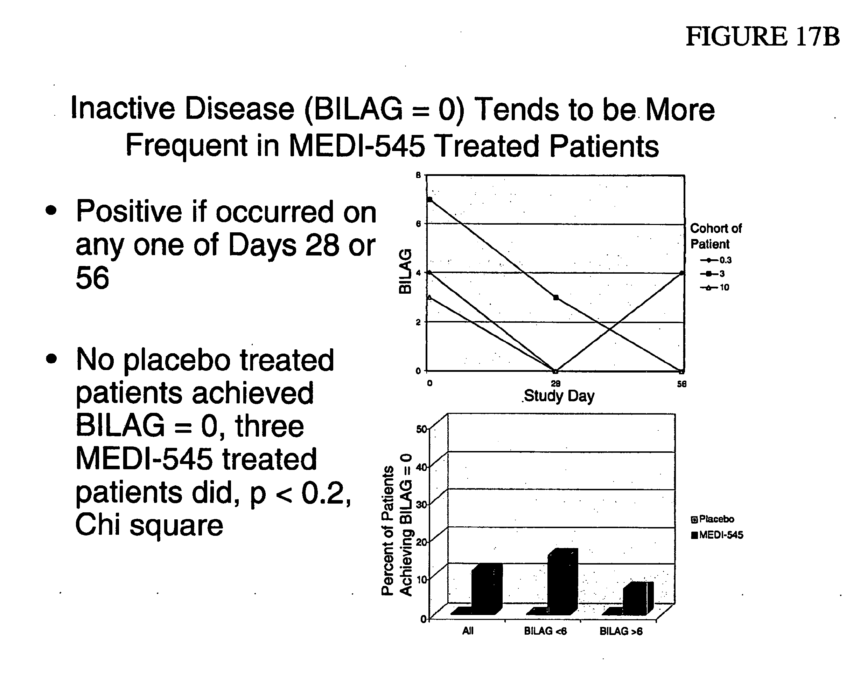

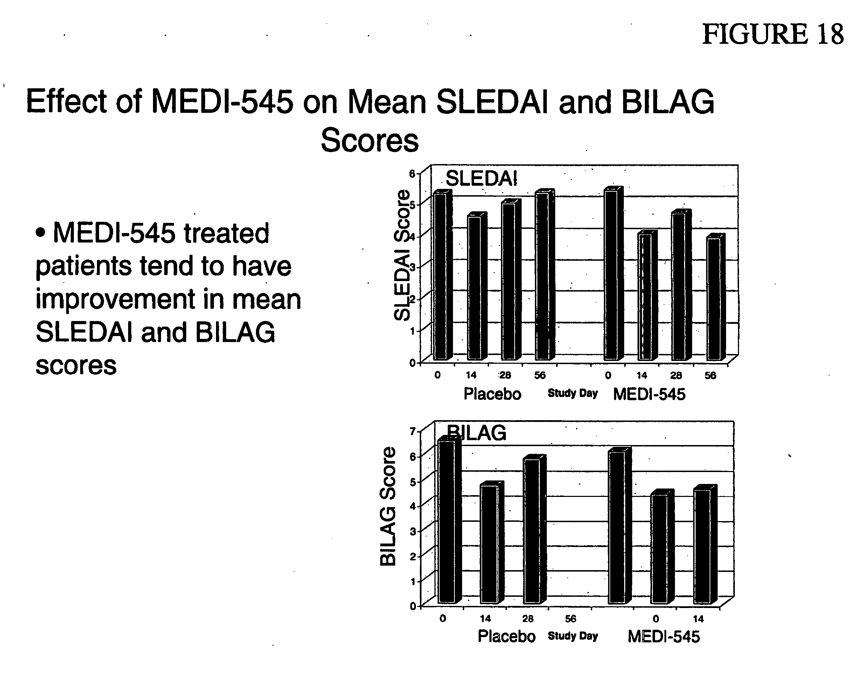

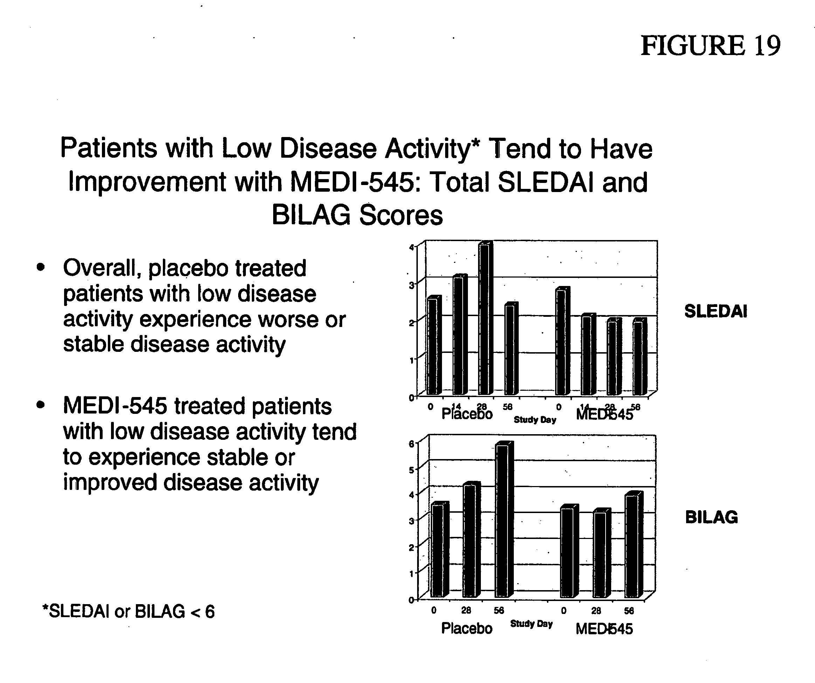

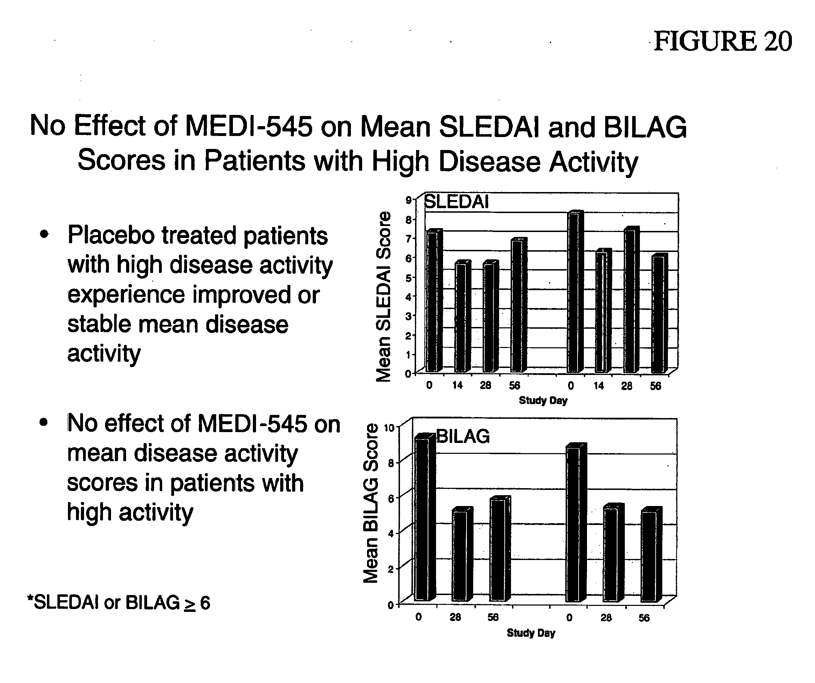

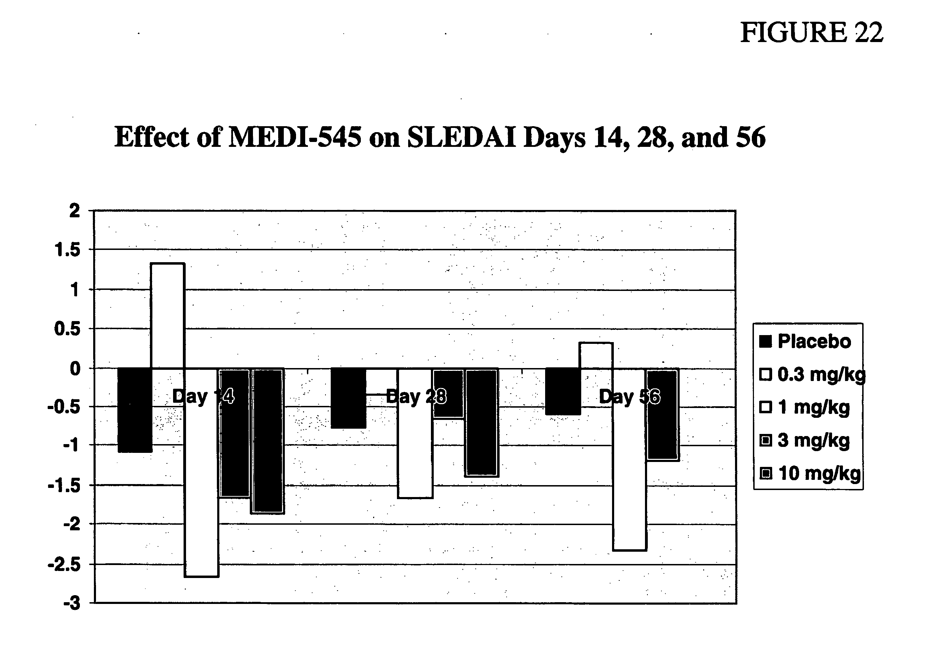

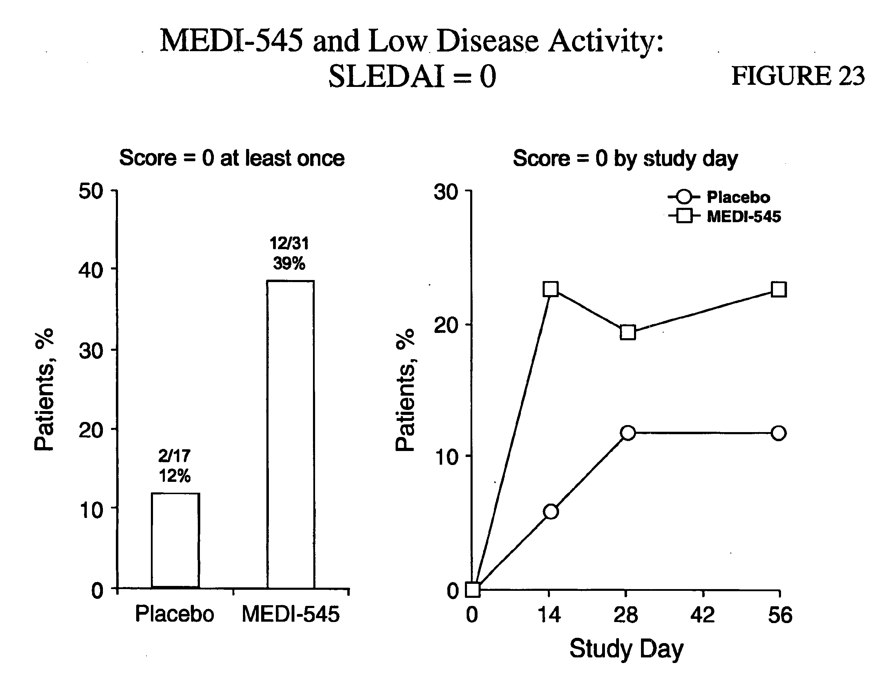

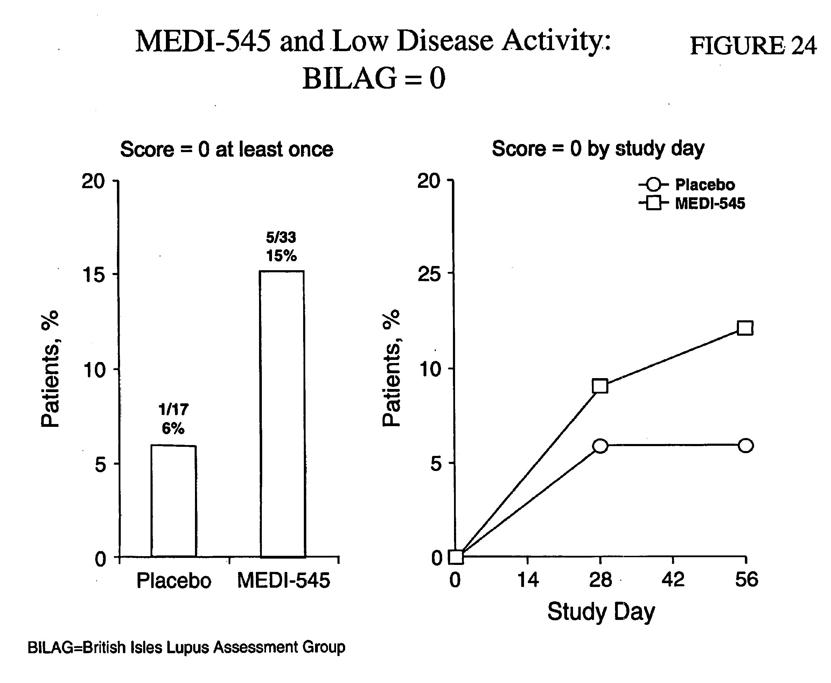

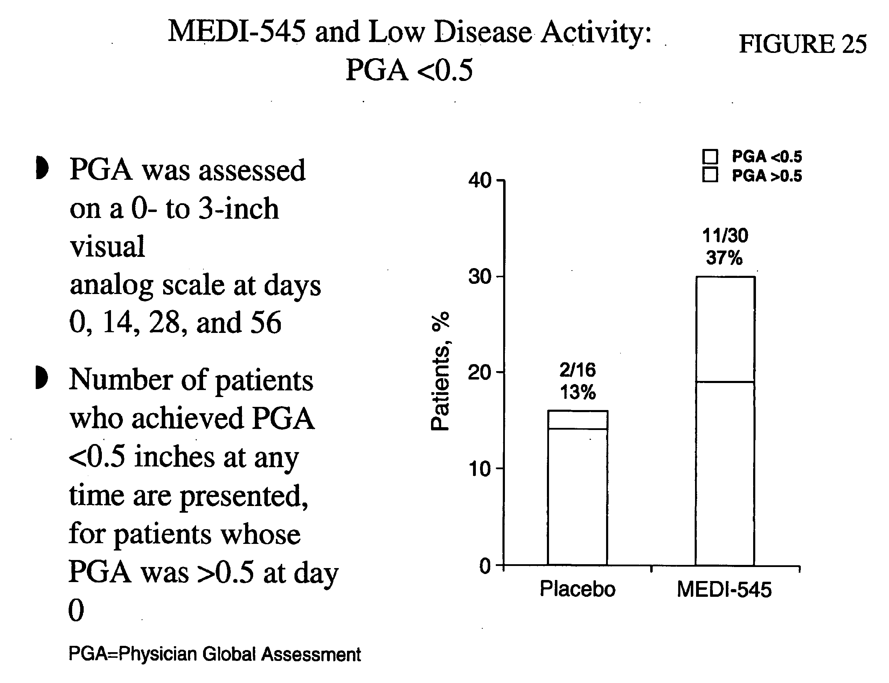

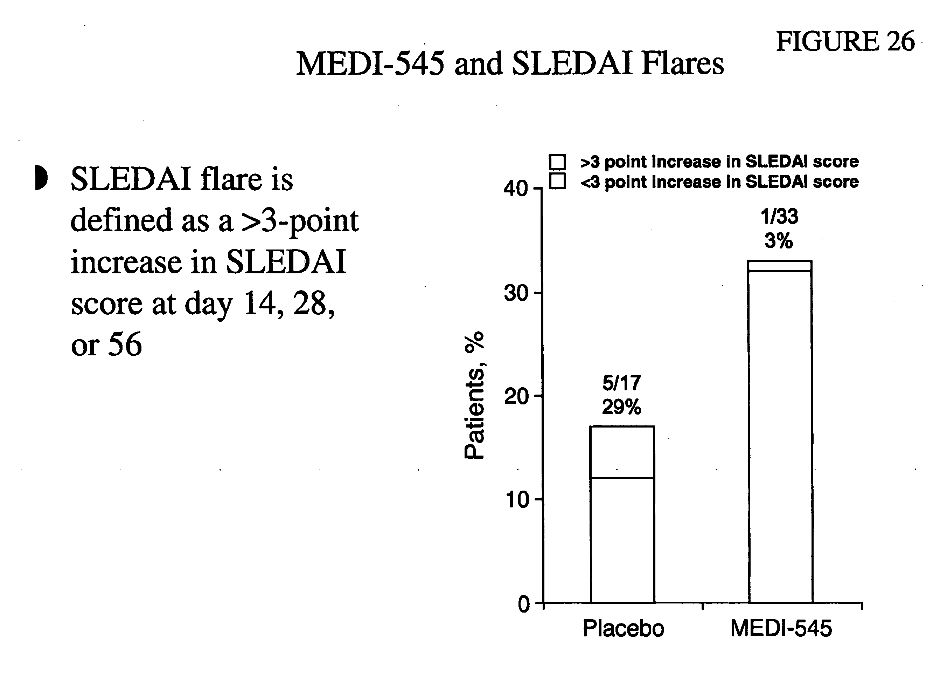

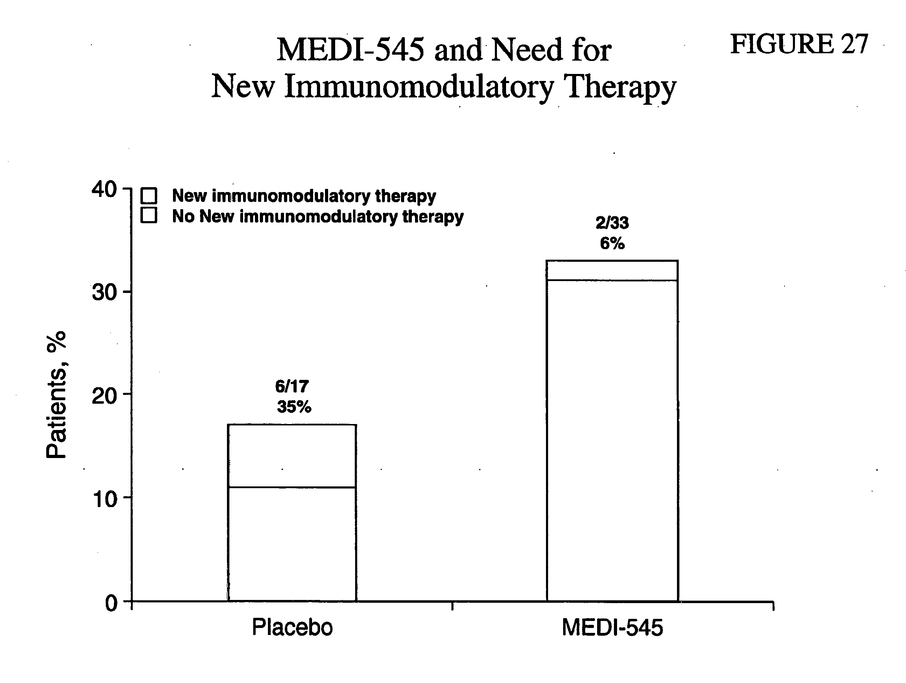

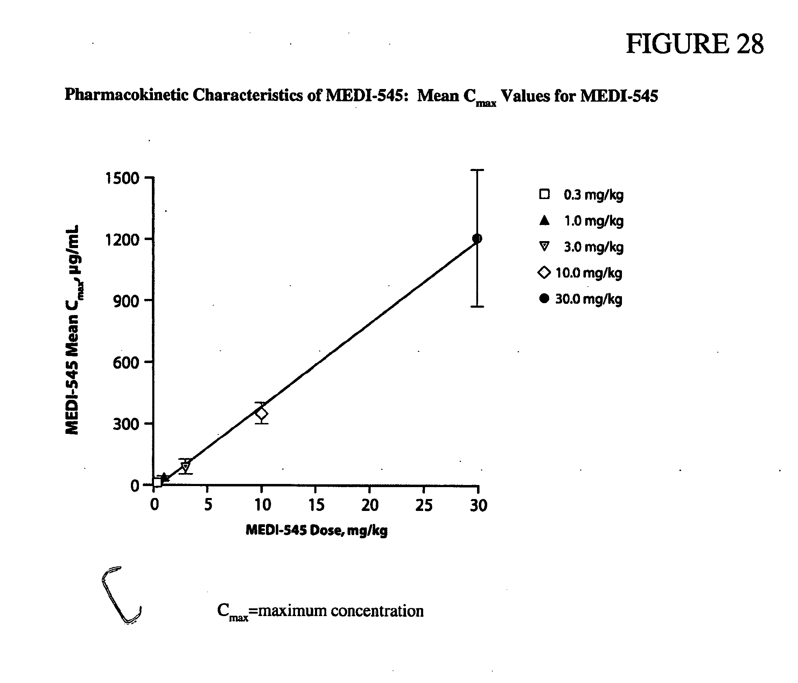

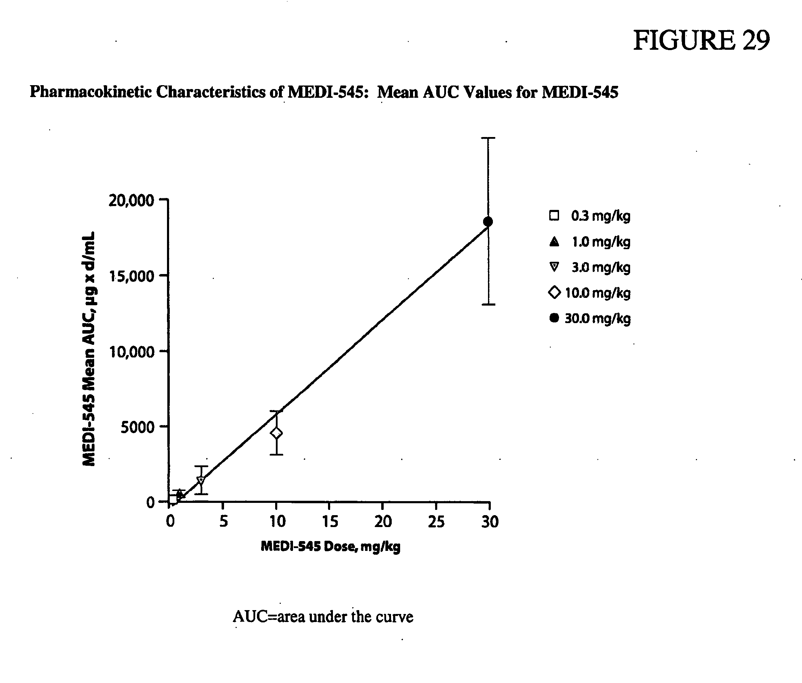

Data from this clinical trial was analyzed using routine statistical analyses and is summarized herein in Figures 2-29.

### Example 3: Anti-IFNα Antibody Drug Label

Based on clinical trial data, it can be conceived that the drug label for an anti-IFNα antibody drug may read substantially as follows:

### HIGHLIGHTS

Indications and Usage: This anti-IFNα antibody is indicated for reducing disease activity, reducing signs and symptoms, inducing complete clinical response, improving response rate based on composite responder ndex, reducing overall disease flares, reducing renal flares, and reducing the use of systemic corticosteroids in patients with moderately to severely systemic lupus erythematosus in combination with other disease modifying therapy.

Dosage and Administration: This anti-IFNα antibody can be administered as an .intravenous infusion or subcutaneous injection.

### Anti-IFNα antibody Intravenous Infusion:

The recommended dose of this anti-IFNα antibody is 500 mg intravenous infusion at 2 weeks then every 8 weeks thereafter. Some patients may gain additional benefit from increasing the frequency of infusions to every 4 weeks following the second infusion and some patients may gain additional benefit from use of a loading dose (500-1000 mg intravenously), followed by 500 mg intravenous infusion at 2 weeks then every 8 weeks thereafter. The loading dose may be with 10, 20, or 30 mg/kg.

### Anti-IFNα antibody Subcutaneous Injection:

The recommended dose of this anti-IFNα antibody is 100 mg every 2-4 weeks that can be self administered by the patients by subcutaneous injection. Some patients may benefit from a single loading dose of 500 - 1000 mg intravenous infusion followed by a subcutaneous injection of 100 mg at 2 weeks then every 2-4 weeks thereafter.

### Dosage Forms and Strengths:

Anti-IFNα antibody single-use prefilled syringe contains 100mg of anti-IFNα antibody in a single-dose, 1 ml syringe with a 27-gauge, ½-inch needle.

Anti-IFNα antibody liquid concentrate for IV injection is available in single-use vials in the following strength: 500 mg of anti-IFNα antibody in a 5 mL vial

Contraindications: Contraindicated in patients with a history of hypersensitivity to anti-IFNα antibodies. Past history of severe viral infections or active hepatitis B, hepatitis C or HIV infection.

Warnings and Precautions: Anaphylaxis and hypersensitivity reactions (<0.1%);

Adverse Reactions: Most common adverse reactions (<10%) are mild and transient injection site reactions and headache.

### INDICATIONS AND USAGE

This anti-IFNα antibody is indicated for reducing disease activity, reducing signs and symptoms, inducing complete clinical response, improving response rate based on composite responder ndex, reducing overall disease flares, reducing renal flares, and reducing the use of systemic corticosteroids in patients with moderately to severely systemic lupus erythematosus in combination with other disease modifying therapy.

### DOSAGE AND ADMINISTRATION

This anti-IFNα antibody can be administered as an intravenous infusion or subcutaneous injection.

### Anti-IFNα antibody Intravenous Infusion:

The recommended dose of this anti-IFNα antibody is 500 mg intravenous infusion at 2 weeks then every 8 weeks thereafter. Some patients may gain additional benefit from increasing the frequency of infusions to every 4 weeks following the second infusion and some patients may gain additional benefit from use of a loading dose (500-1000 mg intravenously), followed by 500 mg intravenous infusion at 2 weeks then every 8 weeks thereafter.

### Anti-IFNα antibody Subcutaneous Injection:

The recommended dose of this anti-IFNα antibody is 100 mg every 2-4 weeks that can be self administered by the patients by subcutaneous injection. Some patients may benefit from a single loading dose of 500 - 1000 mg intravenous infusion followed by a subcutaneous injection of 100 mg at 2 weeks then every 2-4 weeks thereafter. The loading dose may include 10, 20, or 30 mg/kg of antibody.

### DOSAGE FORMS AND STRENGTHS

Anti-IFNα antibody single-use prefilled syringe contains 100mg of anti-IFNα, antibody in a single-dose, 1ml syringe with a 27-gauge, ½-inch needle.

Anti-IFNα antibody liquid concentrate for IV injection is available in single-use vials in the following strength: 500 mg anti-IFNα antibody in a 5 mL vial

### CONTRAINDICATIONS

Contraindicated in patients with a history of hypersensitivity to anti-IFNα antibodies. Past history of severe viral infections such as herpes zoster or herpes ophtalmica or active hepatitis B, hepatitis C or HIV infection.

### WARNINGS AND PRECAUTIONS

Infections - do not infuse this anti-IFNα antibody during an active infection. If an infection develops, monitor carefully, and stop MEDI 545 if infections do not respond to appropriate therapy.

Malignancies - do not occur more often than in controls with active autoimmune disease.

Anaphylaxis or serious allergic reactions may occur in less than 0.5% of patients

Infusion reaction may occur in less than 2% of patients.

### ADVERSE REACTIONS

The most common adverse reaction with this anti-IFNα antibody was injection site reactions with subcutaneous administration. In placebo-controlled trials, 20% of patients treated with this anti-IFNα antibody developed injection site reactions (erythema and/or itching, hemorrhage, pain or swelling), compared to 14% of patients receiving placebo. Most injection site reactions were described as mild and generally did not necessitate drug discontinuation.

The proportion of patients who discontinued treatment due to adverse events during the double-blind, placebo-controlled portion of Studies I, II, III and IV was 7% for patients taking this anti-IFNα antibody and 4% for placebo-treated patients. The most common adverse events leading to discontinuation of this anti-IFNα antibody were exacerbation of disease (0.7%), rash (0.3%) and pneumonia (0.3%).

### Infections

In placebo-controlled trials, the rate of infection was 1 per patient-year in the anti-IFNα antibody -treated patients and 0.9 per patient-year in the placebo-treated patients. The infections consisted primarily of herpes virus reactivation including varicella zoster, herpes simplex type 1, upper respiratory tract infections, bronchitis and urinary tract infections. The type of infection did not differ between the anti-IFNα antibody treated patients and placebo-treated patients. Most patients continued on this anti-IFNα antibody after the infection resolved. The incidence of serious infections was 0.04 per patient-year in anti-IFNα antibody treated patients and 0.02 per patient-year in placebo-treated patients. Serious infections observed included varicella zoster, pneumonia, cellulitis, and pyelonephritis (see WARNINGS).

### Immunogenicity

Patients in Studies I, II, and III were tested at multiple time points for antibodies to this anti-IFNα antibody during the 6- to 12-month period. Approximately 1 % (10 of 1062) of adult RA/DM/PM/SS patients receiving this anti-IFNα antibody developed low-titer antibodies to this anti-IFNα antibody at least once during treatment, which were neutralizing in vitro. No apparent correlation of antibody development to adverse events was observed.

### CLINICAL PHARMACOLOGY

This anti-IFNα antibody has been shown to exhibit high affinity for antigen as measured by Biacore analysis; high potency neutralization of a broad spectrum of IFN-α subtypes and reversal of IFN-α induced cell surface and soluble marker expression in healthy donor peripheral blood mononuclear cells (PBMCs). This anti-IFNα antibody inhibits IFN-α induced signature in healthy donor peripheral blood mononuclear cells (PBMCs) and inhibits IFN-α induced signature in lupus PBMCs in the skin and reduce expression of type 1 IFN-α induced protein in tissue. This anti-IFNα antibody normalizes increased expression of interferon α induced proteins in peripheral blood, whole blood and tissues. Normalization was associated with clinical efficacy

Pharmacokinetics: The toxicity and pharmacokinetics (PK) of intravenous (IV) delivery of this anti-IFNα antibody were assessed in three separate studies in cynomolgus monkeys. There were no dose-limiting toxicities observed in any of the three studies. The highest dose evaluated was 100 mg/kg. Mean half-life was about 18 days. There were no anti- anti-IFNα antibodies detected in any of the three monkey studies. Tissue cross-reactivity of this anti-IFNα antibody was assessed in studies against both human and cynomolgus monkey tissues. There was no detectable binding of this anti-IFNα antibody in either of these studies.

### HOW SUPPLIED/STORAGE AND HANDLING

Each anti-IFNα antibody single-use prefilled syringe contains 100mg of antibody in a single-dose 1 ml syringe with a 27-gauge, ½-inch needle. Anti-IFNα antibody single-use prefilled syringe and autoinjector are supplied in a carton, or "kit" containing 1 prefilled syringe or one autoinjector.

Anti-IFNα antibody liquid concentrate for IV injection is supplied in individually-boxed single-use vials in the following strength: 500 mg MEDI 545 in a 5 mL vial.

Store the product under refrigeration at 2°C to 8°C (36°F to 46°F). Do not freeze. Do not use beyond the expiration date. This product contains no preservative.

Whereas, particular embodiments of the invention have been described above for purposes of description, it will be appreciated by those skilled in the art that numerous variations of the details may be made without departing from the invention as described in the appended claims.

All publications, patents and patent applications mentioned in this specification are herein incorporated by reference into the specification to the same extent as if each individual publication, patent or patent application was specifically and individually indicated to be incorporated herein by reference.

## Claims

1. An anti-interferon α monoclonal antibody for use in reducing SLEDAI flares in a human subject with moderate, severe, or very severe SLE,
wherein the human subject has a pre-treatment SLEDAI score of at least 5.

2. The anti-interferon α monoclonal antibody of claim 1, wherein the post-treatment SLEDAI score is equal to the pre-treatment SLEDAI score minus at least 1, with 0 being the lowest post-treatment SLEDAI score attainable.

3. The anti-interferon α monoclonal antibody of claims 1 or 2, wherein the improvement in SLEDAI score is evident at least 14 days, at least 28 days, or at least 56 days post antibody administration.

4. The anti-interferon α monoclonal antibody of any of the preceding claims, wherein the anti-interferon α monoclonal antibody is administered at a dose selected from the group consisting of 0.5 mg/kg, 1.0 mg/kg, 2.0 mg/kg, 3.0 mg/kg, 4.0 mg/kg, 5.0 mg/kg, 6.0 mg/kg, 7.0 mg/kg, 8.0 mg/kg, 9.0 mg/kg, 10.0 mg/kg, 11.0 mg/kg, 12.0 mg/kg, 13.0 mg/kg, 14.0 mg/kg, 15.0 mg/kg, 16.0 mg/kg, 17.0 mg/kg, 18.0 mg/kg, 19.0 mg/kg, 20.0 mg/kg, 25 mg/kg, and 30 mg/kg.

5. The anti-interferon α monoclonal antibody of any of the preceding claims, further comprising administration of at least one additional therapeutic agent as part of the treatment regimen.

6. The anti-interferon α monoclonal antibody of claim 5, wherein the additional therapeutic is selected from the group consisting of: corticosteroids, NSAIDS, antimalarials, immunomodulating agents, and anticoagulants.

7. The anti-interferon α monoclonal antibody of claim 6, wherein the corticosteroid is prednisone.

8. The anti-interferon α monoclonal antibody of any of the preceding claims, wherein the anti-interferon α monoclonal antibody is administered parenterally, intravenously, or subcutaneously.

9. The anti-interferon α monoclonal antibody of claim 8, wherein the anti-interferon α monoclonal antibody is administered subcutaneously.
